# EUROPÄISCHE PATENTSCHRIFT

(11) **EP 0 817 853 B1**
(45) Veröffentlichungstag und Bekanntmachung des Hinweises auf die Patenterteilung: **27.06.2007**
(21) Anmeldenummer: 96910941.2
(22) Anmeldetag: 28.03.1996
(51) Int. Cl.: C12N 15/40, A61K 39/12

(54) **INFEKTIÖSER cDNA-KLON DES TICK-BORNE ENZEPHALITIS (TBE)-VIRUS, DAVON ABGELEITETER REKOMBINANTER IMPFSTOFF UND HERSTELLUNG DESSELBEN SOWIE EIN PHARMAZEUTISCHES PRODUKT, DAS EINE REPLIZIERBARE NUKLEINSÄURE ENTHÄLT**
INFECTIOUS DNA CLONE OF THE TICK-BORNE ENCEPHALITIS (TBE) VIRUS; RECOMBINANT VACCINE DERIVED FROM THE SAID CLONE AND PRODUCTION OF THE SAID VACCINE; AND A PHARMACEUTICAL PRODUCT CONTAINING A REPLICABLE NUCLEIC ACID
CLONE INFECTIEUX D'ADN-C DU VIRUS DE L'ENCEPHALITE TRANSMISE PAR LES TIQUES (TBE), VACCIN RECOMBINE OBTENU A PARTIR DE CELUI-CI ET FABRICATION DE CE VACCIN; PRODUIT PHARMACEUTIQUE COMPORTANT UN ACIDE NUCLEIQUE POUVANT ETRE REPLIQUE

(30) Priorität: 31.03.1995 DE 19512142
(43) Veröffentlichungstag der Anmeldung: 14.01.1998
(73) Patentinhaber: Baxter Aktiengesellschaft, 1221 Wien (AT)
(72) Erfinder: HEINZ, Franz, Xaver, A-1080 Wien (AT); KUNZ, Christian, A-1180 Wien (AT); MANDL, Christian, A-1160 Wien (AT)
(74) Vertreter: Polz, Leo
(86) Internationale Anmeldenummer: PCT/EP1996/001376
(87) Internationale Veröffentlichungsnummer: WO 1996/030521

(56) Entgegenhaltungen:
- EP-A- 0 464 287
- EP-A- 0 541 089
- WO-A-93/06214
- WO-A-94/21797
- WO-A-95/05853
- NEW BIOL, DEC 1989, 1 (3) P285-96, UNITED STATES, XP000602631 RICE CM ET AL: "Transcription of infectious yellow fever RNA from full-length cDNA templates produced by in vitro ligation."
- VACCINE, 1994, XP002021790 ZHOU X ET AL: "Self-replicating Semliki-Forest virus RNA as recombinant vaccine"
- NUCLEIC ACIDS RESEARCH, 1995, 23, 1495-1501, XP002021791 JOHANNING FW ET AL: "A SINDBIS VIRUS MESSENGER-RNA POLYNUCLEOTIDE VECTOR ACHIEVES PROLONGED AND HIGH-LEVEL HETEROLOGOUS GENE-EXPRESSION IN-VIVO"
- NATURE, MAR 12 1992, 356 (6365) P152-4, ENGLAND, XP000310569 TANG DC ET AL: "Genetic immunization is a simple method for eliciting an immune response."
- VIROLOGY (1989), 173(1), 291-301 CODEN: VIRLAX;ISSN: 0042-6822, XP000602619 MANDL, CHRISTIAN W. ET AL: "Genome sequence of tick - borne encephalitis virus ( Western subtype) and comparative analysis of nonstructural proteins with other flaviviruses"
- J VIROL, SEP 1992, 66 (9) P5425-31, UNITED STATES, XP000602633 SUMIYOSHI H ET AL: "Infectious Japanese encephalitis virus RNA can be synthesized from in vitro-ligated cDNA templates."
- VIROLOGY (1988), 166(1), 197-205 CODEN: VIRLAX;ISSN: 0042-6822, XP000602618 MANDL, CHRISTIAN W. ET AL: "Sequence of the structural proteins of tick - borne encephalitis virus ( Western subtype) and comparative analysis with other flaviviruses"
- GENE (1994), 149(2), 193-201 CODEN: GENED6;ISSN: 0378-1119, XP002014004 YAMSHCHIKOV, VLADIMIR F. ET AL: "Generation of long flavivirus expression cassettes by in vivo recombination and transient dominant selection"
- VIROLOGY (1995), 214(2), 611-18 CODEN: VIRLAX;ISSN: 0042-6822, XP002014005 GRITSUN, T. S. ET AL: "Infectious transcripts of tick - borne encephalitis virus, generated in days by RT-PCR"
- PROC NATL ACAD SCI U S A, SEP 27 1994, 91 (20) P9519-23, UNITED STATES, XP002021792 RAZ E ET AL: "Intradermal gene immunization: the possible role of DNA uptake in the induction of cellular immunity to viruses."

### SEQUENZPROTOKOLL

(1) ALLGEMEINE ANGABEN:
   (i) ANMELDER:
      (A) NAME: IMMUNO AG
      (B) STRASSE: Industriestraße 67
      (C) ORT: Wien
      (D) BUNDESLAND: Austria
      (E) LAND: Austria
      (F) POSTLEITZAHL: 1220
   (ii) BEZEICHNUNG DER ERFINDUNG: Infektioeser cDNA-Klon des Tick-Borne-Enzephalitis (TBE)-Virus, davon abgeleiteter rekombinanter Impfstoff und Herstellung desselben sowie ein pharmazeutisces Produkt, das eine replizierbare Neukleinsäure enthält
   (iii) ANZAHL DER SEQUENZEN: 20
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 46 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 1:
      CGGTCGACGA TTTAGGTGAC ACTATAAGAT TTTCTTGCAC GTGCAT 46
(2) ANGABEN ZU SEQ ID NO: 2:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 2:
      GACGGACGAA CAGATGAATA 20
(2) ANGABEN ZU SEQ ID NO: 3:
   (i) SEQUENZKENNZEICHEN:
      (A) LANGE: 20 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 3:
      CCCGGAAGGC TCGTAAAAGA 20
(2) ANGABEN ZU SEQ ID NO: 4:
   (i) SEQUENZKENNZEICHEN:
      (A) LANGE: 20 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 4:
      CCACCACATA GCGCACCAGG 20
(2) ANGABEN ZU SEQ ID NO: 5:
   (i) SEQUENZKENNZEICHEN:
      (A) LÄNGE: 40 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 5:
      CCGACGTCAA TATTTTTTTT TTTTTTTTTT TTGTTTAAAG 40
(2) ANGABEN ZU SEQ ID NO: 6:
   (i) SEQUENZKENNZEICHEN:
      (A) LÄNGE: 13 Basenpaare
      (B) ART: Nucleotid
      (C) STRANGFORM: Einzelstrang
      (D) TOPOLOGIE: linear
   (ii) ART DES MOLEKULS: Genom-DNA
   (xi) SEQUENZBESCHREIBUNG: SEQ ID NO: 6:
      CTAGTCACAC TAT 13
(2) ANGABEN ZU SEQ ID NO: 7:
   (i) SEQUENZKENNZEICHEN:
      (A) LANGE: 11 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 7:
      CGATAGTGTG A 11
(2) ANGABEN ZU SEQ ID NO: 8:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 8:
      GCTACGGCAT GCCACAAGAT 20
(2) ANGABEN ZU SEQ ID NO: 9:
   (i) SEQUENZKENNZEICHEN:
      (A) LÄNGE: 51 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 9:
      CCGGCCGTCG ACTTAATACG ACTCACTATA GAGATTTTCT TGCACGTGCA T 51
(2) ANGABEN ZU SEQ ID NO: 10:
   (i) SEQUENZKENNZEICHEN:
      (A) LANGE: 20 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 10:
      AGGGGGGGCG GTTCTTGTTC 20
(2) ANGABEN ZU SEQ ID NO: 11:
   (i) SEQUENZKENNZEICHEN:
      (A) LÄNGE: 19 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 11:
      AGCGGGTGTT TTTCCGAGT 19
(2) ANGABEN ZU SEQ ID NO: 12:
   (i) SEQUENZKENNZEICHEN:
      (A) LANGE: 34 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 12:
      AAGACGTCGC TAGCGGGTGT TTTTCCGAGT CACA 34
(2) ANGABEN ZU SEQ ID NO: 13:
   (i) SEQUENZKENNZEICHEN:
      (A) LANGE: 27 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 13:
      CGCGGATCCA TCAACTCAGC AGTGCCT 27
(2) ANGABEN ZU SEQ ID NO: 14:
   (i) SEQUENZKENNZEICHEN:
      (A) LÄNGE: 35 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 14:
      CGCGGATCCG CTAGCGGGTG TTTTTCCGAG TCACA 35
(2) ANGABEN ZU SEQ ID NO: 15:
   (i) SEQUENZKENNZEICHEN:
      (A) LÄNGE: 34 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 15:
      ATTGATGACT CAGTTCCCCA ACATGGATGA TGTT 34
(2) ANGABEN ZU SEQ ID NO: 16:
   (i) SEQUENZKENNZEICHEN:
      (A) LÄNGE: 40 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 16:
      GTCGACTTAA TACGACTCAC TATAGAGATT TTCTTGCACG 40
(2) ANGABEN ZU SEQ ID NO: 17:
   (i) SEQUENZKENNZEICHEN:
      (A) LANGE: 24 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 17:
      GGAAAAACAC CCGCTAGCGA CGTC 24
(2) ANGABEN ZU SEQ ID NO: 18:
   (i) SEQUENZKENNZEICHEN:
      (A) LANGE: 24 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 18:
      GGAAAAACAC CCGCTAGCGG ATCC 24
(2) ANGABEN ZU SEQ ID NO: 19:
   (i) SEQUENZKENNZEICHEN:
      (A) LÄNGE: 11141 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 19:
   AGATTTTCTT GCACGTGCAT GCGTTTGCTT CGGACAGCAT TAGCAGCGGT TGGTTTGAAA     60
   GAGATATTCT TTTGTTTCTA CCAGTCGTGA ACGTGTTGAG AAAAAGACAG CTTAGGAGAA    120
   CAAGAGCTGG GGATGGTCAA GAAGGCCATC CTGAAAGGTA AGGGGGGCGG TCCCCCTCGA    180
   CGAGTGTCGA AAGAGACCGC AACGAAGACG CGTCAACCCA GAGTCCAAAT GCCAAATGGG    240
   CTTGTGTTGA TGCGCATGAT GGGGATCTTG TGGCATGCCG TAGCCGGCAC CGCGAGAAAC    300
   CCCGTATTGA AGGCGTTCTG GAACTCAGTC CCTCTGAAAC AGGCAACAGC AGCACTGCGG    360
   AAGATCAAAA GGACGGTGAG TGCCCTAATG GTTGGCTTGC AAAAACGTGG GAAAAGGAGG    420
   TCAGCGACGG ACTGGATGAG CTGGTTGCTG GTCATCACTC TGTTGGGGAT GACGCTTGCT    480
   GCAACGGTGA GGAAAGAAAG GGATGGCTCA ACTGTGATCA GAGCTGAAGG AAAGGATGCA    540
   GCAACTCAGG TGCGTGTGGA GAATGGCACC TGTGTGATCC TGGCTACTGA CATGGGGTCA    600
   TGGTGTGATG ATTCACTGTC CTATGAGTGT GTGACCATAG ATCAAGGAGA AGAGCCTGTT    660
   GACGTGGATT GTTTTTGCCG GAACGTTGAT GGAGTCTATC TGGAGTACGG ACGCTGTGGG    720
   AAACAGGAAG GCTCACGGAC AAGGCGCTCA GTGCTGATCC CATCCCATGC TCAGGGAGAG    780
   CTGACGGGAA GGGGACACAA ATGGCTAGAA GGAGACTCGC TGCGAACACA CCTTACTAGA    840
   GTTGAGGGAT GGGTCTGGAA GAACAAGCTA CTTGCCTTGG CAATGGTTAC CGTTGTGTGG    900
   TTGACCCTGG AGAGTGTGGT GACCAGGGTT GCCGTTCTTG TTGTGCTCCT GTGTTTGGCA    960
   CCGGTTTACG CTTCGCGTTG CACACACTTG GAAAACAGGG ACTTTGTGAC TGGTACTCAG   1020
   GGGACTACGA GGGTCACCTT GGTGCTGGAA CTGGGTGGAT GTGTTACTAT AACAGCTGAG   1080
   GGGAAGCCTT CAATGGATGT GTGGCTTGAC GCCATTTACC AGGAGAACCC TGCTAAGACA   1140
   CGTGAGTACT GTTTACACGC CAAGTTGTCG GACACTAAGG TTGCAGCCAG ATGCCCAACA   1200
   ATGGGACCAG CCACTTTGGC TGAAGAACAC CAGGGTGGCA CAGTGTGTAA GAGAGATCAG   1260
   AGTGATCGAG GCTGGGGCAA CCACTGTGGA CTGTTTGGAA AGGGTAGCAT TGTGGCCTGT   1320
   GTCAAGGCGG CTTGTGAGGC AAAAAAGAAA GCCACAGGAC ATGTGTACGA CGCCAACAAA   1380
   ATAGTGTACA CGGTCAAAGT CGAACCACAC ACGGGAGACT ATGTTGCCGC AAACGAGACA   1440
   CATAGTGGGA GGAAGACGGC ATCCTTCACA ATTTCTTCAG AGAAAACCAT TTTGACTATG 1500
   GGTGAGTATG GAGATGTGTC TTTGTTGTGC AGGGTCGCTA GTGGCGTTGA CTTGGCCCAG 1560
   ACCGTCATCC TTGAGCTTGA CAAGACAGTG GAACACCTTC CAACGGCTTG GCAGGTCCAT 1620
   AGGGACTGGT TCAATGATCT GGCTCTGCCA TGGAAACATG AGGGAGCGCA AAACTGGAAC 1680
   AACGCAGAAA GACTGGTTGA ATTTGGGGCT CCTCACGCTG TCAAGATGGA CGTGTACAAC 1740
   CTCGGAGACC AGACTGGAGT GTTACTGAAG GCTCTCGCTG GGGTTCCTGT GGCACACATT 1800
   GAGGGAACCA AGTACCACCT GAAGAGTGGC CACGTGACCT GCGAAGTGGG ACTGGAAAAA 1860
   CTGAAGATGA AAGGTCTTAC GTACACAATG TGTGACAAAA CAAAGTTCAC ATGGAAGAGA 1920
   GCTCCAACAG ACAGTGGGCA TGATACAGTG GTCATGGAAG TCACATTCTC TGGAACAAAG 1980
   CCCTGTAGGA TCCCAGTCAG GGCAGTGGCA CATGGATCTC CAGATGTGAA CGTGGCCATG 2040
   CTGATAACGC CAAACCCAAC AATTGAAAAC AATGGAGGTG GCTTCATAGA GATGCAGCTG 2100
   CCCCCAGGGG ATAACATCAT CTATGTTGGG GAACTGAGTC ATCAATGGTT CCAAAAAGGG 2160
   AGCAGCATCG GAAGGGTTTT CCAAAAGACC AAGAAAGGCA TAGAAAGACT GACAGTGATA 2220
   GGAGAGCACG CCTGGGACTT CGGTTCTGCT GGAGGCTTTC TGAGTTCAAT TGGGAAGGCG 2280
   GTACATACGG TCCTTGGTGG CGCTTTCAAC AGCATCTTCG GGGGAGTGGG GTTTCTACCA 2340
   AAACTTTTAT TAGGAGTGGC ATTGGCTTGG TTGGGCCTGA ACATGAGAAA CCCTACAATG 2400
   TCCATGAGCT TTCTCTTGGC TGGAGGTCTG GTCTTGGCCA TGACCCTTGG AGTGGGGGCG 2460
   GATGTTGGTT GCGCTGTGGA CACGGAACGA ATGGAGCTCC GCTGTGGCGA GGGCCTGGTC 2520
   GTGTGGAGAG AGGTCTCAGA ATGGTATGAC AATTATGCCT ACTACCCGGA GACACCGGGG 2580
   GCCCTTGCAT CAGCCATAAA GGAGACATTT GAGGAGGGAA GCTGTGGTGT AGTCCCCCAG 2640
   AACAGGCTCG AGATGGCCAT GTGGAGAAGC TCGGTCACAG AGCTGAATTT GGCTCTGGCG 2700
   GAAGGGGAGG CAAATCTCAC AGTGGTGGTG GACAAGTTTG ACCCCACTGA CTACCGAGGT 2760
   GGTGTCCCTG GTTTACTGAA AAAAGGAAAG GACATAAAAG TCTCCTGGAA AAGCTGGGGC 2820
   CATTCAATGA TCTGGAGCAT TCCTGAGGCC CCCCGTCGCT TCATGGTGGG CACGGAAGGA 2880
   CAAAGTGAGT GTCCCCTAGA GAGACGGAAG ACAGGTGTTT TCACGGTGGC AGAATTCGGG 2940
   GTTGGCCTGA GAACAAAGGT CTTCTTGGAT TTCAGACAGG AACCAACACA TGAGTGTGAC 3000
   ACAGGAGTGA TGGGAGCTGC AGTCAAGAAC GGCATGGCAA TCCACACAGA TCAAAGTCTC 3060
   TGGATGAGAT CAATGAAAAA TGACACAGGC ACTTACATAG TTGAACTTTT GGTCACTGAC 3120
   CTGAGGAACT GCTCATGGCC TGCTAGCCAC ACTATCGATA ATGCTGACGT GGTGGACTCA 3180
   GAGTTATTCC TTCCGGCGAG CCTGGCAGGA CCCAGATCCT GGTACAACAG GATACCTGGC 3240
   TATTCAGAAC AGGTGAAAGG GCCATGGAAG TACACGCCTA TCCGAGTTAT CAGAGAGGAG 3300
   TGTCCCGGCA CGACCGTTAC CATCAACGCC AAGTGTGACA AAAGAGGAGC ATCTGTGAGG 3360
   AGTACCACAG AGAGTGGCAA GGTTATCCCA GAATGGTGCT GCCGAGCGTG CACAATGCCA 3420
   CCAGTGACGT TCCGGACTGG AACTGATTGC TGGTATGCCA TGGAAATACG GCCAGTCCAT 3480
   GACCAGGGGG GGCTTGTTCG CTCAATGGTG GTTGCGGACA ACGGTGAATT ACTTAGTGAG 3540
   GGAGGAGTCC CCGGAATAGT GGCATTGTTT GTGGTCCTTG AATACATCAT CCGTAGGAGG 3600
   CCCTCCACGG GAACGACGGT TGTGTGGGGG GGTATCGTCG TTCTCGCTCT GCTTGTCACC 3660
   GGGATGGTCA GGATAGAGAG CCTGGTGCGC TATGTGGTGG CAGTGGGGAT CACATTCCAC 3720
   CTTGAGCTGG GGCCAGAGAT CGTGGCCTTG ATGCTACTCC AGGCTGTGTT TGAGCTGAGG 3780
   GTGGGTTTGC TCAGCGCATT TGCATTGCGC AGAAGCCTCA CCGTCCGAGA GATGGTGACC 3840
   ACCTACTTTC TTTTGCTGGT CCTGGAATTG GGGCTGCCGG GTGCGAGCCT TGAGGAGTTC 3900
   TGGAAATGGG GTGATGCACT GGCCATGGGG GCGCTGATAT TCAGGGCTTG CACGGCAGAA 3960
   GGAAAGACTG GAGCGGGGCT TTTGCTCATG GCTCTCATGA CACAGCAGGA TGTGGTGACT 4020
   GTGCACCATG GACTGGTGTG CTTCCTAAGT GTAGCTTCGG CATGCTCGGT CTGGAGGCTG 4080
   CTCAAGGGAC ACAGAGAGCA GAAGGGATTG ACCTGGGTTG TCCCCCTGGC TGGGTTGCTT 4140
   GGGGGAGAGG GCTCTGGAAT CAGACTGCTG GCGTTTTGGG AGTTGTCAGC GCACAGAGGA 4200
   AGACGATCTT TCAGTGAACC ACTAACTGTG GTAGGAGTCA TGCTAACACT GGCCAGCGGC 4260
   ATGATGCGAC ACACTTCCCA GGAGGCTCTC TGTGCACTCG CAGTGGCCTC GTTTCTCTTG 4320
   CTGATGCTGG TGCTGGGGAC AAGAAAGATG CAGCTGGTTG CCGAATGGAG TGGCTGCGTT 4380
   GAATGGTATC CGGAACTAGT GAATGAGGGT GGAGAGGTTA GCCTGCGGGT CCGGCAGGAC 4440
   GCGATGGGTA ACTTTCACTT GACTGAGCTC GAGAAAGAAG AGAGAATGAT GGCTTTTTGG 4500
   CTGATTGCCG GCTTGGCAGC TTCGGCCATT CACTGGTCAG GCATTCTTGG TGTGATGGGA 4560
   CTGTGGACGC TCACGGAAAT GCTGAGGTCA TCCCGAAGGT CTGACCTGGT TTTCTCTGGA 4620
   CAGGGGGGTC GAGAGCGTGG TGACAGACCT TTCGAGGTTA AGGACGGTGT CTACAGGATT 4680
   TTTAGCCCCG GCTTGTTCTG GGGTCAGAAC CAGGTGGGAG TTGGCTACGG TTCCAAGGGT 4740
   GTCTTGCACA CGATGTGGCA CGTGACGAGA GGAGCGGCGC TGTCTATTGA TGACGCTGTG 4800
   GCCGGTCCCT ACTGGGCTGA TGTGAGGGAA GATGTCGTGT GTTACGGAGG AGCCTGGAGC 4860
   CTGGAGGAAA AATGGAAAGG TGAAACAGTA CAGGTCCATG CCTTCCCACC GGGGAGGGCG 4920
   CATGAGGTGC ATCAGTGCCA GCCTGGGGAG TTGATCCTTG ACACCGGAAG AAAGCTTGGG 4980
   GCAATACCAA TTGATTTGGT GAAAGGAACA TCAGGCAGCC CCATTCTCAA CGCCCAGGGA 5040
   GTGGTTGTGG GGCTATACGG AAATGGCCTA AAAACTAATG AGACCTACGT CAGCAGCATT 5100
   GCTCAAGGGG AAGCGGAGAA GAGTCGCCCC AACCTCCCAC AGGCTGTTGT GGGTACGGGC 5160
   TGGACATCAA AGGGTCAGAT CACAGTGCTG GACATGCACC CAGGCTCGGG GAAGACCCAC 5220
   AGAGTCCTCC CGGAGCTCAT TCGCCAATGC ATTGACAGGC GCCTGAGAAC GTTGGTGTTG 5280
   GCTCCAACTC GTGTGGTACT CAAAGAAATG GAGCGTGCTT TGAATGGGAA ACGGGTCAGG 5340
   TTCCACTCAC CAGCAGTCAG TGACCAACAG GCTGGAGGGG CAATTGTCGA TGTGATGTGT 5400
   CACGCAACCT ATGTCAACAG AAGGCTACTC CCACAGGGAA GACAAAATTG GGAGGTGGCA 5460
   ATCATGGATG AGGCCCACTG GACGGACCCC CACAGCATAG CTGCCAGAGG TCATTTGTAT 5520
   ACTCTGGCAA AAGAAAACAA GTGTGCACTG GTCTTGATGA CAGCGACACC TCCTGGTAAG 5580
   AGTGAACCCT TTCCGGAGTC CAATGGAGCC ATTACTAGTG AGGAAAGACA GATTCCTGAT 5640
   GGGGAGTGGC GTGACGGGTT TGACTGGATC ACTGAGTATG AAGGGCGCAC CGCCTGGTTT 5700
   GTCCCTTCGA TTGCAAAAGG TGGTGCTATA GCTCGCACCT TGAGACAGAA GGGGAAAAGT 5760
   GTGATTTGTT TGAACAGCAA AACCTTTGAA AAGGACTACT CCAGAGTGAG GGATGAGAAG 5820
   CCTGACTTTG TGGTGACGAC TGATATCTCG GAGATGGGAG CTAACCTTGA CGTGAGCCGC 5880
   GTCATAGATG GGAGGACAAA CATCAAGCCC GAGGAGGTTG ATGGGAAAGT CGAGCTCACC 5940
   GGGACCAGGC GAGTGACCAC GGCTTCCGCT GCCCAACGGC GCGGAAGAGT TGGTCGGCAA 6000
   GACGGACGAA CAGATGAATA CATATACTCT GGACAGTGTG ATGATGATGA CAGTGGATTA 6060
   GTGCAATGGA AAGAGGCGCA AATACTTCTT GACAACATAA CAACCTTGCG GGGGCCCGTG 6120
   GCCACCTTCT ATGGACCAGA ACAGGACAAG ATGCCGGAGG TGGCCGGTCA CTTTCGACTC 6180
   ACTGAAGAGA AAAGAAAGCA CTTCCGACAT CTTCTCACCC ATTGTGACTT CACACCGTGG 6240
   CTGGCATGGC ACGTCGCAGC GAATGTATCC AGCGTCACGG ATCGAAGCTG GACATGGGAA 6300
   GGGCCGGAGG CAAATGCCGT GGATGAGGCC AGTGGTGACT TGGTCACCTT TAGGAGCCCG 6360
   AATGGGGCGG AGAGAACTCT CAGGCCGGTG TGGAAGGACG CACGTATGTT CAAAGAGGGA 6420
   CGTGACATCA AAGAGTTCGT GGCGTACGCG TCTGGGCGTC GCAGCTTCGG AGATGTTCTG 6480
   ACAGGAATGT CGGGAGTTCC TGAGCTCCTG CGGCACAGAT GCGTCAGTGC CCTGGATGTC 6540
   TTCTACACGC TTATGCATGA GGAACCTGGC AGCAGGGCAA TGAGAATGGC GGAGAGAGAT 6600
   GCCCCAGAGG CCTTTCTGAC TATGGTTGAG ATGATGGTGC TGGGTTTGGC AACCCTGGGT 6660
   GTCATCTGGT GCTTCGTCGT CCGGACTTCA ATCAGCCGCA TGATGCTGGG CACGCTGGTC 6720
   CTGCTGGCCT CCTTGCTACT CTTGTGGGCA GGTGGCGTCG GCTATGGGAA CATGGCTGGA 6780
   GTGGCTCTCA TCTTTTACAC GTTGCTGACG GTGCTGCAGC CTGAGGCGGG AAAACAGAGA 6840
   AGCAGTGACG ACAACAAACT GGCATATTTC TTGCTGACGC TCTGCAGCCT TGCTGGACTG 6900
   GTTGCAGCCA ATGAGATGGG CTTTCTGGAG AAGACCAAGG CAGACTTGTC CACGGCGCTG 6960
   TGGAGTGAAC GGGAGGAACC CCGGCCATGG AGTGAATGGA CGAATGTGGA CATCCAGCCA 7020
   GCGAGGTCCT GGGGGACCTA TGTGCTGGTG GTGTCTCTGT TCACACCTTA CATCATCCAC 7080
   CAACTGCAGA CCAAAATCCA ACAACTTGTC AACAGTGCCG TGGCATCTGG TGCACAGGCC 7140
   ATGAGAGACC TTGGGGGAGG TGCCCCCTTC TTTGGTGTGG CGGGACATGT CATGACCCTC 7200
   GGGGTGGTGT CACTGATTGG GGCTACTCCC ACCTCACTGA TGGTGGGCGT TGGCTTGGCG 7260
   GCACTCCATC TGGCCATTGT GGTGTCTGGT CTGGAGGCTG AATTAACACA GAGAGCTCAT 7320
   AAGGTCTTTT TCTCTGCAAT GGTGCGCAAC CCCATGGTGG ATGGGGATGT CATCAACCCA 7380
   TTCGGGGAGG GGGAGGCAAA ACCTGCTCTA TATGAAAGGA AAATGAGTCT GGTGTTGGCC 7440
   ACAGTGTTGT GCCTCATGTC GGTGGTCATG AACCGAACGG TGGCCTCCAT AACAGAGGCT 7500
   TCAGCAGTGG GACTGGCAGC AGCGGGACAG CTGCTTAGAC CGGAGGCTGA CACGTTGTGG 7560
   ACGATGCCGG TTGCTTGTGG CATGAGTGGT GTGGTCAGGG GTAGCCTGTG GGGGTTTTTG 7620
   CCTCTTGGGC ATAGACTCTG GCTTCGAGCC TCTGGGGGTA GGCGTGGTGG TTCTGAGGGA 7680
   GACACGCTTG GAGATCTCTG GAAGCGGAGG CTGAACAACT GCACGAGGGA GGAATTCTTT 7740
   GTGTACAGGC GCACCGGCAT CCTGGAGACG GAACGTGACA AGGCTAGAGA GTTGCTCAGA 7800
   AGAGGAGAGA CCAATGTGGG ATTGGCTGTC TCTCGGGGCA CGGCAAAGCT TGCCTGGCTT 7860
   GAGGAACGCG GATATGCCAC CCTCAAGGGA GAGGTGGTAG ATCTTGGATG TGGAAGGGGC 7920
   GGCTGGTCCT ATTATGCGGC ATCCCGACCG GCAGTCATGA GTGTCAGGGC ATATACCATT 7980
   GGTGGAAAAG GGCACGAGGC TCCAAAGATG GTAACAAGCC TGGGTTGGAA CTTGATTAAA 8040
   TTCAGATCAG GAATGGACGT GTTCAGCATG CAGCCACACC GGGCTGACAC TGTCATGTGT 8100
   GACATCGGAG AGAGCAGCCC AGATGCCGCT GTGGAGGGTG AGAGGACAAG GAAAGTGATA 8160
   CTGCTCATGG AGCAATGGAA AAACAGGAAC CCCACGGCTG CCTGTGTGTT CAAGGTGCTG 8220
   GCCCCATATC GCCCAGAAGT GATAGAGGCA CTGCACAGAT TCCAACTGCA ATGGGGGGGG 8280
   GGTCTGGTGA GGACCCCTTT TTCAAGGAAC TCCACCCATG AGATGTATTA CTCAACAGCC 8340
   GTCACTGGGA ACATAGTGAA CTCCGTCAAT GTACAGTCGA GGAAACTTTT GGCTCGGTTT 8400
   GGAGACCAGA GAGGGCCAAC CAAGGTGCCT GAACTCGACC TGGGAGTTGG AACGAGGTGT 8460
   GTGGTCTTAG CTGAGGACAA GGTGAAAGAA CAAGACGTAC AAGAGAGGAT CAGAGCGTTG 8520
   CGGGAGCAAT ACAGCGAAAC CTGGCATATG GACGAGGAAC ACCCGTACCG GACATGGCAG 8580
   TACTGGGGCA GCTACCGCAC GGCACCAACC GGCTCGGCGG CGTCACTGAT CAATGGGGTT 8640
   GTGAAACTTC TCAGCTGGCC ATGGAACGCA CGGGAAGATG TGGTGCGCAT GGCTATGACT 8700
   GACACAACGG CTTTCGGACA GCAGAGAGTG TTCAAAGATA AAGTTGACAC AAAGGCACAG 8760
   GAGCCTCAGC CCGGTACAAG AGTCATCATG AGAGCTGTAA ATGATTGGAT TTTGGAACGA 8820
   CTGGCGCAGA AAAGCAAACC GCGCATGTGC AGCAGGGAAG AATTCATAGC AAAAGTGAAA 8880
   TCAAATGCAG CCTTGGGAGC TTGGTCAGAT GAGCAAAACA GATGGGCAAG TGCAAGAGAG 8940
   GCTGTAGAGG ATCCTGCATT CTGGCGCCTC GTGGATGAAG AGAGAGAAAG GCACCTCATG 9000
   GGGAGATGTG CGCACTGCGT GTACAACATG ATGGGCAAGA GAGAAAAGAA ACTGGGAGAG 9060
   TTCGGAGTGG CGAAAGGAAG TCGGGCCATT TGGTACATGT GGCTGGGGAG TCGCTTTTTG 9120
   GAGTTCGAGG CTCTTGGATT CTTGAATGAA GACCATTGGG CCTCTAGAGA GTCCAGTGGA 9180
   GCTGGAGTTG AGGGAATAAG CTTGAACTAC CTGGGCTGGC ACCTCAAGAA GTTGTCAACC 9240
   CTGAATGGAG GACTCTTCTA TGCAGATGAC ACAGCTGGCT GGGACACGAA AGTTACCAAT 9300
   GCAGACTTAG AGGATGAAGA ACAGATCCTA CGGTACATGG AGGGTGAGCA CAAACAATTG 9360
   GCAACCACAA TAATGCAAAA AGCATACCAT GCCAAAGTCG TGAAGGTCGC GAGGCCTTCC 9420
   CGTGATGGAG GCTGCATCAT GGATGTCATC ACAAGAAGAG ACCAAAGAGG TTCGGGTCAG 9480
   GTTGTGACCT ATGCCCTTAA CACCCTCACC AACATAAAGG TGCAATTAAT CCGAATGATG 9540
   GAAGGGGAAG GGGTCATAGA GGCAGCGGAT GCACACAACC CGAGACTGCT TCGAGTGGAG 9600
   CGCTGGCTGA AAGAACACGG AGAAGAGCGT CTTGGAAGAA TGCTCGTCAG TGGTGACGAT 9660
   TGTGTGGTGA GGCCCTTGGA TGACAGATTT GGCAAAGCAC TTTACTTTCT GAATGACATG 9720
   GCCAAGACCA GGAAGGACAT TGGGGAATGG GAGCACTCAG CCGGCTTTTC AAGCTGGGAG 9780
   GAGGTACCCT TTTGTTCACA CCATTTCCAC GAGCTAGTGA TGAAGGATGG ACGCACCCTG 9840
   GTGGTGCCGT GCCGAGACCA AGATGAACTC GTTGGGAGGG CGCGCATCTC ACCGGGGTGC 9900
   GGCTGGAGTG TCCGCGAGAC GGCCTGCCTT TCAAAAGCCT ACGGGCAGAT GTGGCTGCTG 9960
   AGCTACTTCC ACCGACGAGA CCTGAGGACG CTCGGGCTTG CCATTAACTC AGCAGTGCCT 10020
   GCCGATTGGG TTCCTACCGG CCGCACGACG TGGAGCATTC ATGCCAGTGG GGCCTGGATG 10080
   ACCACAGAAG ACATGCTGGA CGTTTGGAAC CGGGTGTGGA TTCTGGACAA CCCTTTCATG 10140
   CAGAACAAGG AAAGGGTCAT GGAGTGGAGG GATGTTCCGT ACCTCCCTAA AGCTCAGGAC 10200
   ATGTTATGTT CCTCCCTTGT TGGGAGGAGA GAAAGAGCAG AATGGGCCAA GAACATCTGG 10260
   GGAGCGGTGG AAAAGGTGAG GAAGATGATA GGTCCTGAAA AGTTCAAGGA CTATCTCTCC 10320
   TGTATGGACC GCCATGACCT GCACTGGGAG CTCAGACTGG AGAGCTCAAT AATCTAAACC 10380
   CAGACTGTGA CAGAGCAAAA CCCGGAAGGC TCGTAAAAGA TTGTCCGGAA CCAAAAGAAA 10440
   AGCAAGCAAC TCACAGAGAT AGAGCTCGGA CTGGAGAGCT CTTTAAACAA AAAAAAAAAA 10500
   AAAAAAAAAA AAAAAAAAAA AAAAAAAAAA AAAAAAAGCC AGAATTGAGC TGAACCTGGA 10560
   GAGCTCATTA AATACAGTCC AGACGAAACA AAACATGACA AAGCAAAGAG GCTGAGCTAA 10620
   AAGTTCCCAC TACGGGACTG CTTCATAGCG GTTTGTGGGG GGAGGCTAGG AGGCGAAGCC 10680
   ACAGATCATG GAATGATGCG GCAGCGCGCG AGAGCGACGG GGAAGTGGTC GCACCCGACG 10740
   CACCATCCAT GAAGCAATAC TTCGTGAGAC CCCCCCTGAC CAGCAAAGGG GGCAGACCGG 10800
   TCAGGGGTGA GGAATGCCCC CAGAGTGCAT TACGGCAGCA CGCCAGTGAG AGTGGCGACG 10860
   GGAAAATGGT CGATCCCGAC GTAGGGCACT CTGAAAAATT TTGTGAGACC CCCTGCATCA 10920
   TGATAAGGCC GAACATGGTG CATGAAAGGG GAGGCCCCCG GAAGCACGCT TCCGGGAGGA 10980
   GGGAAGAGAG AAATTGGCAG CTCTCTTCAG GATTTTTCCT CCTCCTATAC AAAATTCCCC 11040
   CTCGGTAGAG GGGGGGCGGT TCTTGTTCTC CCTGAGCCAC CATCACCCAG ACACAGGTAG 11100
   TCTGACAAGG AGGTGATGTG TGACTCGGAA AAACACCCGC T 11141
(2) ANGABEN ZU SEQ ID NO: 20:
   (i) SEQUENZKENNZEICHEN:
      (A) LÄNGE: 767 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 20:
   TAAACCCAGA CTGTGACAGA GCAAAACCCG GAAGGCTCGT AAAAGATTGT CCGGAACCAA      60
   AAGAAAAGCA AGCAACTCAC AGAGATAGAG CTCGGACTGG AGAGCTCTTT AAACAAAAAA     120
   AAAAAAAAAA AAAAAAAAAA AAAAAAAAAA AAAAAAAAAA AAAGCCAGAA TTGAGCTGAA     180
   CCTGGAGAGC TCATTAAATA CAGTCCAGAC GAAACAAAAC ATGACAAAGC AAAGAGGCTG     240
   AGCTAAAAGT TCCCACTACG GGACTGCTTC ATAGCGGTTT GTGGGGGGAG GCTAGGAGGC     300
   GAAGCCACAG ATCATGGAAT GATGCGGCAG CGCGCGAGAG CGACGGGGAA GTGGTCGCAC     360
   CCGACGCACC ATCCATGAAG CAATACTTCG TGAGACCCCC CCTGACCAGC AAAGGGGGCA     420
   GACCGGTCAG GGGTGAGGAA TGCCCCCAGA GTGCATTACG GCAGCACGCC AGTGAGAGTG     480
   GCGACGGGAA AATGGTCGAT CCCGACGTAG GGCACTCTGA AAAATTTTGT GAGACCCCCT     540
   GCATCATGAT AAGGCCGAAC ATGGTGCATG AAAGGGGAGG CCCCCGGAAG CACGCTTCCG     600
   GGAGGAGGGA AGAGAGAAAT TGGCAGCTCT CTTCAGGATT TTTCCTCCTC CTATACAAAA     660
   TTCCCCCTCG GTAGAGGGGG GGCGGTTCTT GTTCTCCCTG AGCCACCATC ACCCAGACAC     720
   AGGTAGTCTG ACAAGGAGGT GATGTGTGAC TCGGAAAAAC ACCCGCT                   767

## Beschreibung

Die vorliegende Erfindung betrifft eine in vivo replikationsfähige infektiöse Nukleinsäure kodierend für Tick-Borne Enzephalitis (TBE)-Virus, zur pharmazeutischen Verwendung. Insbesondere betrifft die Erfindung eine komplette CDNA, die für ein infektiöses TBEV-RNA-Transkript kodiert zur Verwendung als pharmazeutisches Produkt.

Ein weiterer Aspekt der vorliegenden Erfindung betrifft die Verwendung einer replizierbaren Nukleinsäure , die für ein komplettes TBE Virus oder ein TBE-Viruspartikel kodiert zur Therapie und Prophylaxe von Vertebraten.

Das Tick-Borne Enzephalitis (TBE)-Virus ist ein Vertreter der Familie Flaviviridae. Diese Virusfamilie gliedert sich in drei Genera, nämlich das Genus Pestiviren, das Genus Hepatitis C-Viren und das Genus Flaviviren. Der Begriff 'Flaviviren' wird hier stets synonym für das Genus, nicht die Familie, verwendet.

Die Flaviviren umfassen ungefähr 70 bekannte Vertreter, von denen einige die Erreger bedeutender Erkrankungen des Menschen sind. Dazu zählen insbesondere das Gelbfieber-Virus, die Dengue-Viren (Typ 1-4), das Japan B-Virus und das TBE-Virus. Während die erstgenannten Viren sowie die Mehrzahl der übrigen Flaviviren durch Stechmücken auf den Menschen bzw. verschiedene Wirbeltiere übertragen werden, bilden die 'Tick-Borne', also durch Zecken übertragenen TBE-Viren eine biologisch-ökologisch und epidemiologisch distinkte Untergruppe innerhalb der Flaviviren. Die TBE-Viren unterscheiden sich auch in bestimmten molekularbiologischen, genetischen Eigenschaften von den anderen Flaviviren.

Das Genom der Flaviviren ist ein einzelsträngiges, ungefähr 11.000 Nukleotide langes RNA-Molekül. Dieses Molekül ist positiv-strangig, d.h. es entspricht in seiner Orientierung einer mRNA. Von diesem RNA-Molekül werden alle viralen Proteine an den Ribosomen abgelesen (translatiert). Das RNA-Genom der Flaviviren ist infektiös, d.h. wird virale RNA ohne irgendwelche andere Komponenten des Viruspartikels mit geeigneten Methoden in infizierbare Zellen eingebracht, so führt dies zu einer produktiven Virusinfektion, also der Bildung neuer, infektiöser Viruspartikel.

Gegen eine Reihe von durch Flaviviren verursachte Erkrankungen befinden sich Impfstoffe in Verwendung oder Entwicklung. Prinzipiell können zwei verschiedene Arten von Impfstoffen unterschieden werden, nämlich Lebend- und Totimpfstoffe.

Lebendimpfstoffe sind in ihrer Virulenz verminderte (= attenuierte) Virusstämme, die kein oder nur ein sehr schwaches Krankheitsbild hervorrufen (apathogen sind), aber eine auch gegen das virulente Wildvirus schützende Immunantwort bewirken. Bei den meisten bis heute verwendeten Lebendimpfstoffen handelt es sich entweder um in der Natur vorkommende Viren (z.B. Vacciniavirus) oder um Virusstämme, die im Labor durch wiederholtes Passagieren in verschiedenen biologischen Systemen verändert wurden (z.B. Polio-, Gelbfieberviren). Bei Flaviviren ist ein Lebendimpfstoff gegen des Gelbfieber-Virus seit den 30er Jahren in Verwendung. Lebendimpfstoffe gegen die vier Dengue Virustypen befinden sich in klinischer Erprobung.

Bei Totimpfstoffen handelt es sich um ursprünglich virulente Viren, die in geeigneten biologischen Systemen gezüchtet und dann durch ein chemisches Verfahren (z.B. Behandlung mit Formaldehyd) inaktiviert werden. Totimpfstoffe erfordern bei ihrer Produktion das Arbeiten mit grossen Mengen von virulenten, pathogenen Viren. Durch die Inaktivierung können Struktureigenschaften des Viruspartikels verändert werden. Dies kann zu einer weniger spezifischen und weniger effizienten Immunantwort führen. Bei der Verwendung von Totimpfstoffen kommt es zu keiner Infektion des Geimpften, es wird aber eine Immunantwort induziert, die auch gegen replikationskompetentes, virulentes Virus schützt. Solche Totimpfstoffe sind gegen das TBE-Virus und gegen das Japan B-Virus in Verwendung (Monath, T. 1990).

Im Hinblick auf die Impfstoffherstellung ist bei RNA-Viren deren hohe Mutationsfrequenz zu berücksichtigen. Aufgrund dieser hohen Mutationsfrequenz ist ein zur Produktion eines Impfstoffes verwendetes Saatvirus immer heterogen und kann sich durch Passagierung ebenfalls ständig verändern. Dadurch kann es zu Veränderungen der Eigenschaften des Virus kommen, wodurch sich produktionstechnische und sicherheitstechnische Probleme ergeben können. Verschiedene in Verwendung befindliche Saatviren des Gelbfieber 17D-Impfstoffes weisen bereits erhebliche Sequenzunterschiede auf, die auch biologische Unterschiede vermuten lassen (Post et al, 1992; Ledger et al, 1992).

Aufgrund des Vorhergesagten ergibt sich ohne weiteres, dass es vorteilhaft ist, bei der Impfstoffherstellung anstelle eines heterogenen Saatvirus von einem einheitlichen cDNA-Klon auszugehen. Darüber hinaus ist es von Vorteil, auch für die Herstellung eines Totimpfstoffes einen spezifischen attenuierten Virusstamm einzusetzen.

Ohne einen infektiösen cDNA-Klon können bei RNA-Viren attenuierte Virusstämme nur entweder zufällig aus der Natur isoliert werden, oder sie werden durch Selektion zufälliger Mutanten durch wiederholte Passagierung im Labor gezüchtet. Auf Basis dieser Virusstämme kann man das molekulare Prinzip der Attenuierung, ihr Ausmass und die Wahrscheinlichkeit einer Reversion zum virulenten Wildtyp (letzteres stellt den wichtigsten Sicherheitsfaktor eines Lebendimpfstoffes dar) praktisch nicht beeinflussen. Als Folge davon gibt es derzeit keinen Stamm des TBE-Virus, der als Lebendimpfstoff geeignet ist.

Im Hinblick auf eine gentechnologische Veränderung und Gewinnung verschiedener Flaviviren ist bekannt, dass es schwierig bis unmöglich ist, ein cDNA-Molekül mit der gesamten Information eines Flavivirus-Genoms stabil z.B. in E. coli zu klonieren. Bei diesen Arbeiten wurde ausserdem beobachtet, dass spontan Mutationen auftreten, die dazu führen, dass keine infektiöse RNA gewonnen werden kann. Man nimmt an, dass bestimmte Sequenzelemente für die Bakterien, in denen die cDNA-Klone vermehrt werden sollen, toxisch sind. Hierbei sind die auftretenden Probleme jedoch sehr unterschiedlich: Während für Dengue-4 (Lai et al, 1991) und Japan B-Virus (Sumiyoshi et al, 1992) geeignete Vektorsysteme gefunden werden konnten, um komplette cDNA-Templates herzustellen, konnte dieses Problem für Gelbfieber-Virus nur durch Klonierung von drei einzelnen DNA-Fragmenten überwunden werden (Rice et al, 1989). Im übrigen konnten für Dengue-2 und andere Flaviviren bis heute keine geeigneten Vektorsysteme gefunden werden. Die genauen Ursachen dieser Stabilitätsprobleme sind unbekannt. Aus diesem Grunde lassen sich die Ergebnisse, die bei der Herstellung eines infektiösen Klons eines bestimmten Flavivirus erhalten werden, nicht auf andere Flaviviren übertragen.

Insbesondere war es für das TBE-Virus bisher nicht bekannt, in welchem Vektorsystem, in welcher Orientierung und unter Verwendung welcher DNA-Sequenzen und Wirtszellen ein infektiöser TBEV-Klon generiert werden kann.

Aufgabe der vorliegenden Erfindung ist die Bereitstellung eines infektiösen TBEV-Klons als Ausgangsmaterial zur pharmazeutischen Verwendung.

'Infektiöser Klon' bedeutet hier, dass das Genom des Virus in Form von cDNA mit Hilfe gentechnologischer Methoden in ein Vektorsystem eingebracht wird, das es erlaubt, gezielte Veränderungen an diesem Genom vorzunehmen und durch entsprechende Methoden wieder Viren mit gegebenenfalls veränderten Eigenschaften herzustellen. Es ist bekannt, dass bei komplexen Genomen und insbesondere bei RNA-Genomen, hierbei eine Vielzahl von Problemen zu überwinden sind.

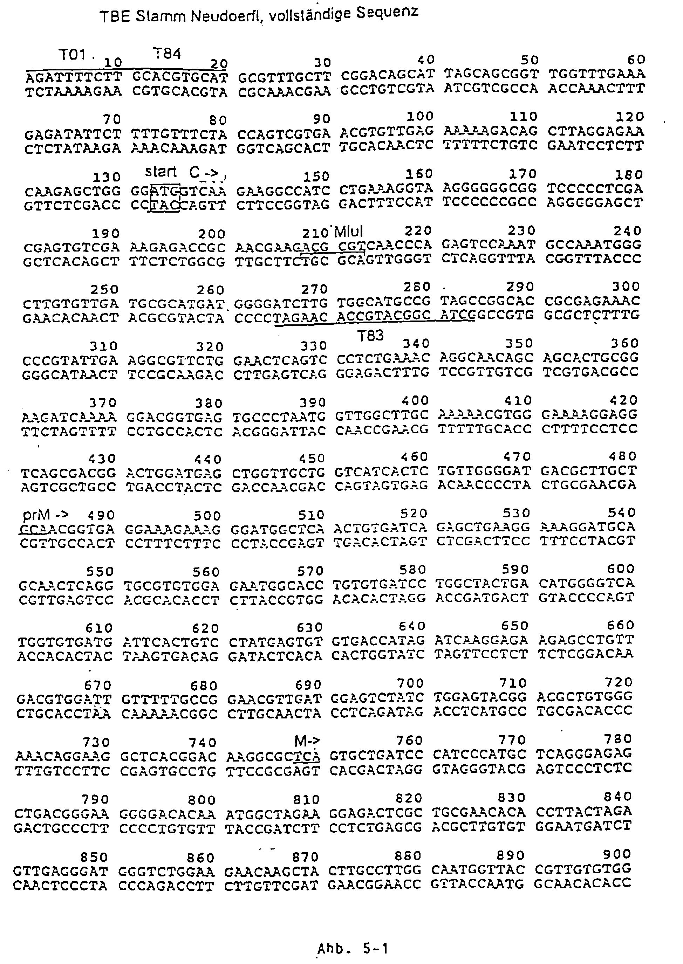

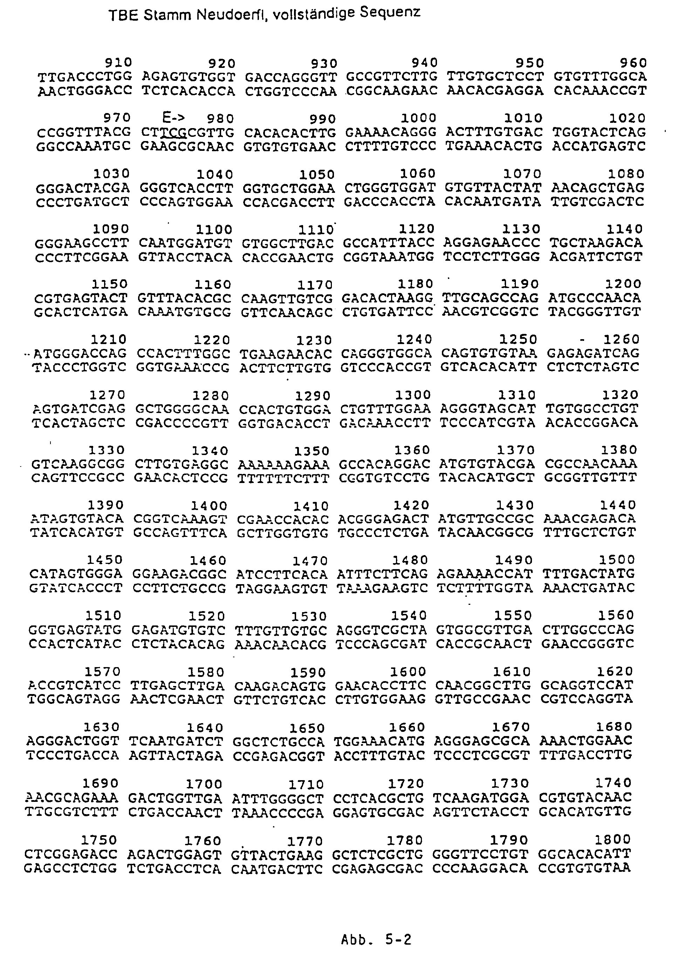

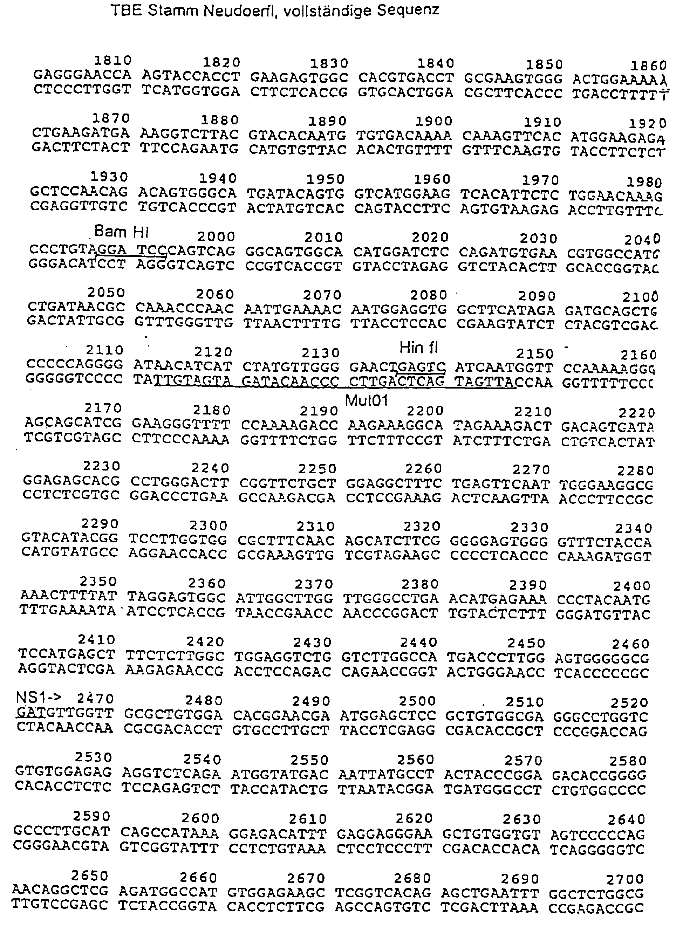

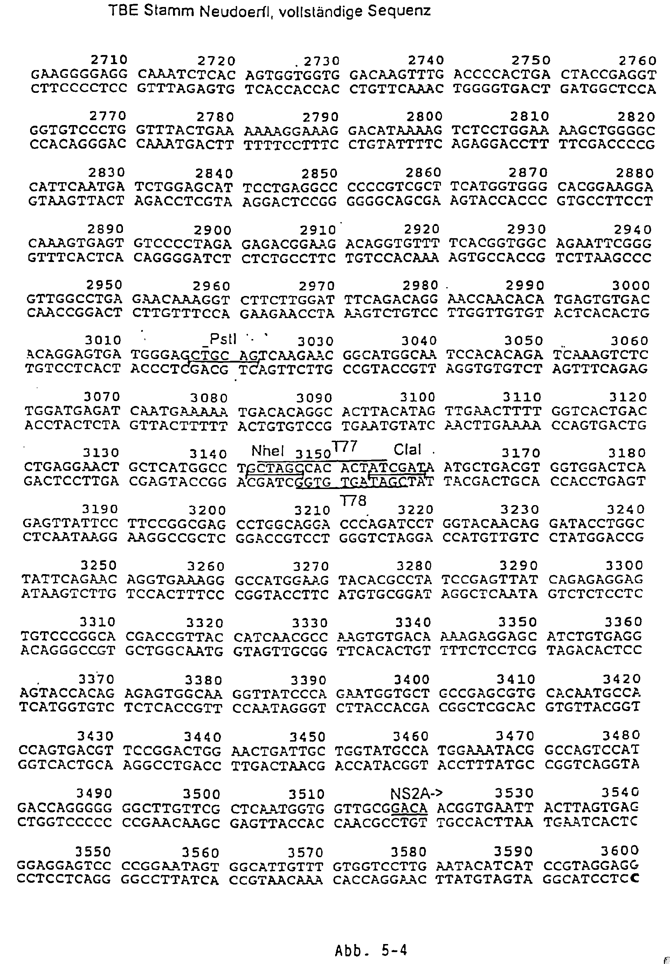

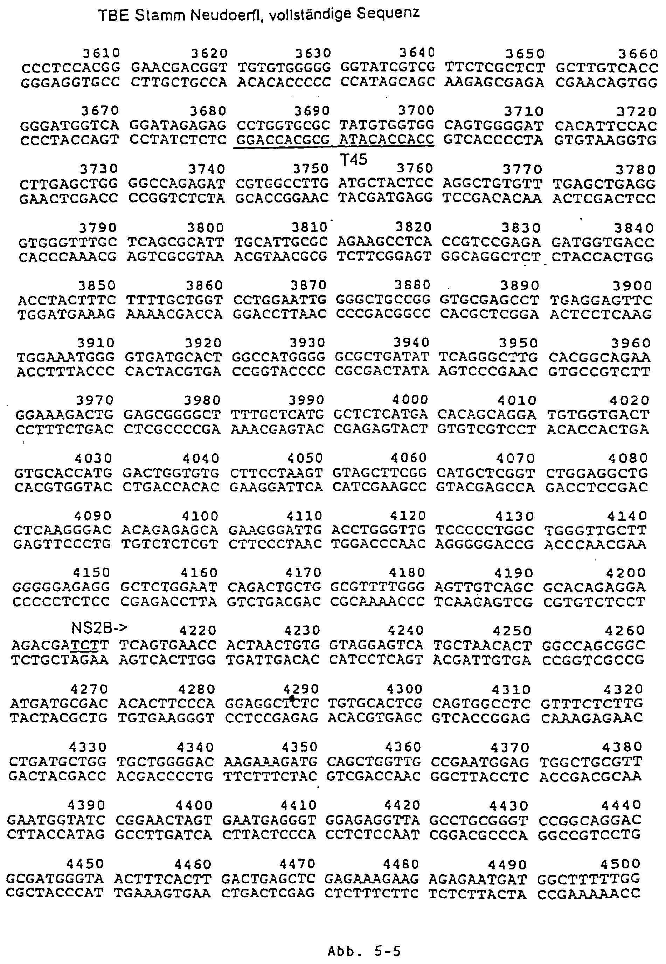

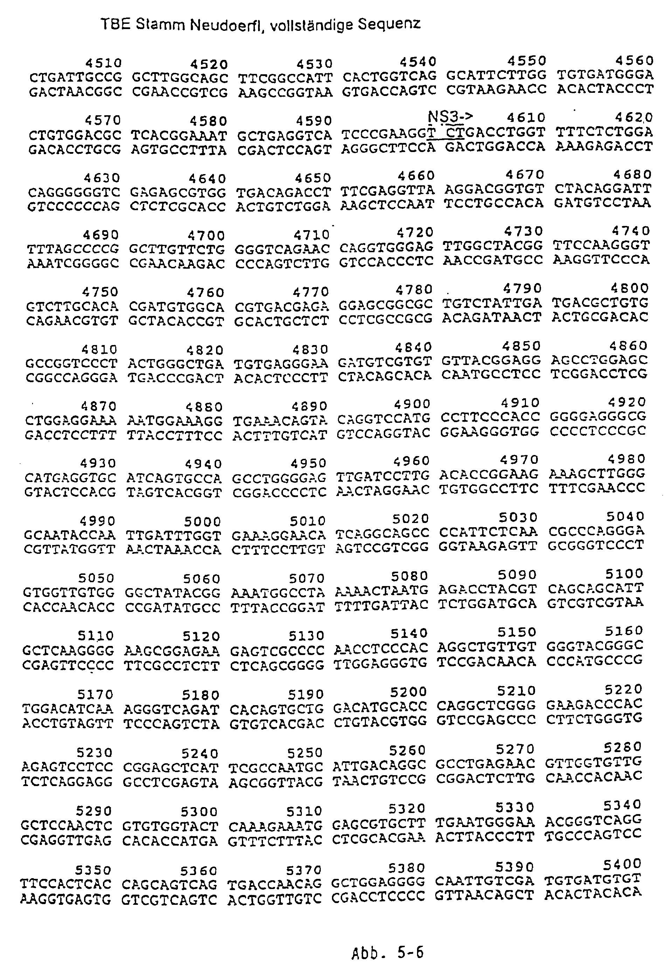

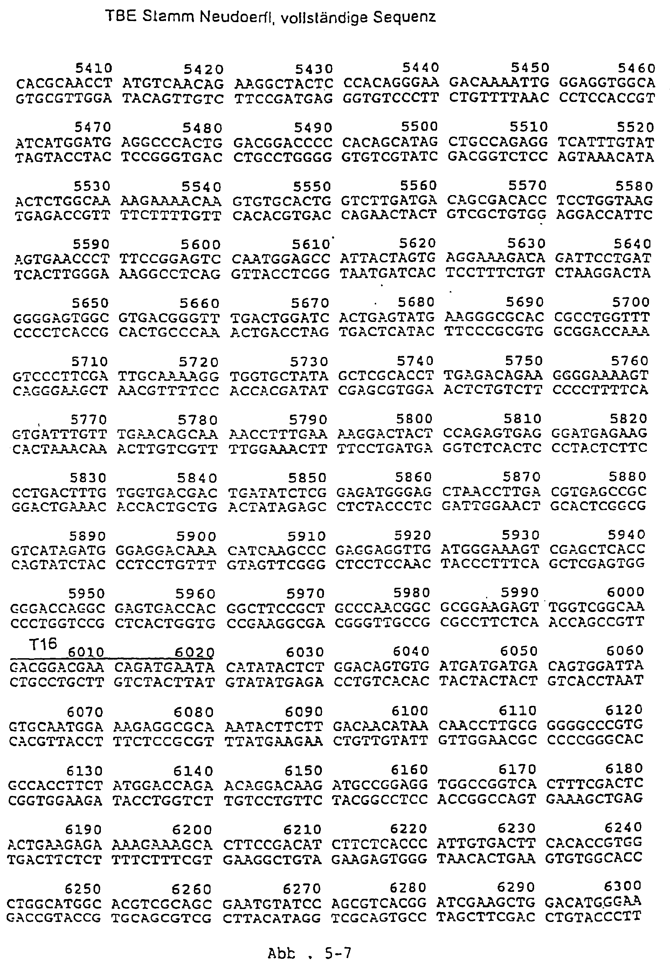

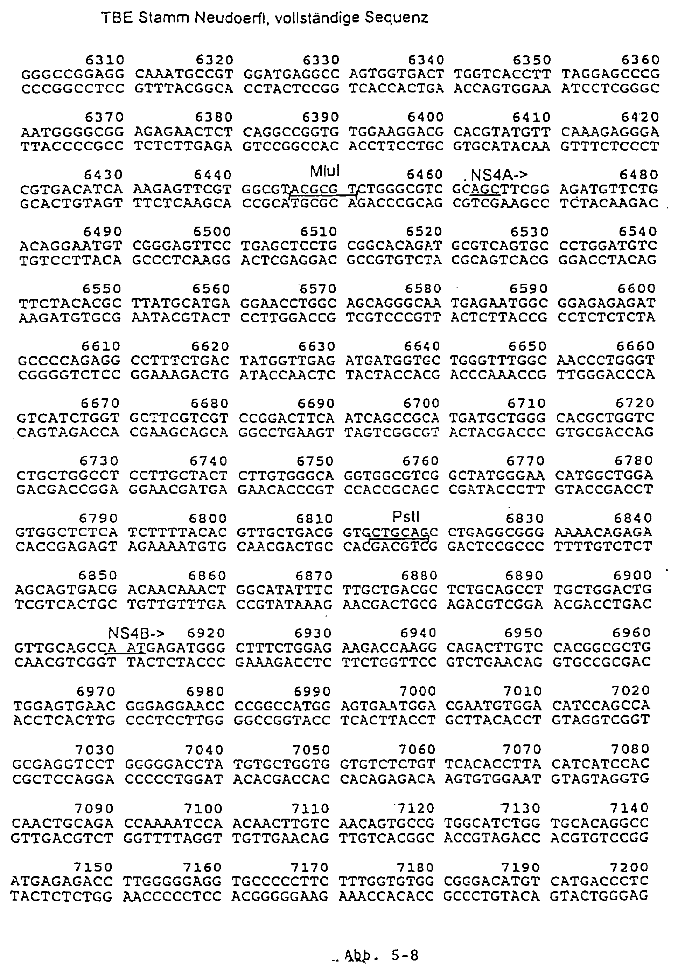

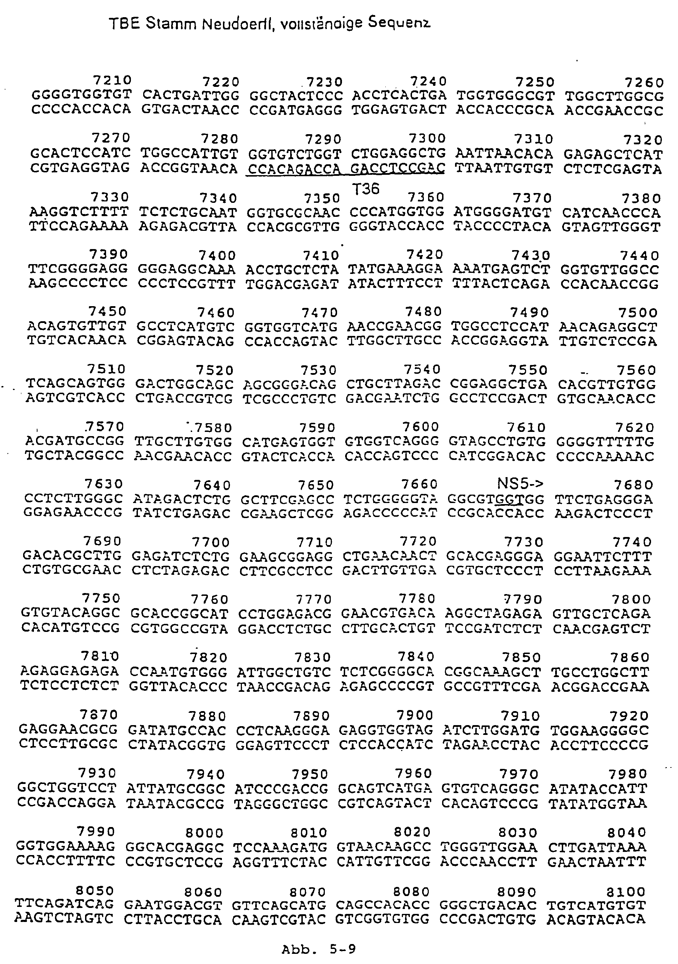

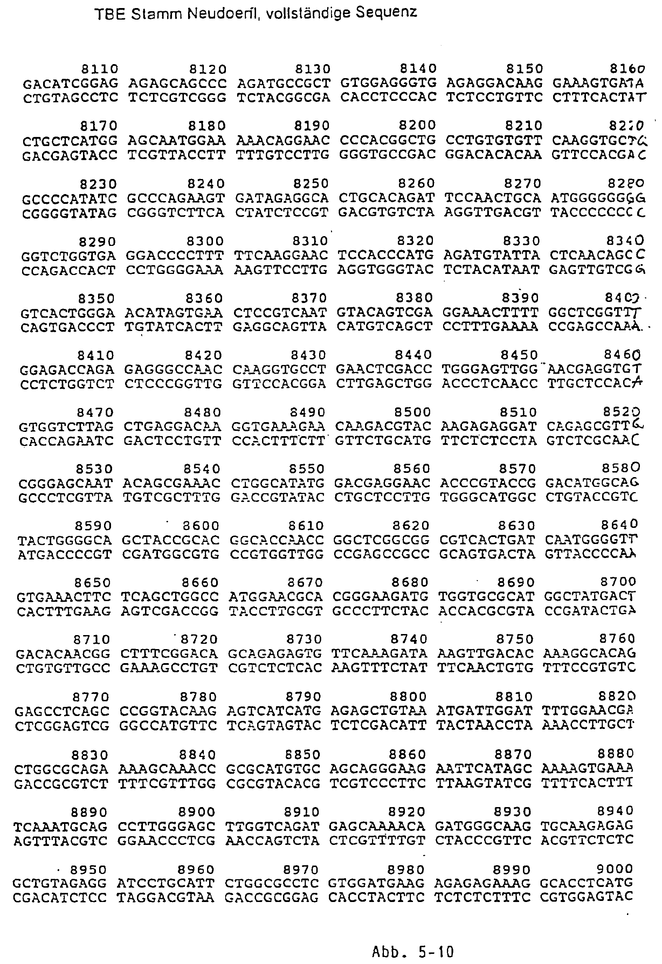

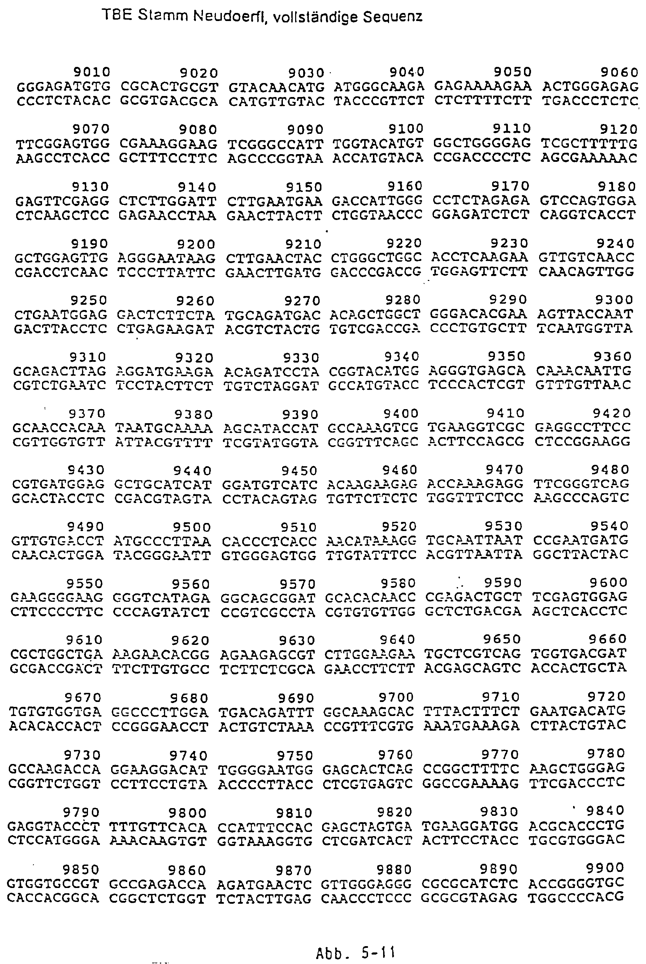

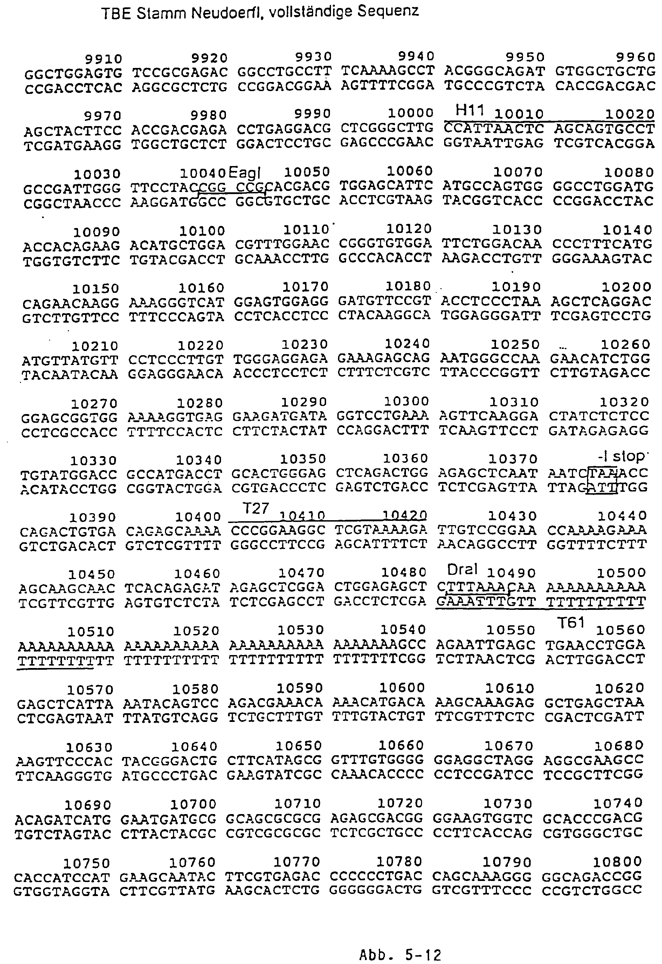

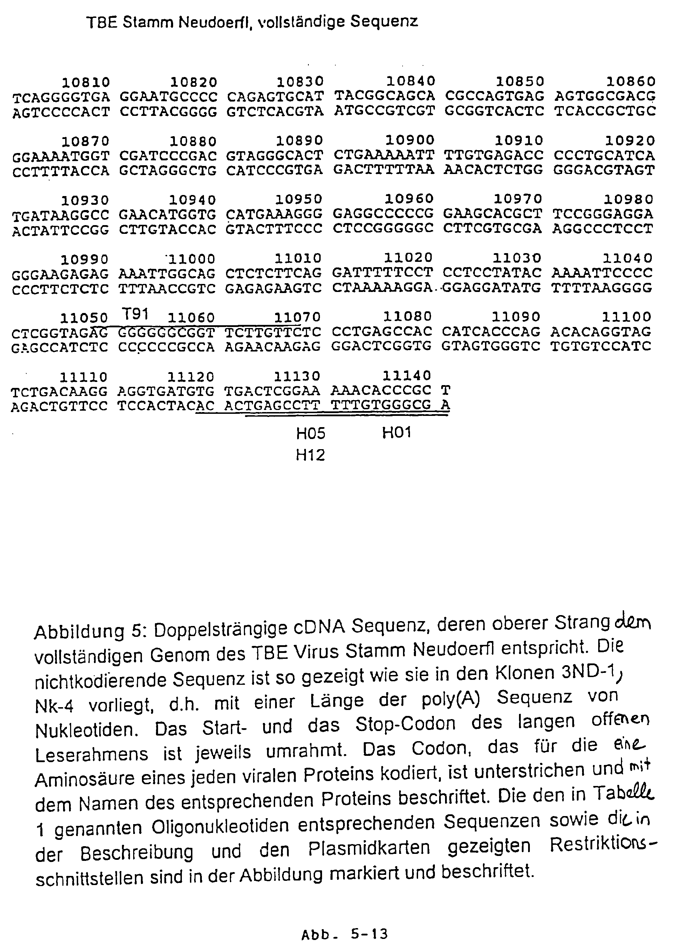

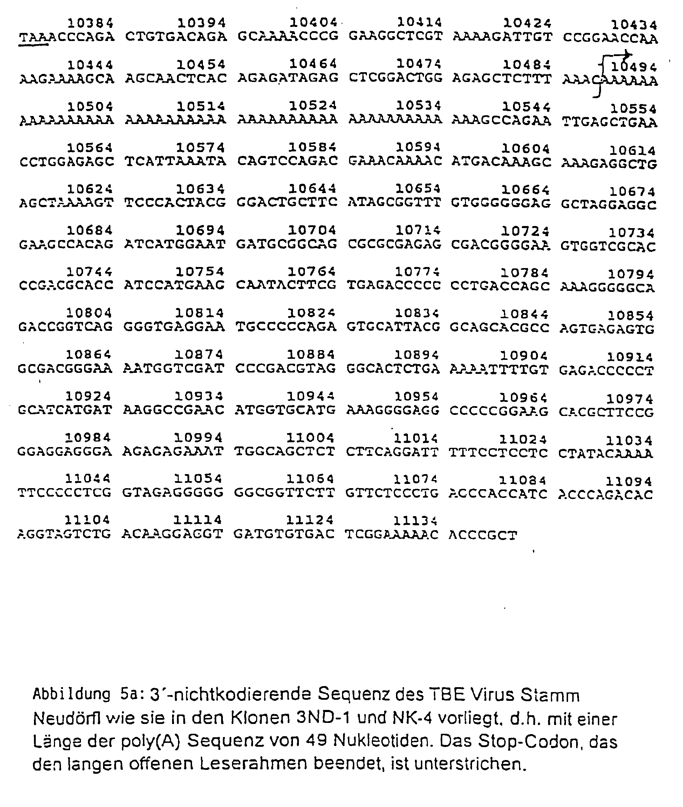

Die vorliegende Erfindung stellt eine komplette Nukleinsäure zur Verfügung die für ein infektiöses TBEV-RNA-Transkript kodiert und gekennzeichnet ist durch
a) die Sequenz gemäss Fig. 5, sowie
b) Sequenzen, die mit der Gesamtsequenz von Fig. 5 zu 70% homolog sind. Nach einer besonderen Ausführungsform beträgt diese Homologie 80 und bevorzugt 95%.

Die Nukleinsäuresequenz gemäss Fig. 5 stellt die komplette cDNA des TBE-Virus vom Stamm Neudörfl dar. Diese Gesamtsequenz einschliesslich der 5'- und 3'-nicht-kodierenden Nukleotidsequenz dieses Stammes bildet eine wesentliche Voraussetzung für das erfindungsgemässe Verfahren zur Gewinnung eines infektiösen TBEV-Klons. Die im Stand der Technik bisher veröffentlichte Sequenz umfasste lediglich eine Teilsequenz, welche bis zum Nukleotid 10488 reichte. Die Sequenz, beginnend mit Poly(A) an Position 10489 ist neu und komplettiert erstmals die Nukleinsäuresequenz, welche für ein infektiöses TBEV kodiert.

Die schnelle Entwicklung gentechnologischer Methoden der letzten Jahre hat es in zunehmendem Masse möglich gemacht, Nukleinsäuren gezielt zu verändern. Die bekannten Methoden der gezielten genetischen Veränderung beschränken sich aber auf doppelsträngige DNA-Moleküle. Viren mit relativ kleinen Genomen aus doppelsträngiger DNA, wie beispielsweise die Polyomaviren, konnten deshalb schon sehr bald gezielt genetisch verändert werden: Das Genom wurde direkt in ein geeignetes bakterielles Vektorsystem kloniert, verändere und in seiner veränderten Form in Wirtszellen gebracht, wo es zur Bildung der gewünschten Virusmutante führte (Shah, 1990).

Im Gegensatz zu DNA-Viren müssen bei RNA-Viren wesentlich aufwendigere Wege beschritten werden. Zunächst muss eine doppelsträngige Kopie (cDNA) des RNA-Genoms hergestellt werden. Diese cDNA wird dann als Ganzes oder in Fragmenten in geeigneten bakteriellen Systemen kloniert, analysiert und gezielt verändert. Von dieser DNA wird dann in vitro oder in einer Zelle eine positiv-strängige RNA-Kopie generiert, die zur Bildung des gewünschten, gezielt abgewandelten Virus führt. Dabei ist Voraussetzung, dass ein solcher infektiöser Klon alle genetischen Elemente, die für die Replikation des Virus notwendig sind, umfasst. Das Fehlen eines einzigen Nukleotids oder der Einbau eines einzigen falschen Nukleotids kann dazu führen, dass kein infektiöses Virus generiert wird. Erstmals ist es auf der Basis der in Fig. 5 zur Verfügung gestellten DNA-Sequenz des Stammes Neudörfl möglich, zu einem infektiösen Klon zu gelangen, der es ermöglicht, das Genom eines TBE-Virus gezielt zu verändern. Dies erfolgt, indem die klonierte cDNA in infektiöse RNA überführt wird. Dieser Vorgang kann entweder in der Zelle oder in vitro stattfinden. Es ist dabei erforderlich, dass dieser Transkriptionsschritt unter der Kontrolle eines geeigneten Promotor/Enzym-Systems steht, welches erlaubt, den gesamten cDNA-Klon in RNA zu übersetzen. Die häufig verwendeten RNA-Polymerasen, wie z.B. SP6, T3, T7, unterscheiden sich hinsichtlich ihrer Fähigkeit, lange Transkripte herzustellen; sie besitzen die Eigenschaft, die Tanskription bevorzugt an für jedes Enzym verschiedenen Stellen abzubrechen. Auch diesbezüglich war bisher nicht bekannt, welches Enzym in der Lage ist, einen cDNA-Klon des TBE-Genoms in eine vollständige RNA zu transkribieren.

Wie bereits vorstehend ausgeführt, ist es möglich, die cDNA gezielt durch Mutationen in ihrem kodierenden oder nicht-kodierenden Bereich zu verändern. Auf diese Weise können die Virulenz oder die Ausbeute des Virus beeinflusst werden. Die Erfindung stellt somit für TBEV kodierende cDNA-Sequenzen zur Verfügung, die durch Substitution, Insertion oder Deletion so verändert sind, dass ein in seiner Virulenz vermindertes TBEV produziert wird, oder dass dadurch die Replikationsrate von TBEV beeinflusst wird.

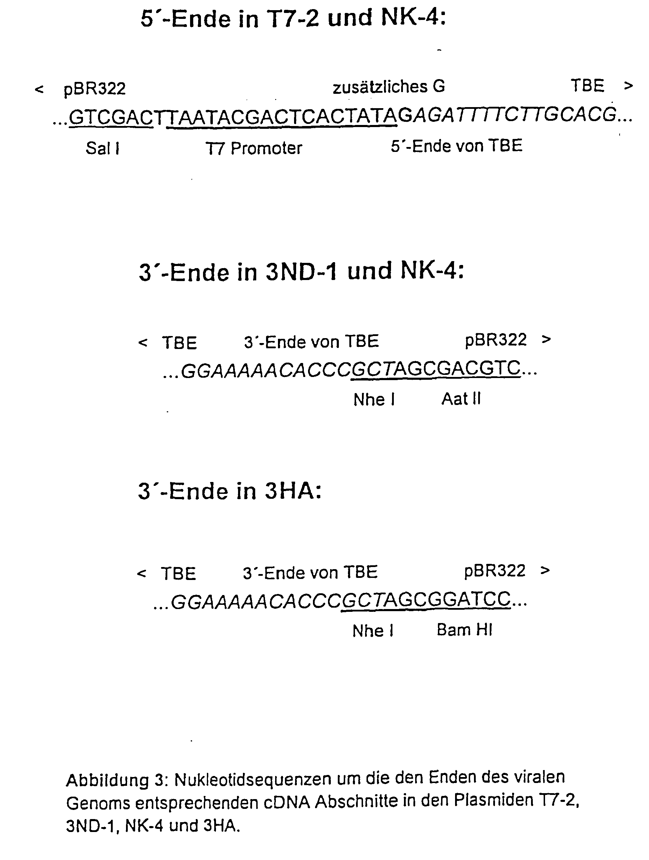

Nach einer besonderen Ausführungsform wird eine cDNA zur Verfügung gestellt, die sich durch eine Mutation am 5'-Ende auszeichnet. Das 5'-Ende der Flaviviren trägt eine CAP-Struktur am ersten Nukleotid, einem A (Mandl et al, 1989). Eine solche Struktur wird mit den gängigen RNA-Polymerasen nur mit schlechter Effizienz gebildet. Erfindungsgemäss wird die cDNA gemäss Fig. 5 so verändert (Fig. 3), dass das CAP an ein 5'-terminales G angehängt wird. Auf diese Weise entsteht eine RNA, die gegenüber der nativen viralen RNA um ein Nukleotid verlängert ist. Diese gezielte Veränderung der cDNA führt dazu, dass infektiöse RNA in wesentlich höheren Ausbeuten gebildet wird.

Weitere Modifizierungen der für TBEV kodierenden cDNA können an der pr(M)-Spaltstelle durchgeführt werden. Die Spaltung des pr(M)-Proteins zu dem in reifen Virionen vorhandenen M-Protein ist für die Infektiosität des TBE-Virus notwendig. Verminderung der Effizienz dieser Spaltung führt zu einer Attenuierung des Virus.

Eine weitere Mutation, die zu einer herabgesetzten Virulenz von TBEV führt, besteht in der Entfernung einer Glykosylierungsstelle, wie z.B. durch Mutation an der entsprechenden N-Glykosylierungsstelle des E-Proteins.

Eine weitere Mutation kann durch Veränderung der Aminosäure 384 des E-Proteins erfolgen, wie dies z.B. in der Mutante B4 (Holzmann et al, 1990) vorliegt.

Ein weiteres attenuierendes Prinzip für TBE-Virus besteht im Einbau von einer oder mehreren Mutationen, entsprechend der Sequenz des TBE-Virusisolats 263. Bei diesem Stamm handelt es sich um ein natürlich vorkommendes TBE-Virus mit attenuierten Eigenschaften, dessen Genomsequenz sich an den in Tabelle 3 gezeigten Stellen von der des TBE-Virus Stamm Neudörfl unterscheidet.

Durch Mutationen an der für TBEV kodierenden cDNA, wie sie beispielhaft oben angeführt werden, ist es möglich, ein attenuiertes Virus zu gewinnen, welches sich als Lebendimpfstoff eignet. Durch die Kombination von mindestens zwei attenuierenden Prinzipien kann der Grad der Attenuierung auf das gewünschte Mass erhöht und die Sicherheit multiplikativ erhöht werden (die Wahrscheinlichkeit der Reversion zum virulenten Wildtyp bei zwei unabhängigen attenuierten Mutationen entspricht dem Produkt der Einzelwahrscheinlichkeiten). Die Reversionshäufigkeit von einzelnen Aminosäuremutationen kann durch Ausnutzung der degenerierten Natur des genetischen Codes durch Veränderung von zwei Basen der entsprechenden Codons stark eingeschränkt werden.

Die Erfindung stellt somit die Möglichkeit zur Verfügung, anstelle von zufällig aus der Natur isolierten attenuierten Virusstämmen, das molekulare Prinzip der Attenuierung, ihr Ausmass und die Wahrscheinlichkeit der Reversion zum virulenten Wildtyp gezielt zu beeinflussen. Sie stellt damit erstmals TBEV-Mutanten zur Verfügung, die als Lebendimpfstoff geeignet sind.

Darüber hinaus ist es aber gemäss der Erfindung auch möglich, die terminalen nicht-kodierenden (NC) Regionen des TBE-Virus, gezielt zu beeinflussen. Diese nicht-kodierenden Genomabschnitte viraler RNA enthalten meist besonders wichtige regulatorische Elemente. Diese Elemente bestimmen die Replikation des viralen Genoms, beeinflussen die Bildung viraler Proteine und sind wahrscheinlich erforderlich, um die Verpackung viraler Genome in Virionen zu gewährleisten. Die biologischen Eigenschaften eines Virus, einschliesslich seiner Virulenz und Pathogenität werden somit ganz entscheidend von den NC-Genomabschnitten beeinflusst. Es ist deshalb für das Funktionieren eines infektiösen TBEV von besondere Bedeutung, dass diese NC-Genomabschnitte korrekt und genetisch stabil in die für TBEV kodierende cDNA eingebaut werden.

Der Vergleich etlicher Stechmücken-übertragener Flaviviren ergab, dass sowohl die 5'- als auch die 3'-terminalen NC-Genomabschnitte bestimmte zwischen diesen Viren konservierte Sequenzelemente enthalten. Die Analyse der Genomsequenzen von TBE-Viren zeigt in diesen Bereichen aber grosse Abweichungen von den Verhältnissen, wie sie für Stechmücken-übertragene Viren gefunden wurden. Diese Abweichungen können wie folgt zusammengefasst werden:
i) 5'-terminale Sekundärstrukturen sind innerhalb der TBE-Viren nicht konserviert und unterscheiden sich von den konservierten Sekundärstrukturen von Steckmückenübertragenen Viren;
ii) während Stechmücken-übertragene Flaviviren ein zweites Startcodon aufweisen, kommt ein solches bei TBE-Viren nicht vor;
iii) während Stechmücken-übertragene Flaviviren in der 3'-NC-Region konservierte Sequenzmotive aufweisen, fehlen diese bei TBE-Viren völlig;
iv) eine potentielle Zirkularisierungssequenz der Stechmücken-übertragenen Flaviviren hat kein Homolog bei TBE-Viren.
v) Die 3'-NC-Region des TBE-Virus zeigt selbst bei nahe verwandten Stämmen eine ausserordentliche Variabilität, wie sie sonst bei keinem anderen Flavivirus gefunden wurde. So besitzt z.B. der Stamm Neudorfl eine fast doppelt so lange 3'-NC-Region (siehe Fig. 5a) wie der Stamm Hypr. Ersterer weist Sequenzelemente auf, die bei anderen Stämmen und anderen Flaviviren nicht vorkommen. Eines dieser Elemente ist ein poly(A)-Motiv, dessen Länge zwischen 6 As und mehreren hundert As variieren kann. Eine derartige Struktur wurde bei Steckmückenübertragenen Flaviviren nie aufgefunden.

Die Erfindung umfasst die erstmals zur Verfügung gestellte 3'-NC-Region des Stammes Neudörfl, wie sie in Fig. 5a wiedergegeben ist, sowie solche 3'-NC-Sequenzen, die der von Fig. 5a zu 65%, vorzugsweise zu 80% homolog sind. Der Verwendung dieser 3'-NC-Sequenzen kommt besondere Bedeutung zu für die Herstellung von Impfstoffen gegen Varianten und Subtypen von TBEV.

Das TBEV kommt in der Natur bekanntlich in verschiedenen Varianten vor. Insbesondere findet man im Osten Russlands und in China ein als fernöstlicher Subtyp bezeichnetes TBE-Virus, das mit dem Auftreten eines wesentlich stärkeren Krankheitsbildes mit höherer Morbidität und Letalität verbunden ist. Andererseits weisen diese Subtypen, wie auch viele andere Stämme des TBE-Virus sowie nahe verwandte andere TBE-Viren (z.B. Looping III, Turkish Encephalitis Virus, Omsk Hemorrhagic Fever Virus) mehr oder weniger starke Abweichungen ihrer Antigenstrukturen von jenen des Stammes Neudörfl auf. Für eine optimale protektive Wirkung sollten aber die antigenen Eigenschaften eines Impfstoffes mit jenen der in der entsprechenden Region zirkulierenden Viren möglichst übereinstimmen. Da diese Stämme aber teilweise unterschiedliche Virulenzeigenschaften aufweisen und sich im Aufbau ihrer 3'-NC-Region signifikant voneinander unterscheiden können, ist es problematisch, für jeden dieser Stämme attenuierte Impfstoffe auf der Basis spezifischer eigener infektiöser Klone herzustellen.

Durch die vorliegende Erfindung ist es möglich, Sequenzen, die für Proteine von verschiedenen Stämmen, Subtypen oder nahen verwandten TBE-Viren kodieren, mit der 3'-NC-Region des Stammes Neudörfl gemäss Fig. 5a oder einer leicht davon abgewandelten Sequenz, zu kombinieren. Auf diese Weise gelingt es, attenuierte Impfstoffe mit den gewünschten antigenen Eigenschaften herzustellen. Die Erfindung umfasst somit für ein infektiöses TBEV kodierende Nukleinsäuren, welche neben der kodierenden Sequenz eines beliebigen TBEV-Stammes oder Subtyps die 3'-nicht-kodierende Sequenz gemäss Fig. 5a umfassen.

Ausserdem sollen gemäss der Erfindung auch für infektiöses TBEV kodierende Nukleinsäuren umfasst sein, die sich aus der kodierenden Sequenz nach Fig. 5 und einer 3'-nicht-kodierenden Sequenz eines verwandten oder eines anderen TBEV oder Flavivirus zusammensetzen. Besonders bevorzugt sind solche Konstrukte, die die TBEV-kodierende Sequenz nach Fig. 5 und die 3'-nicht-kodierende Sequenz des TBEV-Stammes Hypr umfassen.

Die Erfindung schliesst sämtliche infektiösen TBEV-Klone mit ein, die sich als RNA-Transkript aus den vorstehend genannten Sequenzen ableiten.

Im weiteren umfasst die Erfindung solche cDNA-Konstrukte, die durch Einbau einer für einen Immunmodulator kodierenden Nukleotidsequenz zu einem infektiösen Klon mit einer effektiveren Immunantwort führen. Aus gesundheitspolitischen, organisatorischen und kostentechnischen Gründen ist es wünschenswert, dass ein Impfstoff eine lang anhaltende und breite Immunantwort induziert. Bei vielen derzeit in Verwendung stehenden Vakzinen sind häufige Auffrisch-Impfungen nötig, und die Immunisierung gegen verschiedene Viren kann nicht durch einen einzigen Impfstoff erfolgen. In die 3'-NC-Region können zusätzliche genetische Informationen eingebracht werden, wodurch die Immunantwort moduliert oder stimuliert werden kann. Dies ist auch möglich, wenn man Teile der 3'-NC-Region durch entsprechende, für einen Immunmodulator kodierende Sequenzen ersetzt. Derartige, für einen Immunmodulator kodierende Sequenzen können ausgewählt werden aus DNA-Sequenzen, die für Interleukine, Diphtherietoxin, Choleratoxin, E. coli-hitzelabilem Toxin und Pertussistoxin kodieren. Auf diese Weise kann die Effizienz einer Vakzine erhöht und/oder eine länger wirkende Immunantwort erzielt bzw, die Breite der Immunantwort (z.B. gegen mehrere Varianten des TBE-Virus) erweitert werden.

Die Herstellung des erfindungsgemäss geschützten infektiosen TBEV-Klons wird durch die nachfolgende Beschreibung sowie die Ausführungsbeispiele und die sich anschliessenden Abbildungen und Tabellen näher erläutert:
- Abb. 1: zeigt die Plasmidkarte des Vektorplasmids pBR322 mit für die Konstruktion der beschriebenen Klone relevanten Restriktionsschnittstellen.
- Abb. 2: zeigt die Plasmidkarte des Klons T7-2 mit den relevanten Restriktionsschnittstellen.
- Abb. 3: zeigt die Nukleotidsequenzen der 5'- und 3'-terminalen Enden der Klone T7-2, NK-4, 3ND-1 und 3HA.

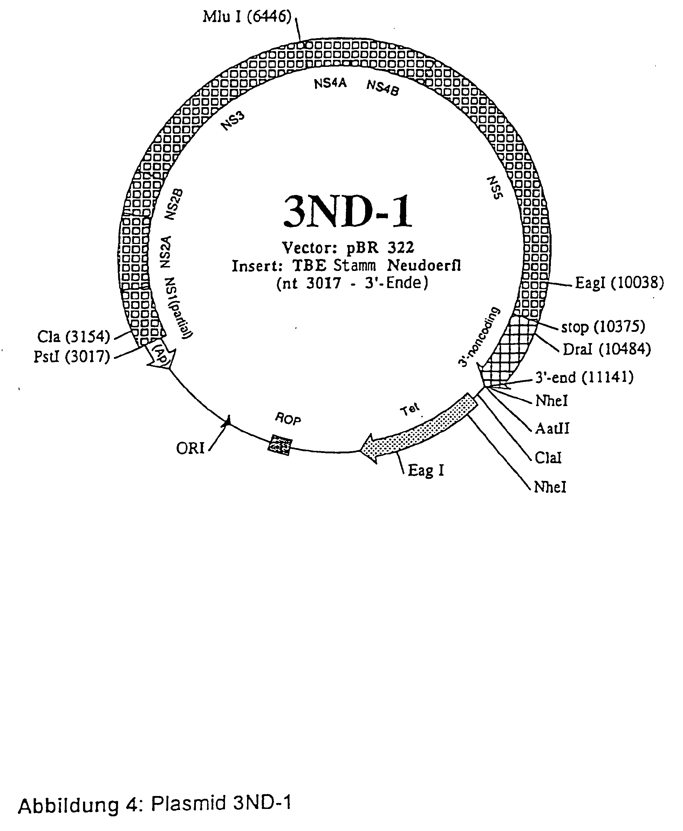

- Abb. 4: zeigt die Plasmidkarte des Klons 3ND-1, mit den relevanten Restriktionsschnittstellen.
- Abb. 5: zeigt die doppelsträngige komplette cDNA-Sequenz des TBEV Stamm Neudörfl Genoms einschliesslich der 3'-nicht-kodierenden Sequenz, wie sie in den Klonen 3ND-1 und NK-4 mit einer poly(A)-Länge von 49 Nukleotiden vorliegt. Das Stop-Codon, das den offenen Leserahmen terminiert, ist unterstrichen.
- Abb. 5a: gibt die 3'-nicht-kodierende Sequenz von TBEV Stamm Neudörfl wieder.

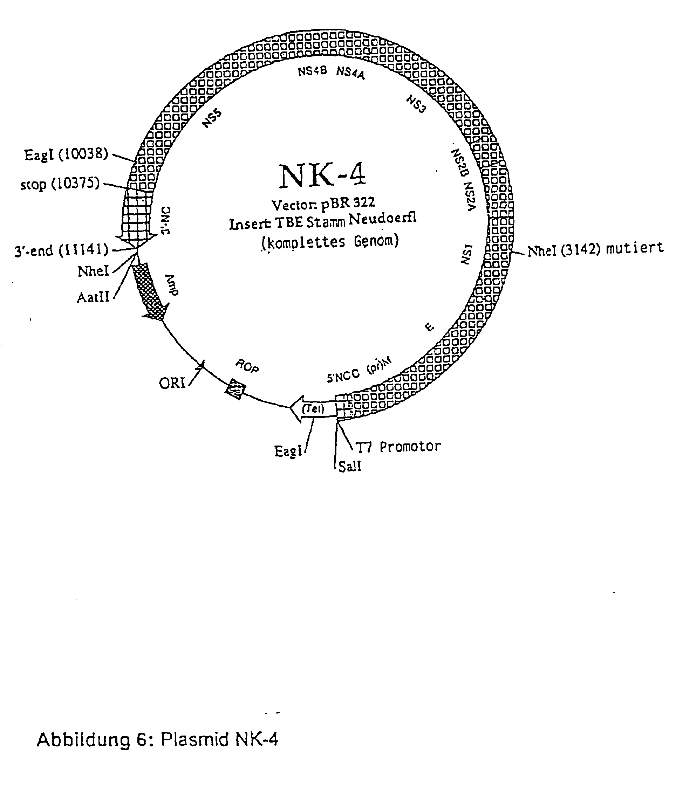

- Abb. 6: zeigt die Plasmidkarte des Klons NK-4 mit den relevanten Restriktionsschnittstellen.
- Abb. 7: zeigt die Plasmidkarte des Klons 3HA mit den relevanten Restriktionsschnittstellen.
- Abb. 8: zeigt einen Vergleich der Neurovirulenz und Neuroinvasivität zwischen TBE-Virus Stamm Neudörfl und der Mutante RB4.
- Abb. 9: gibt das Ergebnis des Tierversuches mit infektiöser RNA gemäss Beispiel 15 wieder.
- Tabelle 1: zeigt eine Liste der für die Herstellung der cDNA aus TBE-Virus-RNA und die Inserierung von Oligonukleotiden verwendeten Primern und deren Position auf der TBEV-Nukleotidsequenz entsprechend Abb. 5.
- Tabelle 2: zeigt die Sequenzunterschiede zwischen dem hergestellten infektiösen Klon von TBEV und der wt-Sequenz von TBE-Virus Stamm Neudörfl. Durch Sequenzanalyse der cDNA des infektiösen Klons von TBEV und der wt-DNA-Sequenz des Stammes Neudorfl konnten einige Sequenzänderungen, die bei der Herstellung der IK auftraten, identifiziert werden.
- Tabelle 3: zeigt Sequenzunterschiede zwischen TBE-Virus Stamm Neudörfl und dem attenuierten natürlichen Isolat 263.
- Tabelle 3a: gibt noch einmal in übersichtlicher Form Modifikationen und deren Position an, die erfindungsgemäss angewandt werden können, um zu einem attenuierten Stamm zu gelangen.

Tabelle 4 gibt die Antikörper-Titer in überlebenden Mäusen gemäss Beispiel 16 wieder.

Die erfindungsgemässe Herstellung von TBE-Virus mit Hilfe des infektiösen TBEV-Klons kann mit Hilfe folgender Verfahrensschritte erfolgen:
a) In vitro Herstellung eines RNA-Transkripts aus einem rekombinanten DNA-Konstrukt, welches die DNA gemäss Fig. 5 umfasst,
b) Transfektion von Wirtszellen mit dem unter a) erhaltenen RNA-Transkript, und
c) Gewinnung infektiöser Viruspartikel.

Alternativ kann die Transkription der RNA auch in vivo (also in Wirtszellen) durchgeführt werden:
a) Transfektion von Wirtszellen mit einem DNA-Konstrukt, welches die DNA von Fig. 5 umfasst, und
b) Gewinnung infektiöser Viruspartikel.

In den vorstehend genannten Verfahren bedient man sich zur Transformation bzw. Transfektion der Wirtszellen eines DNA-Konstruktes, welches neben den für TBEV kodierenden DNA-Sequenzen ein Vektorsystem sowie einen Promotor umfasst. Geeignete Promotoren werden ausgewählt aus den prokaryontischen Promotoren SP6, T3 und T7 oder aus den eukaryontischen Promotoren von CMV, RSV, ss-Aktin, SV40 oder Adeno-2.

Sofern ein in seiner Virulenz veränderter TBEV-Klon hergestellt oder die Ausbeute an infektiösem Virus durch Veränderung des TBEV-Genoms verbessert werden soll, ist es erforderlich, von einer, wie vorstehend ausgeführt, modifizierten DNA auszugehen. Die entsprechenden Herstellungsschritte für die Gewinnung von TBE-Virus mit Hilfe eines infektiösen Klons umfassen dann entweder
a) die in vitro Herstellung eines RNA-Transkripts von einem rekombinanten modifizierten DNA-Konstrukt,
b) die Transfektion von Wirtszellen mit dem viralen RNA-Transkript, und
c) die Gewinnung infektiöser modifizierter Viruspartikel, oder
a1) die Transfektion einer Wirtszelle mit einem rekombinanten modifizierter DNA-Konstrukt, und
b1) die Gewinnung infektiöser modifizierter Viruspartikel.

Die vorstehend aufgeführten alternativen Verfahren zur Herstellung von TBE-Virus mit Hilfe eines infektiösen TBEV-Klons erfolgen über transformierte oder transfizierte prokaryontische oder eukaryontische Wirtszellen, die ebenfalls von der Erfindung umfasst sein sollen. Als besonders bevorzugte Wirtszellen eignen sich E. coli, Vero-, CHO- oder BHK-Zellen.

Ein weiterer Aspekt der vorliegenden Erfindung betrifft ein pharmazeutisches Produkt, das einen verbesserten Impfstoff zum Schutz vor viralen Infektionskrankheiten darstellt. Erfindungsgemäss enthält das pharmazeutische Produkt eine *in vivo* replikationsfähige, infektiöse Nukleinsäure, die für ein komplettes TBE Virus oder ein virales TBE Partikel kodiert. In einer besonderen Ausführungsform stellt das erfindungsgemässe pharmazeutische Produkt ein 'Lebendimpstoff-Aguivalent' dar.

Ein zusätzlicher Aspekt der vorliegenden Erfindung ist, dass die replikationsfähige Nukleinsäure, enthaltend im erfindungsgemässen pharmazeutischen Produkt, selbst ein Vektor für die in *vivo* Expression eines biologisch aktiven Produkts darstellt und für die Therapie und/oder Prophylaxe bei Vertebraten eingesetzt werden kann.

Wie oben schon erwähnt werden konventionelle Impfstoffe in zwei Kategorien eingeteilt, Totimpfstoffe und Lebendimpfstoffe. Im Gegensatz zu Totimpfstoffen enthalten Lebendimpfstoffe infektiöse, aber nicht-pathogene Viren, deren Vermehrung zur Ausbildung einer Immunität führt, ohne eine Erkrankung auszulösen. Solche infektiösen, aber nichtpathogenen Viren sind hauptsächlich attenuierte Viren, die aufgrund von Mutationen eine verminderte Virulenz im Vergleich zum Wildtyp aufweisen. Attenuierte Virusstämme stellen die Basis der meisten heute beim Menschen verwendeten viralen Lebendimpfstoffe dar, wie zum Beispiel jene gegen Mumps, Masern, Röteln, Poliomyelitis oder Gelbfieber.

In bestimmten Fällen kann auch das pathogene Wildvirus selbst als Impfvirus verwendet werden, wie beispielsweise bei einem Impfstoff gegen Adenovirusinfektionen. Hierbei werden die pathogenen Adenoviren, in einer Kapsel verpackt, oral verabreicht, wodurch die natürliche Infektionsroute, nämlich der Respirationstrakt, umgangen wird. Eine Virusvermehrung findet erst nach Auflösung der Kapsel im Magen-Darm-Trakt statt, wo die Adenoviren nicht pathogen sind, also zu keiner Erkrankung führen, aber eine zur Immunität führende Immunreaktion induzieren (Couch et al 1968).

Die Herstellung von konventionellen Lebendimpfstoffen erfordert die grosstechnische Produktion von vermehrungsfähigen Viren, die üblicherweise in Zellkultursystemen oder in embryonierten Hühnereiern gezüchtet werden müssen. Diese Art der Virusproduktion ist nicht nur ein sehr teures Verfahren, sie birgt auch eine Reihe von Risikofaktoren. Das Kultivieren von Zellen, insbesonders im grosstechnischen Massstab, ist anfällig für Kontaminationen mit Fremdviren oder anderen Agentien. So enthielten beispielsweise Chargen des Gelbfieberimpfstoffes Hühnerleukämieviren, die in den für die Impfstoffherstellung verwendeten embryonierten Hühnereiern enthalten waren. Ein ähnlicher Fall war die Kontamination eines Polioimpfstoffs mit SV 40, einem beim Affen vorkommenden Virus, das in den Kulturzellen vorhanden war.

Das Ausschliessen solcher Kontaminationen erfordert aufwendige Massnahmen während der Produktion, und es müssen zusätzlich teure Kontrollverfahren zum Nachweis des vollstandigen Fehlens von Fremdviren und anderer kontaminierender Agentien, wie zum Beispiel fremder Nukleinsäuren, durchgeführt werden.

Ein weiteres Problem konventioneller Herstellungsverfahren ist, dass es während der für die Virusvermehrung nötigen Replikationsrunden zu Mutationen kommen kann, die die biologischen Eigenschaften des Impfvirus verändern und zu einer unerwünschten Erhöhung der Virulenz führen können. Um dieses Gefahrenpotential auszuschliessen, müssen bei bestimmten Impfstoffen mit jeder Charge aufwendige Virulenztests an Versuchstieren durchgeführt werden, bevor diese zur Verwendung am Menschen freigegeben werden können.

Weiters handelt es sich bei den zur Virusvermehrung verwendeten Zellkulturen häufig um nicht-humane Zellen, so dass Kontaminationen mit Bestandteilen des Zellsubstrates im Impfstoff allergische Reaktionen auslösen können.

Da die Wirksamkeit von konventionellen Lebendimpfstoffen in kritischer Weise vom Gehalt an infektiösen Viren abhängt, muss die Stabilität des Impfstoffes, insbesondere durch permanente Kühlung, gewährleistet werden. Dies ist vor allem für die Anwendung in warmen (z.B. tropischen) Gegenden problematisch, aber auch für spezielle Anwendungssituationen, bei denen die Kühlkette nicht eingehalten werden kann.

Ein verbesserter Impfstoff sollte die hier beschriebenen Gefahrenpotentiale ausschliessen und eine höhere Stabilität besitzen.

Ein Ansatz bei der Herstellung von Impfstoffen war, nackte DNA als Impfstoff zu verwenden. Dieser Ansatz wird in der Literatur als 'genetische Immunisierung' bezeichnet.

DNA kann in grossen Mengen und vollkommen frei von kontaminierenden Agentien hergestellt werden. Ein Impfstoff, dessen Hauptbestandteil DNA ist, ist sehr stabil und kann, vor allem in lyophilisierter Form, auch in warmen Gegenden und unter Bedingungen, wo die permanente Kühlung (geschlossene Kühlkette) nicht gewährleistet ist, eingesetzt werden.

Die Verabreichung nackter nicht-replizierender DNA ist im Stand der Technik schon mehrfach vorbeschrieben. Verschiedene Markergene (z.B. für beta-Galaktosidase oder Luciferase) wurden in entsprechenden Vektorkonstrukten, nach Inokulation in Versuchstiere *in vivo* exprimiert (Wolff J.A. et al., 1990; Hansen E. et al., 1991). Danach wurde der Versuch unternommen, durch Inokulation von DNA einen therapeutischen Effekt zu erzielen (Cohen J, 1993). Ein naheliegendes Modell für die intramuskuläre Verabreichung von nackter DNA war die Injektion von DNA, die für Dystrophin kodiert. Dystrophin ist ein Muskelprotein, das Muskeldystrophie-Patienten in unzureichendem Ausmass produzieren. Acsadi G. et al. (1991) konnten nachweisen, dass nach intramuskulärer Injektion eines entsprechenden DNA-Konstrukts in Mäusen ein Dystrophin-Gen exprimiert wurde. Weiters konnten die variablen Gene (V-Regionen) bestimmter Antikörper mit Hilfe der Injektion von nackter DNA zur Expression gebracht werden, und es wurde gezeigt, dass gegen die so exprimierten V-Regionen *in vivo* antiidiotypische Antikörper gebildet werden (Hawkins R.E., 1993; 1994; Watanabe A. et al., 1993).

Bei der 'genetischen Immunisierung' wird nackte DNA verabreicht, die für ein oder mehrere Antigene eines Virus kodiert. *In vivo* werden dann die viralen Antigene synthetisiert, die separat, jedes für sich, eine Immunantwort auslösen und damit in weiterer Folge einen Schutz vor Virusinfektionen bewirken. Bisher sind Versuche mit einer Reihe von Virusantigenen beschrieben: Hämagglutinin und Nukleoprotein von Influenza (Raz E. et al., 1994; Ulmer J.B. et al., 1993; Montgomery D.L. et al., 1993; Fynan E.F. et al., 1993 und Robinson H.L. et al., 1993; WO 93/19183), das Oberflächenantigen des Hepatitis-B Virus (Davis H.L et al., 1993), die Glykoproteine gI, gIII, gIV des Herpesvirus BHV-1 (Cox G.J. et al., 1993) und das Hüllprotein gp160 von HIV (Wang B. et al., 1993).

WO 90/11092 beschreibt die Expression von exogenen Polynukleotidsequenzen nach Injektion in Ratten, Mäuse und Menschen. Die Polynukleotidsequenzen kodieren für Proteine, deren *in vivo* Expression eine therapeutische (Adenosin-Deaminase, Wachstumshormon) oder eine immunisierende (gp120 von HIV, nef von HIV) Wirkung hat. Die immunisierende Wirkung wird dadurch erreicht, dass die Polynukleotidsequenzen für ein oder mehrere virale Antigene kodieren, die *in vivo* exprimiert werden und einzeln eine Immunantwort auslösen.

WO 93/17706 beschreibt den speziellen Fall einer 'genetischen Immunisierung' gegen HIV. Hierzu werden DNA-Konstrukte verwendet, die eines oder mehrere Gene des HIV-Virus enthalten. Die viralen Sequenzen wurden dabei so gewählt, dass die DNA-Konstrukte in *vivo* nicht replizierbar waren.

Auch WO 94/16737 beschreibt die genetische Immunisierung mit DNA-Molekülen, die für ein oder mehrere Antigene von HIV kodieren. Die Aufnahme der DNA in die Zielzellen wird durch Zusatz eines Polynukleotid-Funktions-Verstärkers ('polynucleotide function enhancer') verbessert.

Die dabei für die Immunisierung eingesetzte DNA kann als eine spezielle Form des Totimpfstoffes oder als 'Totimpfstoff-Äquivalent' betrachtet werden, bei dem die DNA nichtrepliziert und nur für ausgewählte Antigene eines Virus kodiert.

Eine weitere Aufgabe der vorliegenden Erfindung ist daher, eine *in vivo* replikationsfähige Nukleinsäure zur Herstellung eines Impfstoffes zur Verfügung zu stellen, der die für die Induktion einer Immunantwort äquivalenten Eigenschaften eines Lebendimpfstoffes aufweist und als 'Lebendimpfstoff-Aquivalent' bezeichnet werden kann. Die replikationsfähige Nukleinsäure ist ebenso für den Einstz der Therapie oder Prophylaxe geeignet.

Erfindungsgemäss wird die Aufgabe durch die Bereitstellung eines pharmazeutischen Produktes gelöst, das eine replikationsfähige, infektiöse Nukleinsäure enthält. Die Nukleinsäure kodiert vorzugsweise für ein komplettes TBE-Virus oder Teile davon, die zu einem viralen Partikel assemblieren können. Das pharmazeutische Produkt enthält vorzugsweise zusätzlich zur Nukleinsäure einen physiologisch akzeptablen Träger, der die Applikation bei Vertebraten ermöglicht.

Die im erfindungsgemässen pharmazeutischen Produkt verwendete Nukleinsäure ist entweder eine DNA oder ein RNA.

Die im erfindungsgemässen pharmazeutischen Produkt verwendete Nukleinsäure ist vorzugsweise eine replikationsfähige, infektiöse Nukleinsäure. "Infektiös" bedeutet hier, dass die Nukleinsäure in die Zielzelle aufgenommen wird, die genetischen Kontrollelemente der Nukleinsäure normal in diesen Zellen funktionieren und von dieser Nukleinsäure komplette Viren, Viruspartikel oder biologisch aktive Produkte gebildet werden. Dies kann auch erfordern, dass zusätzlich Nukleinsäuren in den Zielzellen zur Expression gebracht werden, die für Proteine kodieren, die zur Bildung eines replikationsfähigen Komplexes führen. Dies ist insbesondere bei Viren der Fall, die keine plus-Strang RNA enthalten, die als mRNA translatiert werden kann (Schnell et al 1994).

Die in Vertebraten bei der Applikation der replikationsfähigen Nukleinsäure, die für ein komplettes Virusgenom kodiert, *in vivo* neu produzierten Viren sind erfindungsgemäss selbst wiederum infektiös und lösen im Patienten, analog zu einem Lebendimpfstoff, eine protektive Immunantwort aus. In dieser Ausführungsform stellt das pharmazeutische Produkt ein 'Lebendimpfstoff-Äquivalent' dar. Das Lebendimpfstoff-Äquivalent unterscheidet sich vom konventionellen Lebendimpfstoff dadurch, dass ein gewünschtes Impfvirus nicht zuerst in Zellkultur oder embryonierten Hühnereiern gezüchtet werden muss, sondern die Nukleinsäure enthaltend im pharmazeutischen Produkt *in vivo,* also im Patienten selbst, produziert wird.

In einer Infektionsstudie wurde festgestellt, dass nach Injektion von infektiöser Nukleinsäure, die vom pathogenen TBEV-Wildtypstamm Neudörfl abgeleitet war, alle Tiere einer Versuchsgruppe von 10 Mäusen die für eine TBEV-Infektion typischen neurologischen Symptome, wie Tremor, Lähmungserscheinungen und Krämpfe aufwiesen. Nach 20 Tagen waren acht von zehn Mäusen gestorben. Die beiden überlebenden Tiere hatten hohe Antikörpertiter gegen TBEV im Blut, was anzeigt, dass auch sie eine TBEV-Infektion durchgemacht hatten. Dieses Ergebnis entspricht einem typischen Infektionsversuch mit einer mittleren Dosis (beispielsweise 100 pfu) von TBE-Virusstamm Neudörfl.

Eine Protektionsstudie beinhaltete dann die Injektion einer infektiösen Nukleinsäure, die von der attenuierten TBE-Virusmutante RB4 abgeleitet war. In diesem Fall erkrankten keine Tiere aus einer Versuchsgruppe von 10 Mäusen. 8 von diesen zehn Mäusen entwickelten hohe Antikörpertiter und waren dadurch vor einem Challenge mit einem pathogenen TBEV-Wildtypstamm geschützt.

Durch die vorliegende Erfindung wird zum ersten Mal gezeigt, dass bei Verabreichung von nackter, replikationsfähiger, infektiöser Nukleinsäure von TBEV komplette infektiöse Viren entstehen, die sich im Organismus vermehren und ausbreiten, was durch die Entstehung der typischen Symptome einer TBEV-Infektion und dem Virusnachweis im Gehirn im Falle des Wildtyp-Virusstammes und durch die Entstehung einer Immunität im Falle des attenuierten Stammes belegt ist.

Ein Aspekt der vorliegenden Erfindung ist, dass die in einem pharmazeutischen Produkt verwendete Nukleinsäure die gesamte genetische Information eines TBE-Virus enthält, die für ein komplettes Virus kodiert. Durch die Bereitstellung eines pharmazeutischen Produkts, das eine Nukleinsäure umfasst, welche die gesamte genetische Information des TBE-Virus enthält, ist gewährleistet, dass alle für die Induktion einer effektiven Immunantwort notwendigen Antigene in einer Einheit und einer dem nativen Virus äquivalente Struktur präsentiert werden. Die Nukleinsäure kann dabei abgeleitet sein von einem DNA oder RNA-Virus.

Ein besonderer Aspekt der Erfindung ist die *in vivo-*Replikation der Nukleinsäure. Dadurch wird ermöglicht, dass die Nukleinsäure *in vivo*, also im lebenden Organismus, insbesondere in Vertebraten, repliziert wird. Erfindungsgemäss wird für die *in vivo*-Replikation der Nukleinsäure der viruseigene Replikationsmechanismus verwendet. Es können jedoch auch gegebenenfalls zur verbesserten *in vivo*-Replikation der Nukleinsäure die viruseigenen durch andere Replikationselemente ersetzt werden. Dadurch kann die für die Immunisierung eingesetzte Menge an Nukleinsäure relativ niedrdig gehalten werden.

Ein besonderer Aspekt der Erfindung ist, dass die replikationsfähige Nukleinsäure für Produkte eines TBE-Virus kodiert, die zu einem viralen Partikel assemblieren, insbesonders für Proteine, die Antigene darstellen und eine protektive Immunantwort auslösen.

In einer speziellen Ausführungsform stellt die verwendete Nukleinsäure ein inkomplettes, aber replikationsfähiges Virusgenom oder ein Replikon dar, das ohne komplette Viren zu bilden, eine Immunantwort oder einen therapeutischen oder prophylaktischen Effekt hervorruft, indem es beispielweise für Proteine kodiert, die *in vivo* synthetisiert werden und sich zu viralen Partikeln assemblieren, die eine Immunreaktion auslösen. Die kodierenden Nukleinsäuresequenzen sind dabei so ausgewählt, dass sich die Antigene nach ihrer in *vivo* Expression spontan zu Viruspartikeln assemblieren. Für eine Reihe von Viren wurde gezeigt, dass die Expression von einzelnen Genen zur Assemblierung von immunogen wirkenden Viruspartikeln führt. Beispiele hierfür sind die Bildung von viralen Partikeln durch *in vitro*-Expression in Zellkulturen wie etwa von prM und E-Protein von Flaviviren (Konishi et al. 1992), von preSl-preS-S Antigen des Hepatitis B-Virus entsprechend EP 0 198 474 oder von env-, pol- und gagkodierenden Sequenzen von HIV(Karacostas et al. 1989, Kräusslich et al. 1993, Haffer et al. 1990). In den viralen Partikeln organisieren sich die Antigene zu Strukturen, wie sie auch im nativen Virus vorkommen. Dadurch wird mit dem erfindungsgemässen pharmazeutischen Produkt, das für ein virales Partikel kodierende Sequenzen enthält, eine im Vergleich zu einzeln exprimierten Proteinen (Sub-Unit-Vakzinen) effizientere Antigenpräsentation erreicht. Aus diesem Grund ist auch die protektive Immunisierung mit einer Nukleinsäure, kodierend für eine Reihe von vitalen Proteinen, die sich zu Partikeln assemblieren, effektiver im Vergleich zu Nukleinsäuren, die lediglich für ein Antigen kodieren.

In einer weiteren Ausführungsform stellt die Nukleinsäure ein Replikon dar, das durch in *vivo* Expression von biologisch aktiven Produkten, insbesondere genetische Defekte ausgleichen kann. Die erfindungsgemässe replikationsfähige Nukleinsäure kodiert dabei für ein biologisch aktives Produkt, wie z.B. eine RNA oder ein biologisch aktives Protein, insbesondere einen Blutfaktor, vorzugsweise Faktor II, Faktor V, Faktor VII, Faktor VIII, Faktor IX, Faktor X, Faktor XIII, Protein S, Protein C, Plasminogen oder von Willebrand-Faktor. Erfindungsgemäss kann dies durch Bereitstellung eines von einem replikationsfähigen Virusgenom abgeleiteten Vektors und in vivo Expression der gewünschten Proteine erfolgen.

Ein weiterer Aspekt der vorliegenden Erfindung ist daher, dass die replikationsfähige Nukleinsäure als Vektor für die *in vivo* Expression von biologisch aktiven Produkten eingesetzt werden kann.

Gemass einem zusätzlichen Aspekt der Erfindung ist die infektiose Nukleinsäure in einem Vektor enthalten, der die Replikation und Amplifikation der Nukleinsäure in einem entsprechenden Wirtssystem auch *in vitro* erlaubt. Der Vektor kann dabei ausgewählt sein aus Vektoren, die in eukaryotischen oder prokaryotischen Zellsystemen replizierbar sind. Die Vektoren können abgeleitet sein von Plasmiden oder Bakteriophagen; vorzugsweise verwendet werden Plasmide, insbesondere pBR322, BlueScript, etc.. Als Wirtszellen können aus dem Stand der Technik bekannnte eukaryotische oder prokaryotische Zellen eingesetzt werden. Gemäss der vorliegenden Erfindung wird die im pharmazeutischen Produkt enthaltene Nukleinsäure vorzugsweise in prokaryotischen Zellen, insbesondere *E. coli,* vermehrt.

Die Vermehrung der *in vivo* replikationsfähigen Nukleinsäure mittels eines Vektors, der zusätzlich die Replikation und Amplifikation in einem geeigneten Wirtssystem *in vitro* ermöglicht, erlaubt die grosstechnische Produktion von grossen Mengen an identischer Nukleinsäure. Durch diese Art der Herstellung von Nukleinsäure zur Verwendung im erfindungsgemässen pharmazeutischen Produkt ist das bei der konventionellen Lebendimpfstoffproduktion in Zellkulturen auftretende Risiko von im Virusgenom auftretenden Spontanmutationen, ausgeschlossen.

Erfindungsgemäss ist die für das pharmazeutische Produkt verwendete Nukleinsäure entweder eine DNA, cDNA oder eine RNA.

Erfindungsgemäß handelt es sich bei der replikationsfähigen Nukleinsäure um eine, ein vollständiges RNA-Virusgenom repräsentierende cDNA, von TBEV oder um die genomische RNA des TBE Virus, die in einem physiologisch akzeptablen Träger, beispielsweise einer physiologischen Pufferlösung, in Körperzellen eines lebenden Organismus eingebracht wird, wobei *in vivo* ein komplettes Virus entsteht.

Gegebenenfalls wird erfindungsgemäss von der kompletten genomische RNA eines RNA-Virus eine cDNA hergestellt. Die cDNA kann dann selbst im erfindungsgemässen pharmazeutischen Produkt enthalten sein, gegebenenfalls können von der cDNA auch wieder RNA-Kopien hergestellt werden, die dann anstelle der cDNA im pharmazeutischen Produkt enthalten sind. Die Wirksamkeit eines pharmazeutischen Produktes, enthaltend einen 'infektiösen Klon' von TBEV wird in einem nachfolgenden Ausführungsbeispiel gezeigt.

In einer speziellen Ausführung der vorliegenden Erfindung kann die cDNA, die eine vollständige Kopie des TBE Virus darstellt mit genetischen Kontrollelementen kombiniert werden, die eine Transkription *in vitro* oder *in vivo* erlaubt. Solche in der vorliegenden Erfindung verwendeten cDNA-Klone von RNA-Viren bieten den weiteren Vorteil, dass ihre Vervielfältigung durch DNA-Replikation und nicht durch RNA-Replikation, wie es bei der Vermehrung von RNA Viren in Zellkultur der Fall ist, erfolgt. Im Vergleich zur DNA-Replikation weist die RNA-Replikation eine wesentlich höhere Fehlerrate auf, wodurch Mutationen entstehen können, die unerwünschte Effekte auf die biologischen Eigenschaften des Virus, beispielsweise die Erhöhung der Virusvirulenz, haben. Diese Probleme werden durch die Verwendung von cDNA oder von von cDNA abgeleiteter RNA im erfindungsgemässen pharmazeutischen Produkt verhindert.

Aus der bereitgestellten cDNA kann ebenfalls durch in vitro-Transkription wieder eine infektiöse RNA hergestellt werden, die direkt im erfindungsgemässen pharmazeutischen Produkt verwendet wird.

Die RNA-Kopien werden nach dem Fachmann bekannten Methoden durch *in*-*vitro*-Transkription hergestellt. Dabei werden im Nukleinsäurevektor enthaltene prokaryotische Promotoren für die RNA-Synthese, beispielsweise T7, SP6 oder T3, verwendet.

Die Verwendung von RNA als Nukleinsäure, die das genomische Material eines Virus repräsentiert, wie sie gemäss der vorliegenden Erfindung am Beispiel vom TBE-Virus beschrieben wird, hat einen zusätzlichen Vorteil gegenüber der Verwendung von DNA. Mit geringer Wahrscheinlichkeit kann es beim Einbringen von DNA in Zellen zur Integration in die chromosomale DNA kommen, wodurch unerwünschte Effekte ausgelöst werden könnten. Dieses Problem tritt nicht auf, wenn im Verfahren gemäss der vorliegenden Erfindung RNA eingesetzt wird.

Ein besonderer Aspekt der vorliegenden Erfindung ist, dass die Nukleinsäure abgeleitet ist von einem attenuierten TBE-Virus. Durch die Verwendung von Nukleinsäure eines attenuierten TBE-Virus ist gewährleistet, dass vergleichbar zu einem konventionellen Lebendimpfstoff, keine pathogenen Viren entstehen, die eine potentielle Erkrankung auslösen.

Erfindungsgemäss kann die Nukleinsäure, die vom TBE-Virus abgeleitet ist, vorzugsweise durch bekannte gentechnologische Verfahren, modifiziert werden. Die Modifikationen können Mutationen, beispielsweise Substitutionen, Deletionen oder Insertionen sein.

Mutationen können beispielsweise gezielt zur Attenuation des von der Nukleinsäure kodierten kompletten Virus führen. Diese Möglichkeit erlaubt es in idealer Weise, gezielt attenuierte Virusstämme herzustellen. Durch diese Möglichkeit ist man nicht mehr wie bisher von zufällig in der Natur vorkommenden Virusstämmen mit geringerer Virulenz oder von langwierigen Selektionsverfahren im Labor abhängig.

Im Falle von TBEV kann eine Attenuierung beispielsweise durch folgende Modifikationen erreicht werden: Mutationen der pr(M)-Spaltstelle, der Glykosylierungsstelle im E-Protein, der Aminosäure 384 des E-Proteins (wie in der Mutante B4) oder andere Mutationen, die den Sequenzveränderungen in den natürlich vorkommenden attenuierten TBEV-Stämmen, beispielsweise dem TBE-Virusisolat 263, entsprechen.

Nach Verabreichung des pharmazeutischen Produktes, enthaltend eine replikationsfähige, infektiöse Nukleinsäure kodierend für ein komplettes TBE-Virus oder ein modifizierten Viruspartikel kommt, es erfindungsgemäss zur *in vivo* Vermehrung der Nukleinsäure und Expression der kodierenden Sequenzen und zur Entstehung von Virusstrukturen. Die Expression der viralen Genomsequenzen wird dabei durch die viruseigenen Transkriptions- und Translationselemente gesteuert.

Durch die Entwicklung der Gentechnologie wurde es möglich, die Expression spezifischer Nukleinsäuresequenzen gezielt zu verändern, insbesondere zu erhöhen. Dies kann durch bekannte Methoden wie z. B. das Ersetzen von wenig effizienten Promotoren durch sogenannte "starke" Promotoren, durch Beeinflussung der Ribosomenbindungsstelle von gering translatierten Proteinen oder durch Einbringen von bestimmten Verstärkern, sogenannten Enhancern, erfolgen.

Ein besonderer Aspekt der vorliegenden Erfindung ist es daher, durch gezielte Veränderung von viruseigenen, genetischen Kontrollelementen eine erhöhte *in vivo* Expression der viralen Proteine und damit eine schnellere und effizientere Bildung von Viren oder Viruspartikel zu erreichen. Dies erfolgt erfindungsgemäss durch die Veränderung oder den Austausch beispielsweise von 5' oder 3' nicht-kodierenden Sequenzen.

Erfindungsgemäss kann die Nukleinsäure durch gezieltes Einführen von eukaryotischen Promotor- und Enhancer-Elementen verändert werden. Dadurch kann die Expression der viralen Komponenten, die von der Nukleinsäure kodiert sind, moduliert werden. Insbesonders kann durch das Einführen spezieller Promotor- und Enhancer-Elemente in die Nukleinsäure die Gesamtexpression aller viralen Gene oder die Expression spezifischer viraler Gene verstärkt werden. Geeignete Promotoren werden ausgewählt aus den eukaryotischen Promotoren von CMV, RSV, β-Actin, TK, SV-40 oder Adeno-2.

Ein weiterer Aspekt der vorliegenden Erfindung ist, dass die Nukleinsäure gegebenenfalls neben der genetischen Information des TBE- Virus zusätzliche, kodierende Sequenzen enthält.

Diese Sequenzen kodieren in einer speziellen Ausführungsform für Immunmodulatoren. Immunmodulatoren nehmen Einfluss auf eine Immunreaktion, beispielsweise indem sie die Immunantwort verstärken. Bei dieser Ausführungsform des erfihdungsgemässen pharmazeutischen Produktes kommt es *in vivo* zu einer Koproduktion von Virus und Immunmodulator, was eine Erhöhung der Effizienz der Impfung bewirkt. Als Immunmodulator kommen beispielsweise Zytokine, insbesondere Interleukine, Diphtherietoxin, Choleratoxin, hitzelabiles Toxin von *E. coli* und Pertussistoxin in Frage. Erfindungsgemäss können ein oder mehrere Gene, die für einen Immunmodulator kodieren, in die Nukleinsäure, die im erfindungsgemässen pharmazeutischen Produkt enthalten ist, eingefügt werden.

Erfindungsgemäss enthält das pharmazeutische Produkt gegebenenfalls eine Nukleinsäure, die vom TBE-Virusgenom abgeleitet ist und zusätzlich kodierende Sequenzen aufweist, die für Antigendeterminanten von anderen Viren, beispielsweise von HBV, HCV oder HIV, oder von anderen Pathogenen, beispielsweise von Bakterien oder Protozoen, kodieren. Diese anderen Antigene werden dann zusammen mit den viralen Antigenen des TBE-Virus exprimiert. Die Präsentation solcher Antigene kann je nach Ausführungsform in einem kompletten Virus oder in einem viralen Partikel erfolgen. Dieses Prinzip kann auch zur Herstellung eines polyvalenten Impfstoffes in Form eines Kombinationsimpfstoffes verwendet werden.

Eine weitere Ausführungsform des vorliegenden pharmazeutischen Produktes ist, dass die in ihm enthaltene Nukleinsäure so konstruiert ist, dass sie neben den für die Replikation nötigen Sequenzen auch Gene für virale Komponenten oder andere biologisch aktive Moleküle enthält. So könnten durch die Verabreichung replizierbarer Nukleinsäure biologisch aktive Proteine in bestimmte Zellen zur Expression gebracht werden.

Die heute zur Verfügung stehenden Methoden der Gentechnologie und die Tatsache, dass sehr viele pathogene Antigene aber auch therapeutisch einsetzbare Proteine kloniert und mit ihrer Sequenz bekannt sind, ermöglichen praktisch alle gewünschten Varianten und Kombinationen von Nukleinsäureimpfstoffen gemäss der vorliegenden Erfindung.

Das pharmazeutische Produkt enthält erfindungsgemäss Nukleinsäuren in einer physiologisch wirksamen Menge, also in einer sicheren und effizienten Menge für die Immunisierung, Therapie oder Prophylaxe.

Üblicherweise ist das pharmazeutische Produkt in einem physiologischen Träger enthalten, der beispielsweise eine physiologische Pufferlösung ist, die unter normalen Bedingungen die Stabilität der erfindungsgemässen Nukleinsäure und somit auch des Impfstoffes gewährleistet.

Für extreme Einsatzbereiche, wo das erfindungsgemässe pharmazeutische Produkt beim Transport oder bei der Lagerung hohen Temperaturen oder hohen Temperaturschwankungen ausgesetzt ist, kann die Nukleinsäure in lyophilisierter Form transportiert und gelagert werden. Lyophilisierte Nukleinsäuren sind sehr stabil und können auch hohen Temperaturschwankungen ausgesetzt werden. Direkt vor der Verwendung einer lyophilisierten Nukleinsäure wird diese in einem physiologisch akzeptablen Träger zur Losung gebracht, danach kann sie sofort einem Patienten verabreicht werden. Um die hohe Stabiliät des erfindungsgemässen pharmazeutischen Produktes zu wahren, muss die Nukleinsäure, unabhängig davon, ob sie in einem passenden Träger oder in lyophilisierter Form vorliegt, in einem luftdichten und vor Licht geschützten Behälter aufbewahrt werden.

Erfindungsgemäss wird das pharmazeutische Produkt für die parenterale Verabreichung, insbesonders die intramuskuläre oder subkutane Verabreichung formuliert. Gegebenfalls kann bei der intramuskulären Verabreichung die Effizienz der Aufnahme in Muskelzellen durch die 'Vorinjektion' einer hypertonischen Zuckerlösung, insbesonders einer hypertonischen Saccharoselösung, erhöht werden.

Daneben ist es auch möglich, das pharmazeutische Produkt in oral verabreichbarer Form bereitzustellen.

Das pharmazeutische Produkt ist erfindungsgemäss in einem Behälter enthalten, der eine medizinische Verabreichung bei Vertebraten erlaubt. In einer besonderen Ausführungsform ist dieser Behälter eine Spritze.

Ein weiterer Aspekt der vorliegenden Erfindung ist ein Verfahren zur Herstellung eines pharmazeutischen Produktes, enthaltend eine replikationsfähige Nukleinsäure, gegebenenfalls eine infektiöse Nukleinsäure, die für ein komplettes Virus, ein virales Partikel oder ein biologisch aktives Protein kodiert. Das Verfahren beinhaltet, dass
a) aus einem Virus die virale Nukleinsäure isoliert und, gegebenenfalls bein einem RNA-Virus eine cDNA hergestellt wird,
b) gegebenenfalls die isolierte Nukleisäure in einen geeigneten Vektor eingebracht wird,
c) gegebenfalls die virale Nukleinsäure modifiziert wird, insbesondere:
   - durch Modifikationen, die zur Expression eines biologisch aktiven Produkts führen,
   - durch Modifikationen, die zur Attenuierung führen, oder
   - durch das Einführen von Nukleotidsequenzen, die auf die Expression der viralen Gene einen modulierenden, beispielsweise einen verstärkenden Effekt haben, oder
   - durch das Einführen von Nukleotidsequenzen, die für einen oder mehrere Immunmodulatoren kodieren, oder
   - durch das Einführen von Nukleotidsequenzen, die für eine oder mehrere zusätzliche Antigendeterminanten kodieren,
d) gegebenenfalls von der cDNA wieder eine RNA hergestellt wird,
e) die so erhaltene Nukleinsäurepräparation in einer physiologisch akzeptablen Menge mit einem Träger gemischt wird, und
f) in einen Behälter zur parenteralen oder oralen Verabreichung in Vertebraten gebracht wird.

Gemäss der vorliegenden Erfindung kann die im pharmazeutischen Produkt enthaltene Nukleinsäure auch über chemische Synthese hergestellt werden.

Die vorliegende Erfindung umfasst die Verwendung eines pharmazeutischen Produktes, das Vertebraten parenteral oder oral verabreicht wird, wodurch *in vivo* komplette Viren oder virale Partikel generiert werden, die eine Immunantwort auslösen und zu einer Immunität gegen die jeweilige virale Infektion führen, oder durch die *in vivo* Expression eines biologisch aktiven Produkts ein anderer prophylaktischer oder therapeutischer Effekt erzielt wird.

Erfindungsgemäss wird das geschützte pharmazeutische Produkt zur Immunisierung von Vertebraten oder zu therapeutischen Zwecken eingesetzt.

Als bevorzugter prophylaktischer oder therapeutischer Effekt gilt insbesondere der Einsatz des erfindungsgemässen pharmazeutischen Produktes in der Gentherapie (Friedmann, 1993), wie beispeilsweise beim Lesch-Nyhan-Syndrom, bei Hämophilie A oder B, bei vWD oder Mukoviszidose, oder bei der Tumortherapie.

Die Anwendung des beschriebenen Verfahrens ist auf alle Systeme möglich, bei denen durch Verabreichung von Nukleinsäuren *in vivo*, also am lebenden Organismus, diese Nukleinsäuren replikationsfähig sind, und gegebenenfalls infektiöses TBE Virus gebildet werden kann, wodurch eine protektive Immunantwort ausgelöst wird oder ein biologisch aktives Protein exprimiert wird.

Die Erfindung wird anhand der folgenden Beispiele näher erläutert.

### Beispiel 1

### Herstellung von cDNA

TBE-Virus wurde nach der schon früher beschriebenen Methode (Heinz und Kunz, 1981) in Hühnerembryofibroblasten-Kulturen gezüchtet und aus dem Überstand gereinigt. Diese Reinigung umfasst im wesentlichen eine Pelletierung des Virus durch Ultrazentrifugation und anschliessend eine zweimalige Auftrennung durch Zentrifugation über Sucrose-Dichtegradienten.

Aus gereinigten Viruspräparationen wurde dann die virale genomische RNA durch zweimalige Extraktion mit Phenol und Chloroform gewonnen und in Gegenwart von RNase-Inhibitor (human placental RNAsin; Promega) mit Ethanol gefällt. Die Menge und Integrität der so gewonnen RNA wurde durch Elektrophorese auf denaturierenden Glyoxal-Agarose-Gelen, die mit Acridin-Orange gefärbt wurden (McMaster und Carmichael, 1977), bestimmt. Ungefähr 1µg solcher RNA wurde dann für die Synthese spezifischer cDNA eingesetzt.

Doppelsträngige cDNA wurde nach der Methode von Gubler und Hoffman (1983) unter Verwendung der im "cDNA Synthesis Kit" (cat. no. 1013 882) der Firma Boehringer-Mannheim enthaltenen Reagenzien, synthetisiert. Als Primer für die cDNA Synthese dienten entweder Zufallssequenz-Hexanukleotide oder spezifische Oligonukleotide von meist 20 Basen Länge (z.B. T36, T45, T61, H01; siehe Tabelle 1, die entsprechend der von Mandl et al. (1988 und 1989) publizierten Sequenz des TBE-Virus Prototyp Stamm Neudörfl synthetisiert worden waren (Medprobe®, Oslo, Norwegen). Die erhaltene cDNA wurde auf 1% Agarose-Gelen überprüft und entweder direkt in ein bakterielles Plasmid kloniert (siehe unten) oder in Verdünnungen von 1:100, 1:1000 oder 1:10000 als Ausgangsmaterial für die Herstellung spezifischer Fragmente mit Hilfe der Polymerase-Kettenreaktion (PCR) eingesetzt. Für die PCR wurden die früher beschriebenen Bedingungen (Mandl et al., 1989) verwendet. Allerdings wurde für die Herstellung langer (1000 bis 4500 Basenpaare) PCR Fragmente die Inkubationszeit bei 68°C, während der die enzymatische DNA Synthese stattfindet, auf bis zu 7 min verlängert. Die für die PCR verwendeten Primer waren synthetische Oligonukleotide von meist 20 Basen Länge (Medprobe®) und wurden in einer Konzentration von 100 ng/ml eingesetzt. Zur spezifischen Konstruktion der den Enden des viralen Genoms entsprechenden cDNA Moleküle und zur spezifischen Mutagenese (siehe unten) wurden auch längere Oligonukleotide (bis zu 51 Basen Länge; siehe Tabelle 1), die ausser der dem viralen Genom entsprechenden Sequenz auch andere Sequenzelemente (Restriktionsschnittstellen, Promoter, Mismatches zur Mutagenese) enthielten, verwendet. Diese Primer wurden in Konzentrationen von bis zu 1 µg/ml in der PCR eingesetzt.

### Beispiel 2:

### Klonierung von cDNA

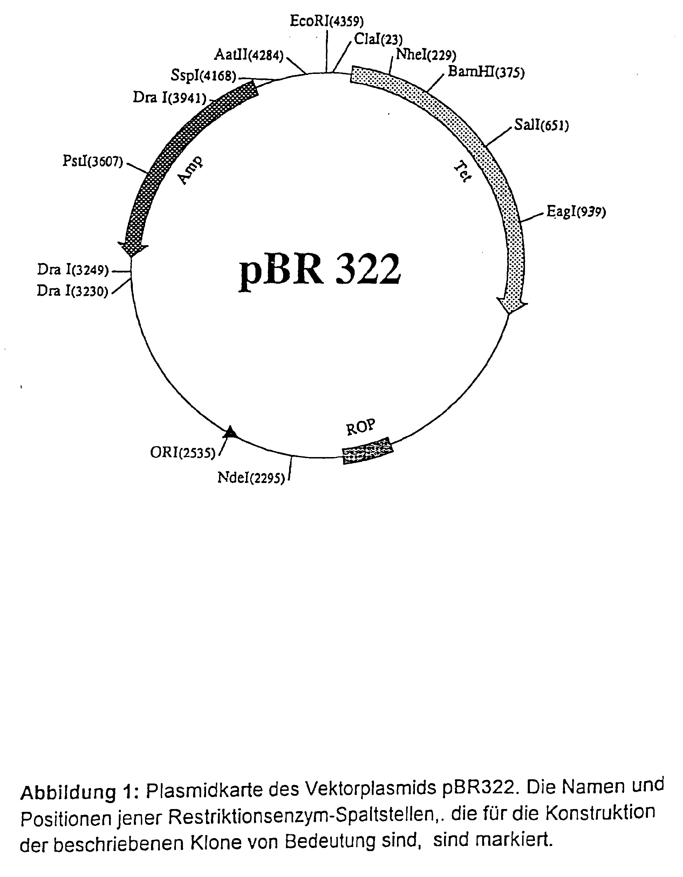

Doppelsträngige cDNA wurde mit Restriktionsenzymen, deren Schnittstellen in der Sequenz des TBE-Virus auf Grund der publizierten Genomsequenzen (Mandl et al., 1988; 1989) bekannt waren, geschnitten und in das Plasmid pBR322 (Abb. 1) kloniert. Dazu wurden 100ng pBR322 (New England Biolabs) mit demselben Restriktionsenzym geschnitten, falls nötig das entsprechende Fragment nach Elektrophorese auf 1% Agarose-Gelen (BioRad) durch Elektroelution (Elektroeluter von IBI) isoliert, und mit der cDNA gemischt. Das Gemisch wurde durch Phenol- und Chloroform-Extraktionen gereinigt, mit Ethanol gefällt und über Nacht in einem Volumen von 20 - 30 µl Ligationspuffer (Stratagene) bei 16°C mit 2µl T4 DNA Ligase (Stratagene) inkubiert. Dann wurde der Ligationsansatz bei 65°C für 5 min erwärmt um das Enzym zu inaktivieren, und 2µl von 5 bis 20-fachen Verdünnungen wurden zur Transformation von E.coli Bakterien (Stamm HB101) verwendet. Die Transformation erfolgte entweder nach der Hitzeschockmethode (Maniatis et al., 1982) unter Verwendung kompetenter Bakterien (Epicurian E.coli, Fa. Stratagene) oder durch Elektroporation mit dem GenePulser (Biorad). Rekombinante Bakterienkolonien wurden nach gängigen Standard-Labormethoden herangezüchtet und zur Isolierung von Plasmid DNA verwendet (Maniatis et al., 1982).

Die oben beschriebene Methode zur direkten Klonierung von cDNA wurde beispielsweise angewendet, um ein 3797 Basenpaar (bp)-langes cDNA Fragment (von PstI 3021 bis PstI 6818) insertiert in der singulären PstI Stelle von pBR322 zu erhalten (Klon PstI, siehe unten). Der Vorteil dieser Methode besteht darin, dass die so klonierten cDNA-Fragmente in der Regel keine Fehler in der Nukleotidsequenz aufweisen, wie das bei Verwendung der PCR häufig zu beobachten ist. Es konnten aber mit dieser Methode nicht allen Abschnitten des Genoms des TBE-Virus entsprechende cDNA-Fragmente kloniert werden. Insbesondere die Herstellung von cDNA Fragmenten, die den Enden des viralen Genoms entsprechen, erforderte den Einsatz der PCR. Fragmente, die mit PCR hergestellt worden waren, wurden anschliessend, nach derselben Methode wie oben beschrieben, kloniert oder es wurden bei der PCR Oligonukleotidprimer eingesetzt, die an ihren 5'-Enden Schnittstellen für Restriktionsenzyme aufwiesen, die dann bei der Klonierung verwendet werden konnten.

### Beispiel 3:

### Charakterisierung und Sequenzanalyse klonierter cDNA

Jedes in ein bakterielles Plasmid klonierte cDNA-Fragment wurde genau hinsichtlich seiner Lage und Orientierung im Plasmid, dem etwaigen Vorkommen von Deletionen oder Insertionen oder von durch Rekombination aufgetretenen Veränderungen untersucht. Dazu wurden einige µg Plasmid DNA aus 2-3 ml Kulturen der entsprechenden E.coli HB101 Kolonie gereinigt (Magic Mini Prep System®, Promega) und mit verschiedenen Restriktionsenzymen verdaut. Die dann auf 1 - 2% Agarose-Gelen aufgetrennten Fragmente ergaben spezifische Bandenmuster, durch die die cDNA Fragmente zunächst grob charakterisiert werden konnten. Von jenen Plasmiden, die auf Grund dieser Analysen ausgewählt wurden, wurden 1 - 2 mg in hochgereinigter Form hergestellt (CsCl-Gradienten-Reinigung, Maniatis et al., 1982). Ca 1µg solcher Plasmidpräparationen wurde dann für eine einzelne Sequenzanalysenreaktion eingesetzt. Die Sequenzanalyse erfolgte unter Verwendung Fluoreszenzfarbstoff-markierter Didesoxyterminatoren (ABI, Perkin Elmer) und des automatisierten Sequenzierautomaten 373A der Fa.ABI (jetzt Perkin Elmer) unter Anwendung der Reagaenzien dieser Firma und der vom Hersteller angegebenen Reaktionsbedingungen für die Sequenzierung doppelsträngiger DNA. Als Primer in den Sequenzreaktionen diente eine Kollektion von etwa Hundert 20mer Oligonukleotiden (Medprobe®, Oslo, Norwegen), die Sequenzen in Abständen von nicht mehr als 400 Nukleotiden auf dem Plus- und dem Minusstrang des TBE-Virus Stamm Neudörfl komplementär waren. Unter Verwendung dieser Primer konnte die Nukleotidsequenz jeder beliebigen Stelle des Genoms in beiden Orientierungen analysiert werden. Prinzipiell wurden nur Fragmente, deren Sequenz vollständig bestätigt worden war, für weiterführende Klonierungsarbeiten herangezogen. Alle PCR bedingten Mutationen, die zu Änderungen der Aminosäuresequenz geführt hätten, wurden durch Austausch von Restriktionsfragmenten mit fehlerlosen Fragmenten aus anderen Klonen, die entweder ebenfalls durch PCR oder durch direkte Klonierung von cDNA erhalten worden waren, nach Standardprotokollen (Maniatis et al., 1982) beseitigt. Die Sequenzen solcher korrigierter Klone wurde wiederum durch vollständige Sequenzanalyse überprüft.

Computeranalysen der Sequenzdaten wurden mit den Microgenie (Beckman) und PcGene (Intellegenetics) Software-Paketen durchgeführt.

### Beispiel 4:

### Konstruktion und Beschreibung von Plasmid T7-2

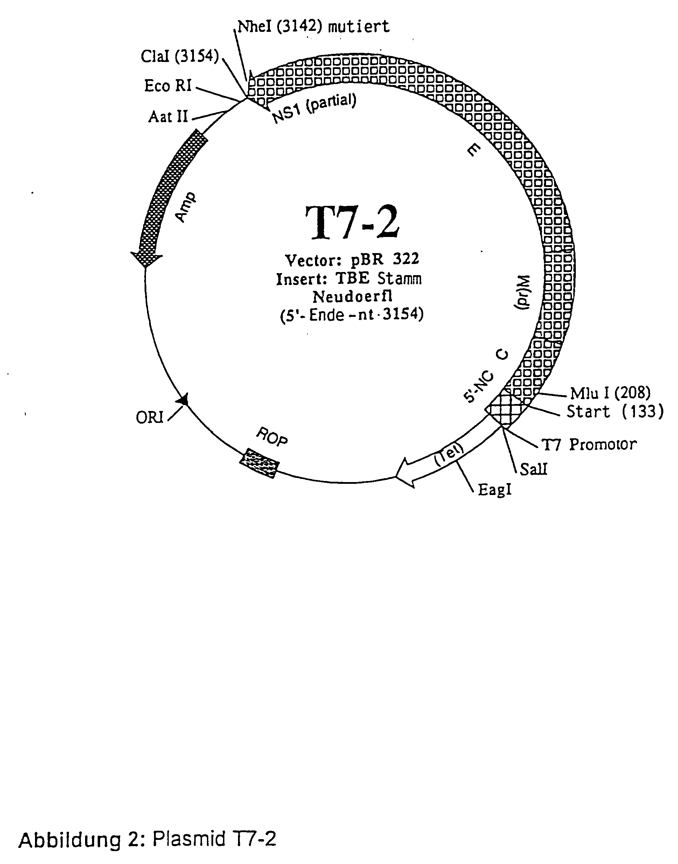

Plasmid T7-2 (Abb.2) enthält cDNA der 5'-terminalen Region des TBE-Virus Stamm Neudörfl insertiert in den Vektor pBR322 (Abb.1). In die Restriktionsschnittstellen ClaI (23) und SalI (651) von pBR322 wurde ein DNA-Fragement kodierend für TBEV-Sequenzen inseriert. Dieses Fragement umfasst das 5'-Ende (Nukleotid Position 1) bis zur in der nativen TBE Sequenz nur einmal vorkommenden ClaI Stelle an Position 3154. Vor dem 5'-Ende befindet sich ein weiteres Nukleotid (G), das in der nativen Sequenz des TBE-Virus nicht vorkommt, sowie eine T7 Promotor-Sequenz und die SalI Restriktionsschnittstelle, mit deren Hilfe das Fragment in den Vektor einligiert wurde. Die genaue Nukleotidsequenz des 5'-Endes und der davor liegenden Sequenzen zeigt Abb. 3. Sequenzanalysen ergeben, dass gegenüber der nativen Sequenz des TBE-Virus Stamm Neudörfl Plasmid T7-2 zwei Nukleotidänderungen aufweist (Tabelle 2):
i) eine stumme Mutation an Position 930 (T->C), die einer natürlich vorkommenden Variabilität der als Ausgangsmaterial verwendeten Viruspräparation entspricht;
ii) eine stumme Mutation an Position 3147 (C->T), die spezifisch eingeführt wurde, um die NheI Stelle an Position 3142 zu zerstören.

Die Herstellung von T7-2 erfolgte über folgende Konstruktionsschritte:

Zunächst wurde ein cDNA Fragment wie in Beispiel 1 beschrieben mittels PCR mit den Primern T01 und T45 (Tabelle 1) hergestellt und in pBR322, geschnitten mit SalI und ClaI(siehe Abb.1), einkloniert. Der erhaltene Klon enthielt die der Sequenz des TBE-Virus Stamm Neudörfl (Abb. 5) von Nukleotidposition 1 bis 3154 mit Ausnahme einer einzigen stummen Mutation an Position 930 (Tabelle 2) exakt entsprechende cDNA. Dieses Fragment wurde mit NheI (3142) und ClaI (3154) geschnitten, und das resultierende Fragment, das aus 9 Basenpaaren und zwei 4 und 2 Nukleotiden langen Überhängen (Abb. 5) besteht, durch ein mit den Oligonukleotiden T77 und T78 (Tabelle 1) gebildetes Insert ersetzt. Dadurch wurde die Mutation an Position 3147 eingeführt. Um das zusätzliche G-Nukleotid sowie die T7-Promotorsequenz am 5'-terminalen Ende der nicht-kodierenden TBEV-Sequenz einzuführen, wurde das Fragment von der 5'-terminalen, durch Primer T01 eingeführten SalI Stelle bis zur MluI Stelle (Position 208) durch ein PCR Fragment, das mit den Primern T83 (hybridisiert hinter MluI Stelle, siehe Abb. 5) und T84 (enthält eine T7-Promotorsequenz, davor eine SalI Schnittstelle und danach ein zusätzliches G gefolgt von den zwanzig 5'-terminalen Nukleotiden der TBE Sequenz; siehe Tabelle 1) hergestellt wurde, ersetzt, und Plasmid T7-2 in der oben beschriebenen Ausführung erhalten.

### Beispiel 5

### Konstruktion und Beschreibung von Plasmid 3ND-1

Plasmid 3ND-1 enthält cDNA des 3'-terminalen Bereiches (Nukleotid 3017-11141) des TBE-Virus Stamm Neudörfl Genoms insertiert in den Vektor pBR322. In pBR322, geschnitten mit PstI (3607) und AatII (4284), wurde ein TBEVcDNA-Fragment, das native TBE Sequenzen von der PstI Restriktionsschnittstelle (Position 3017) bis zum 3'-Ende (Position 11141) umfasst, inseriert. Die zwei 3'-terminalen Nukleotide (CT; Abb. 3) sind Teil einer dort eingeführten NheI Stelle, gefolgt von einer AatII Stelle, mit deren Hilfe das Fragment in den Vektor einligiert wurde. Wie weiter unten ausgeführt, erlaubt die Restriktion mit NheI und die anschliessende partielle Auffüllung des sich ergebenden 5'-überhangs die Herstellung eines der Sequenz des TBE-Virus exakt entsprechenden 3'-Endes. Die genaue Nukleotidsequenz des 3'-Endes und der benachbarten Regionen zeigt Abb. 3. Gegenüber der nativen Sequenz des TBE-Virus Stamm Neudörfl weist 3ND-1 sechs stumme Mutationen auf, die in Tabelle 2 zusammengefasst sind. Diese Mutationen wurden durch Fehler der Taq Polymerase bei der PCR verursacht.

Der Klon 3ND-1 enthält eine vollständige 3'-nicht-kodierende Sequenz, wie sie TBE-Virus Stamm Neudörfl entspricht. Eine solche Sequenz war bisher nicht bekannt. Dieser Sequenzabschnitt des Klons 3ND-1 ist in Abb.5 und 5a vollständig aufgelistet. Die Länge der internen poly(A)-Sequenz ist in nativem Virus offenbar variabel (Mandl et al., 1991), und beträgt bei Klon 3ND-1 49 Nukleotide.

Die Herstellung von Plasmid 3ND-1 erfolgte über folgende Konstruktionsschritte:

Durch direktes Klonieren von cDNA (beschrieben in Beispiel 2) wurde Klon PstI hergestellt, bei dem das von Position 3021 bis Position 6818 des TBE-Virus reichende Fragment in die singuläre PstI Stelle des Vektors pBR322 insertiert ist. Die Orientierung des Inserts war jener des Ampicillin Resistenz-Gens von pBR322 entgegengesetzt. Aus diesem Klon wurde das MluI (6446 in TBE)/ AatII (4284 in pBR322)-Fragment entfernt und durch das mit Hilfe der Primer T16 und T61 (Tabelle 1) hergestellte PCR Fragment ersetzt. Dafür wurde das 4500 Basenpaar lange PCR-Fragment mit MluI (Position 6446 der TBE Sequenz; siehe Abb. 5) und AatII (in Primer T61 enthalten) verdaut und in die entsprechenden Schnittstellen einligiert. Dadurch wurde das TBEV-spezifische Insert bis zur poly(A)-Sequenz (beginnend bei Position 10489) verlängert. cDNA, die der 3'-nicht-kodierenden Region von Stamm Neudörfl entsprach, wurde mit Hilfe des Primers H01 (Tabelle 1) hergestellt und mittels PCR mit den Primern T27 und H05 (Tabelle 1) amplifiziert und in pBR322 zwischen die Restriktionsstellen Dra I (3230) und AatII (4284) (vergleiche Abb. 1) kloniert. Aus den so erhaltenen Klonen wurde das Plasmid Dra-7, enthaltend ein Insert mit TBEV-Sequenzen von Nukleotidposition 10482 bis zum 3'-Ende (Position 11142), das eine poly(A)-Sequenz von 49 nts Länge und keine Nukleotidunterschiede zur Nativsequenz des TBE-Virus Stamm Neudörfl aufwies, ausgewählt. Das Fragment von der DraI Stelle (10484 in TBEV-Genom) bis zur AatII Schnittstelle hinter dem 3'-Ende (siehe Abb. 3) wurde an die oben beschriebene Konstruktion angefügt, um den Klon 3ND-1 in der oben beschriebenen Ausführung zu erhalten.

### Beispiel 6:

### Konstruktion und Beschreibung von Plasmid NK-4

Plasmid NK-4 enthält die vollständige cDNA Sequenz des TBE-Virus Stamm Neudörfl insertiert in den Vektor pBR322 zwischen den Restriktionsschnittstellen Sal I (651) und AatII (4284). Das 5'- und das 3'-Ende entsprechen jenen der Klone T7-2 beziehungsweise 3ND-1 (Abb. 3). Die 3'-nicht-kodierende Sequenz entspricht der in Klon 3ND-1 beschriebenen und ist in Abb. 5a dargestellt. Die insgesamt acht stummen Mutationen gegenüber der nativen Sequenz des TBE-Virus Stamm Neudörfl entsprechen jenen der oben beschriebenen Plasmide (Tabelle 2).

Zur Herstellung von Plasmid NK-4 wurde das ClaI (3154) - AatII 3'-terminale Fragment aus 3ND-1 isoliert und in Plasmid T7-2, geschnitten mit ClaI und AatII, einligiert. Das so gebildete Plasmid hatte ein Grösse von 14,9 kb und konnte nur durch Elektroporation in *E.coli* HB101 Zellen eingebracht werden. Die rekombinanten Klone vermehrten sich nur schlecht und enthielten das Plasmid NK-4 in verglichen mit pBR322 mindestens 100fach geringerer Kopienzahl.

### Beispiel 7:

### Konstruktion und Beschreibung von Plasmid 3HA

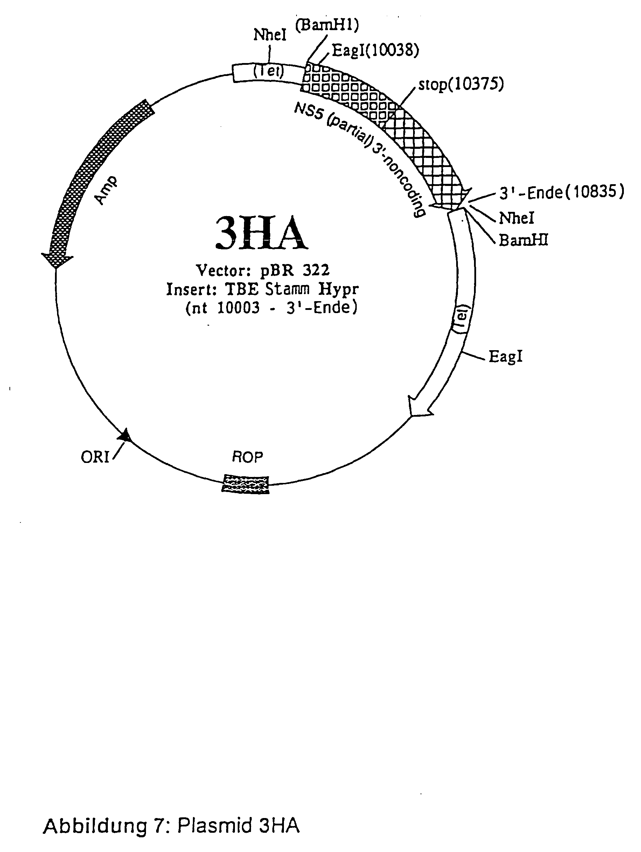

Plasmid 3HA (Abb. 7) enthält cDNA, die der 3'-terminalen Region des Genoms des TBE-Virus Stamm Hypr entspricht. Das Insert beginnt bei Nukleotid 10002 des TBE-Virus, umfasst einen Teil des für das NS5 Protein kodierenden Genomabschnittes sowie die gesamte 3'-nicht-kodierende Sequenz von TBEV Stamm Hypr bis zum 3'-Ende an Position 10835. Wie bei den Klonen 3ND-1 und NK-4 ist unmittelbar am 3'-Ende eine NheI Schnittstelle plaziert. Daran schliesst sich eine BamHI Schnittstelle, die ebenso wie eine weitere BamHI Stelle am anderen Ende des Fragments eingeführt wurde, um es in die entsprechende Restriktionsstelle des Vektors pBR322 an Position 375 einzuklonieren. Die genaue Nukleotidsequenz des 3'-Endes und der umgebenden Sequenzen zeigt Abb. 3.

Zur Herstellung von Plasmid 3HA wurde cDNA von genomischer RNA des TBE-Virus Stamm Hypr unter Verwendung des Primers H01 (Tabelle 1) hergestellt und das entsprechende Fragment mittels PCR mit Hilfe der Primer H11 und H12 (Tabelle 1) amplifiziert. Die PCR Primer bildeten an ihren 5'-Enden BamHI-Schnittstellen, die zur Klonierung des Segments in die BamHI-Stelle von pBR322 dienten. Die 5'-terminale BamHI Stelle erlitt im Zuge der Klonierung eine Deletion einer Base und liegt daher im Plasmid 3HA nicht als funktionelle Erkennungssequenz vor, wird also von BamHI nicht geschnitten. (Die entsprechende Restriktionsschnittstelle ist in Abb. 7 in Klammern angeführt.)

### Beispiel 8

### In-vitro-Transkription von RNA

Jene Plasmide, die eine T7 Promoter Sequenz enthalten (NK-4 und T7-2), beziehungsweise Ligationsprodukte dieser Plasmide mit anderen (siehe unten), wurden dazu verwendet RNA, die dem kompletten Genom des TBE-Virus entspricht, mittels in-vitro-Transkription herzustellen. Die Transkription wurde mit CsCl-Gradienten gereinigter DNA und im MEGAscrip T7 kit® (Ambion, Texas, USA) enthaltenen Reagenzien im wesentlichen entsprechend den vom Hersteller angegebenen Bedingungen durchgeführt. Die Inkubationszeit bei 37°C wurde jedoch auf 3 Std. erhöht und die an die RNA Synthese anschliessende Behandlung mit DNAseI in typischen Experimenten nicht durchgeführt. Ausserdem wurde die Konzentration des Nukleotids GTP in jenen Ansätzen, in denen das m7G(5')ppp(5')G Cap Analog (Ambion) eingesetzt wurde, auf ein Fünftel reduziert. In ein Reaktionsvolumen von 20µl wurde circa 1µg DNA eingesetzt. Die erhaltene RNA wurde unmittelbar weiterverwendet. Die Analyse eines Aliquots solcher RNA Produkte auf Agarose-Gelen (McMaster und Carmichael, 1977) liess erkennen, dass in solchen Reaktionsansätzen von 1µg DNA ungefähr 10µg RNA, deren Grösse jener des viralen Genoms entsprach, synthetisiert wurden.

### Beispiel 9:

### Transfektion von RNA in eukaryontische Zellen und Analyse der Virusvermehrung

BHK Zellen wurden nach gängigen Laborpraktiken in 77 cm² Flaschen gezüchtet und der fast konfluente Zellrasen mit zwei 5 ml Aliquots einer Trypsin-EDTA Lösung (Gibco) abgelöst. Die Zellen wurden in 10 ml Zellkulturmedium resuspendiert und in einem 50 ml Falcon Röhrchen durch Zentrifugation in einer Sigma 4K10 Zentrifuge (800 UpM/7 min/ 20°C) pelletiert. Das Zellpellet wurde dann zweimal in kaltem (4°C) RNAse freiem PBS (Gibco) resuspendiert und abermals wie oben pelletiert. Dann wurde das Pellet in 5 ml PBS resuspendiert und in Aliquots von 0.8 ml (entsprechend circa 1 x 10⁶ Zellen) in 0.4 cm Elektroporations-Küvetten (BioRad) transferiert. RNA, die aus Viruspräparationen gereinigt worden war oder durch T7-in-vitro-Transkription hergestellt worden war, wurde direkt in die Küvetten pipettiert und vorsichtig mit der Zellsuspension vermischt. Die eingesetzten RNA Mengen lagen zwischen einigen pg RNA und einigen mg RNA. Dann wurden die Küvetten in den GenePulser der Firma BioRad eingesetzt und die Elektroporation mit folgenden Parametern durchgeführt: Spannung 1.5 kV; Kapazität 25 µF; kein Einsatz des "capacitance extenders"; Einstellung auf dem Pulse Controller auf "infinity". Unter diesen Bedingungen wurde als Zeitkonstante üblicherweise 0.9 erhalten. 10 sec nach dem ersten Stromstoss wurde ein zweiter ausgeführt und dann die Küvette für 10 min bei Zimmertemperatur stehen gelassen. Erst dann wurden die Zellen in eine neue Zellkulturflasche gesät und für die nächsten Tage in Medium, das 5% fötales Kälberserum enthielt, gezüchtet. Sobald die Zellen konfluent waren, wurde die Serumkonzentration auf 1% abgesenkt.

Zur Überprüfung der Virusvermehrung wurden täglich 50µl Aliquote der Zellkulturflüssigkeit entnommen und bei -20°C tiefgefroren. Mit Hilfe eines 4-Schicht ELISA (Heinz et al. 1986) wurde das TBE-Virus in den Zellkulturüberständen nachgewiesen.

### Beispiel 10:

### Herstellung von TBE-Virus Stamm Neudörfl von rekombinanter cDNA

TBE-Virus Stamm Neudörfl konnte auf zwei verschiedene Arten von den beschriebenen rekombinanten Plasmiden generiert werden:
i) 1 µg NK-4 wurde mit 5 E des Restriktionsenzyms NheI linearisiert und durch Phenol/Chloroformextraktion gereinigt und mit Ethanol gefällt. Die DNA wurde dann in Klenow Reaktionspuffer (New England Biolabs) gelöst und in Gegenwart der Nukleotide dCTP und dTTP (Boehringer-Mannheim) für 15 min bei 37°C mit dem Enzym Klenow (New England Biolabs) in einem Reaktionsvolumen von insgesamt 30 µl inkubiert. Das Reaktionsprodukt wurde neuerlich wie oben gereinigt und dann in die RNA Transkriptionsreaktion eingesetzt.
ii) Mehrere µg 3ND-1 wurden wie oben beschrieben mit NheI geschnitten und der 5'-Überhang durch die Klenow-Reaktion teilweise aufgefüllt. Dann wurde das Plasmid mit ClaI geschnitten und das ungefähr 8000 nts lange TBE-spezifische Fragment auf 0.3% SeaKem Gold Agarose (FMC) von den anderen Reaktionsprodukten abgetrennt und durch Elekroelution gereinigt. Danach wurde ein Aliquot dieser Präparation zur Quantifizierung auf einem Agarose-Gel analysiert. 500 ng T7-2 wurden mit ClaI linearisiert und mit ungefähr 1-2 µg des gereinigten 3ND-1 Fragments vereinigt. Das Gemisch wurde gereinigt und in 30 µl Volumen mit T4 DNA Ligase (Stratagene) über Nacht bei 16°C ligiert. Der Ligationsansatz wurde dann bei 65°C 5 min inaktiviert und neuerlich gereinigt und für die in vitro-RNA-Transkriptionsreaktion eingesetzt.

RNA, die nach einer dieser beiden Methoden hergestellt worden war, wurde in BHK Zellen transformiert (Elektroporation) und die Virusvermehrung durch 4-Schicht ELISA des Zellkulturüberstandes beobachtet. Die Antigenstruktur des so gebildeten rekombinanten Virus wurde mit Hilfe einer Anzahl monoklonaler Antikörper, die gegen das Hüllprotein des TBE-Virus gerichtet sind (Heinz et al., 1983; Guirakhoo et al., 1989), analysiert und entsprach völlig dem des nativen TBE-Virus Stamm Neudörfl.

Die spezifische Infektiosität der nach dieser Methode aus NK-4 hergestellten RNA wurde mit jener von aus Viruspräparationen gereinigter RNA verglichen. Sie betrug für beide RNA Präparationen in verschiedenen Experimenten zwischen 5 und 50 pg RNA pro infektiöser Einheit (bei Elektroporation auf BHK Zellen wie beschrieben). Die Behandlung der Transkriptionsreaktion mit DNAseI nach der Transkription hatte keinen Einfluss auf die spezifische Infektiosität der RNA. RNA, die ohne CAP-Analog hergestellt wurde, wies eine 1000-fach geringere spezifische Infektiosität auf. Wurde das Plasmid nicht mit NheI und anschliessender Klenow-Inkubation behandelt, sondern mit AatII geschnitten, so ergab sich eine ungefähr 10-fach reduzierte spezifische Infektiosität. Allerdings bleibt dabei unklar, ob das 3'-Ende des Genoms des rekombinanten Virus mit jenem des nativen Virus identisch oder ob es zusätzliche Nukleotide enthält, da die AatII Stelle einige Nukleotide nach der NheI Stelle liegt (siehe Abb. 3).

### Beispiel 11:

### Herstellung von TBE-Virus Stamm Neudörfl, welches das 3'-Ende eines anderen Stammes (Stamm Hypr) enthält

Mehrere µg 3HA wurden mit Nhe I geschnitten (ergibt 2 Fragmente: 4210bp, 980 bp) und mit Klenow-Enzym inkubiert, wie oben für NK-4 beschrieben. Das Plasmid wurde mit Eag I weiter verdaut (ergibt 4 Fragmente: 3650; 800; 560; 180) und das entstandene 800 Nukleotide lange TBEV-spezifische Fragment aus einem 1% Agarose-Gel gereinigt. 1 µg NK-4 wurde ebenfalls mit EagI geschnitten und mit ungefähr 200 ng des gereinigten NheI-EagI-Fragments von Plasmid HA3 vereinigt. Das Gemisch wurde gereinigt, ligiert und transkribiert und die RNA, wie oben beschrieben, in BHK Zellen transformiert. Aus dem gebildeten rekombinanten Virus wurde RNA isoliert und in cDNA überführt. Von dieser cDNA wurden durch PCR einige kurze Abschnitte amplifiziert und die Sequenz dieser Fragmente direkt ohne vorherige Klonierung analysiert. Diese Sequenzanalysen bestätigten die erwartete Genomstruktur des rekombinanten Virus, mit der Sequenz des TBE-Virus Stamm Neudörfl bis zur Position 10038 und jener des TBE-Virus Stamm Hypr von 10039 bis zum 3'-Ende. Die mit monoklonalen Antikörpern (Guirakhoo et al. 1989; Holzmann et al. 1992) bestimmte Antigenstruktur entsprach vollkommen jener des Stammes Neudörfl, jedoch zeigte das rekombinante Virus in BHK Zellen eher den für Stamm Hypr charakteristischen stärkeren zytopathischen Effekt als jenen schwächeren, den das native TBE-Virus Stamm Neudörfl hervorruft.

### Beispiel 12:

### Herstellung einer attenuierten Mutante des TBE-Virus unter Verwendung von rekombinanter cDNA

Eine Punktmutation des TBE-Virus Stamm Neudörfl, die zu einer Änderung der Aminosäuresequenz des E Proteins an Position 384 von Tyrosin zu Histidin führt, hat eine signifikante Attenuierung des Virus in Bezug auf seine Neurovirulenz und seine Neuroinvasivität im Tiermodell (Maus) zur Folge (Holzmann et al.,1990). Diese attenuierte Mutante des TBE-Virus wurde nun auch mit Hilfe des erfindungsgemässen infektiösen Klons hergestellt:

Das Plasmid T7-2 wurde mit den Enzymen BamHI (1988) und ClaI (3154) geschnitten, die beiden entstehenden Fragmente der Länge von 5740 bp und 1170 bp wurden auf einem 1% Agarose-Gel aufgetrennt und eluiert. Das 1170 bp lange Fragment wurde mit HinfI (Schnittstelle in TBEV an Position 2136) geschnitten und die resultierenden Fragmente der Länge 1020 bp und 150 bp aufgetrennt und das 1020 bp lange Fragment eluiert. Ungefähr gleiche Mengen des erhaltenen BamHI/ClaI-Fragments (5740 bp) und HinfI/ClaI-Fragments (1020 bp) wurden nun mit einem 4- 5-fachen molaren Überschuss eines kleinen PCR-Fragments gemischt, das die gewünschte Mutation enthielt und wie folgt gewonnen wurde: 1 ng Plasmid T7-2 wurde als Ausgangsmaterial in eine PCR Reaktion mit den Primern T91 und Mut01 (Tabelle 1) eingesetzt. Der Primer Mut01 umspannt sowohl die oben erwähnte HinfI-Stelle (2136) als auch die zu mutierende Nukleotidposition (G statt A am Minusstrang des Genoms an Position 2103). Primer T91 wurde so positioniert, dass das entstehende PCR-Fragment auch die BamHI-Stelle bei Nukleotidposition 1988 enthält. Das erhaltene PCR-Produkt wurde mit den Enzymen BamHI und HinfI geschnitten, über ein 1.2% Agarose-Gel getrennt und das 150 bp lange BamHI/HinfI-Fragment durch Elektroelution isoliert und, wie oben angegeben, mit den beiden anderen Fragmenten gemischt. Das Gemisch dieser drei Fragmente wurde gereinigt, ligiert und in E.coli HB101 transformiert. Rekombinante Plasmide wurden isoliert und die gewünschte Sequenz durch Sequenzanalyse bestätigt. Das so hergestellte Plasmid, das sich nur in einer einzigen Punktmutation von dem Plasmid T7-2 unterschied, konnte in gleicher Weise wie T7-2 zur Herstellung von TBE-Virus verwendet werden (siehe oben). Das erhaltene rekombinante Virus (RB4) wurde mit den monoklonalen Antikörpern ( Heinz et al. 1983; Guirakhoo et al. 1989) analysiert, und wies exakt dasselbe Reaktionsmuster auf wie die von Holzmann et al. (1990) beschriebene attenuierte B4-Mutante.

### Beispiel 13:

### Charakterisierung der Virulenzeigenschaften der rekombinanten Mutante RB4

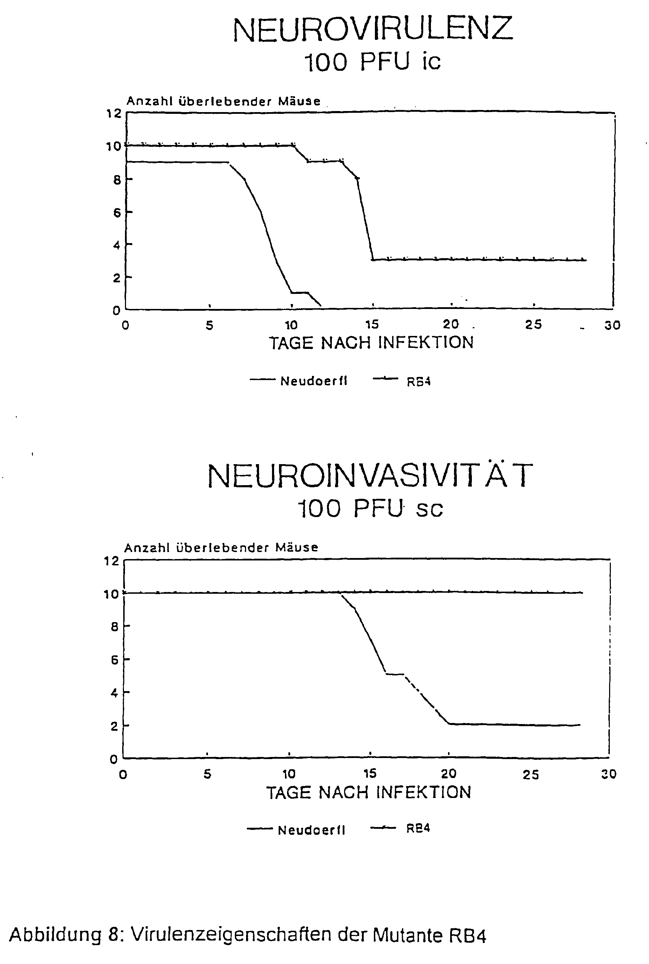

Zur Virulenzbestimmung wurden erwachsene Swiss Albino Mäuse verwendet, die sowohl nach intrazerebraler als auch nach subcutaner Infektion mit dem Wildtyp Stamm Neudörfl eine tödlich verlaufende Enzephalitis entwickeln. Gruppen von je 10 Mäusen wurden mit gleichen Mengen, nämlich jeweils 100 PFU (Plaque Forming Units), des Stammes Neudörfl und der Mutante RB4 intrazerebral bzw. subcutan infiziert. Wie aus der Abb. 8 hervorgeht, war sowohl die Neurovirulenz (i.c. Inokulation), als auch die Neuroinvasivität (s.c. Inokulation) der Mutante RB4 im Vergleich zum Stamm Neudörfl stark reduziert. Das mit Hilfe des infektiösen Klons hergestellte Virus RB4 entspricht in seinen Virulenzeigenschaften somit der mit Hilfe eines monoklonalen Antikörpers selektionierten Variante VB4 (Holzmann et al., 1990).

### Beispiel 14:

### Herstellung infektiöser RNA von 'infektiösen cDNA Klonen' des TBE Virus

5 mg des CsCl gereinigten Plasmids NK-4 (siehe Beispiel 6) wurden mit dem Restriktionsenzym Nhe I linearisiert und das überhängende Ende mit Hilfe des Enzyms Klenow-DNA-Polymerase teilweise aufgefüllt. Daraufhin wurde, wie in Beispiel 8 beschrieben, eine *in-vitro* Transkription durchgeführt, um eine RNA herzustellen, die dem Genom des TBE-Virus Stamm Neudörfl entsprach. Ebenso wurde das in Beispiel 12 beschriebene, mit einem Fragment des Plasmids 3ND1 ligierte und modifizierte Plasmid T7-2 dazu verwendet, eine RNA, die dem Genom einer attenuierten Mutante (RB4) des TBE-Virus entspricht, herzustellen.

Um die Infektiösität der RNAs zu verifizieren, wurde je ein Aliquot (entsprechend ca. 1 ng RNA) dieser beiden mit *in-vitro* Transkription gewonnenen RNAs mittels Elektroporation in BHK-Zellen transfiziert. Drei Tage nach der Elektroporation konnten funktionelle TBE-Viren vom Stamm Neudörfl bzw. von der attenuierten Mutante RB4 im Zellkulturüberstand nachgewiesen werden.

### Beispiel 15:

### Infektion von Mäusen mit infektiöser RNA.

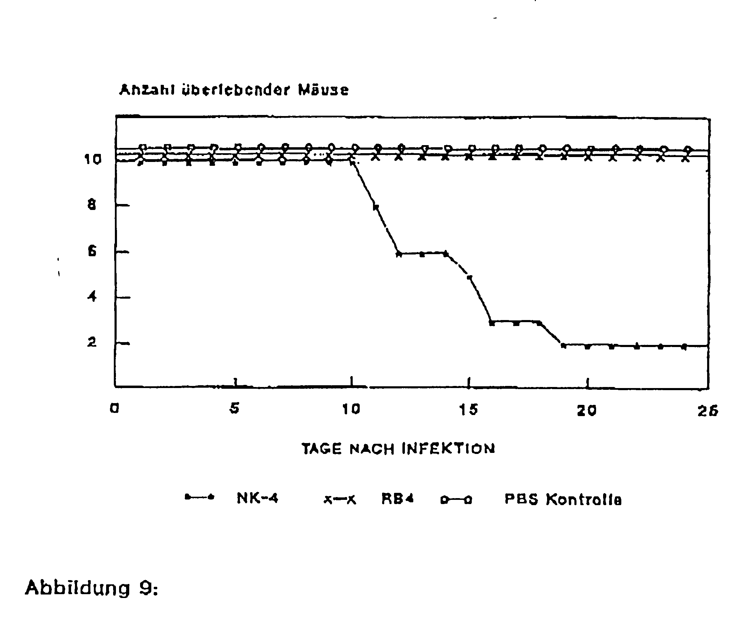

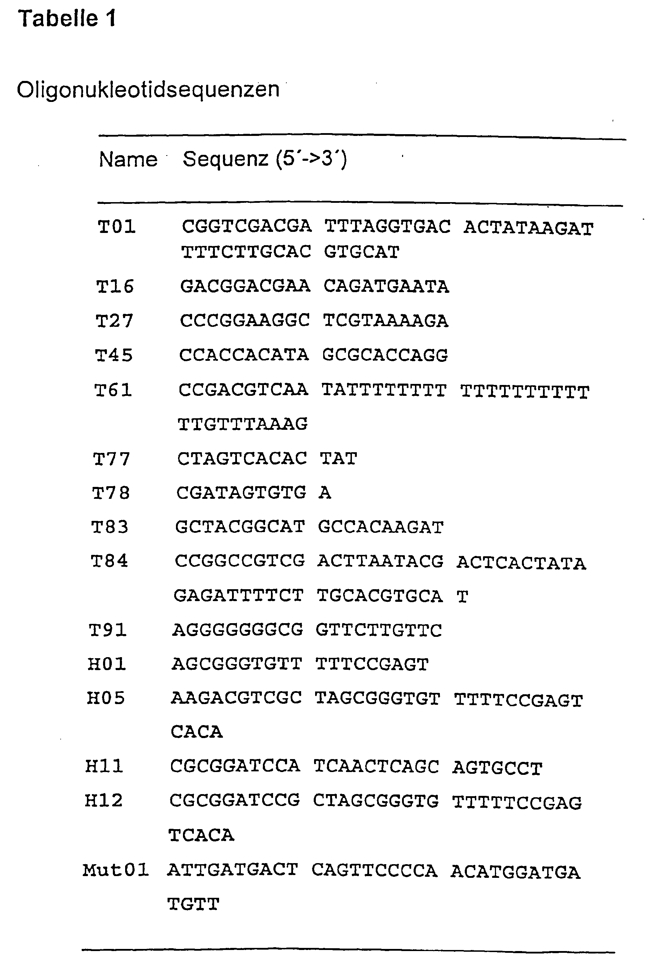

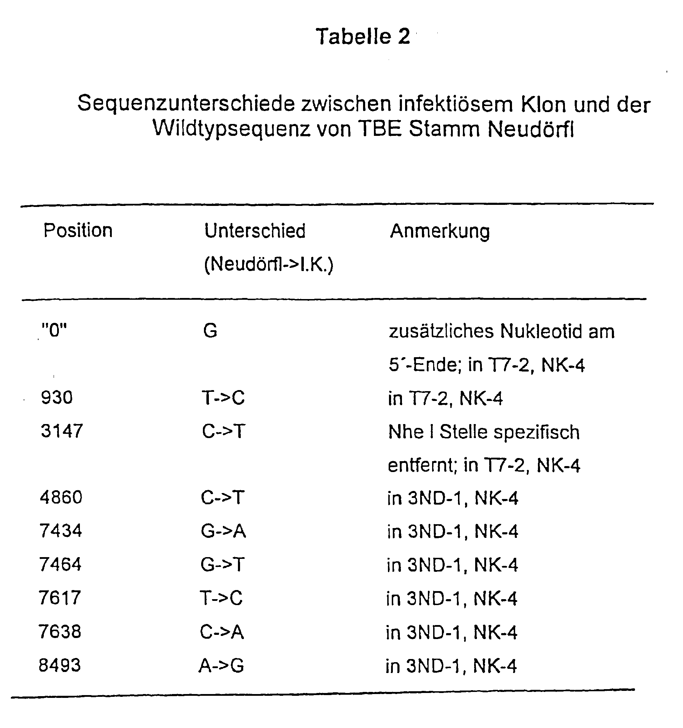

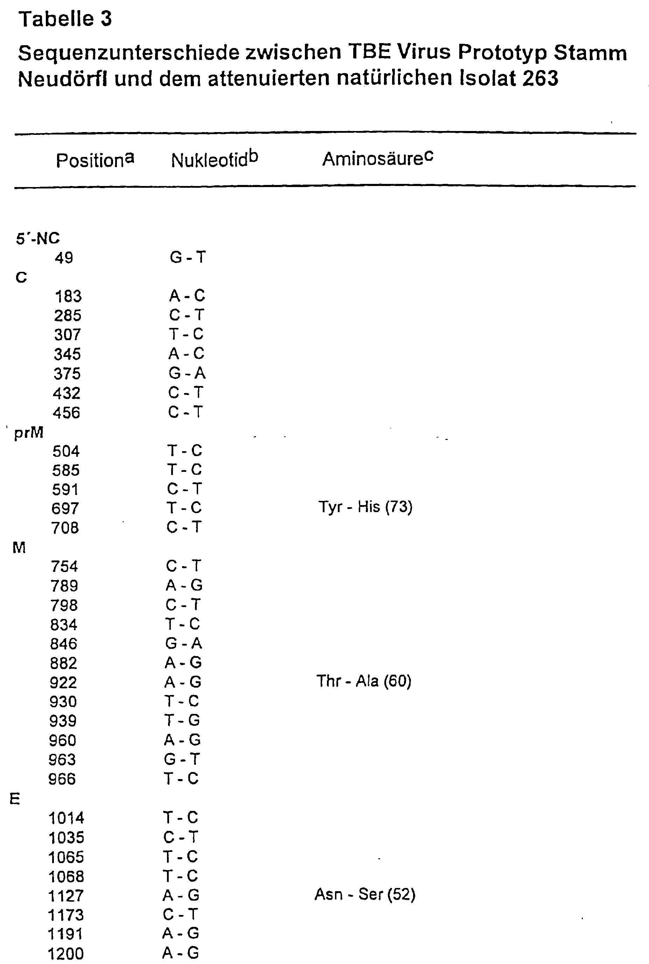

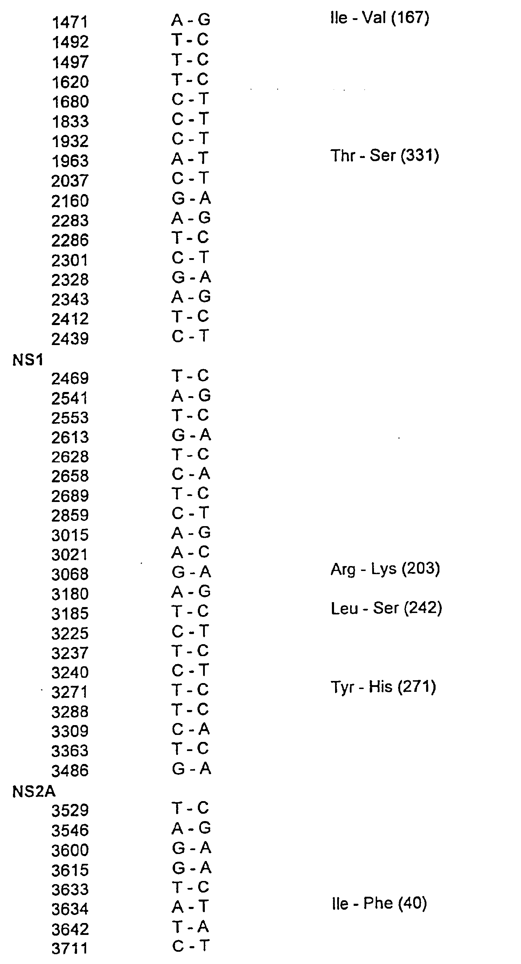

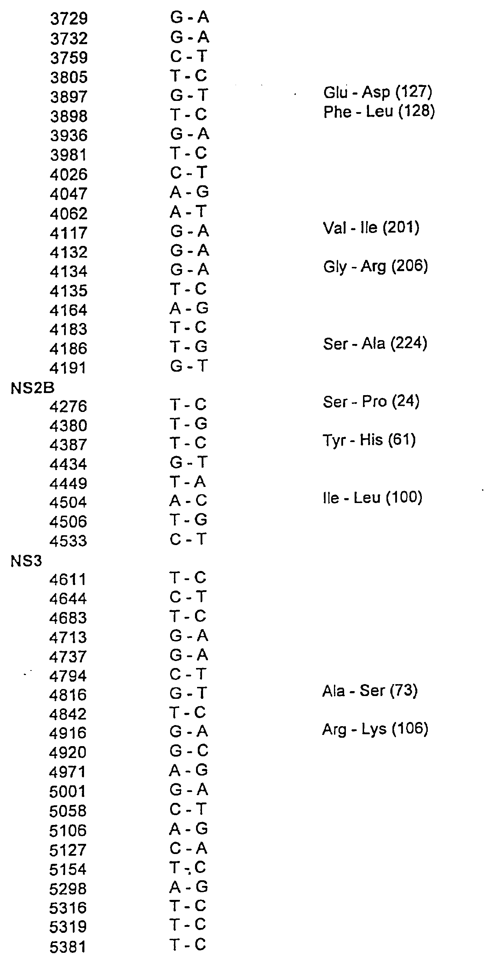

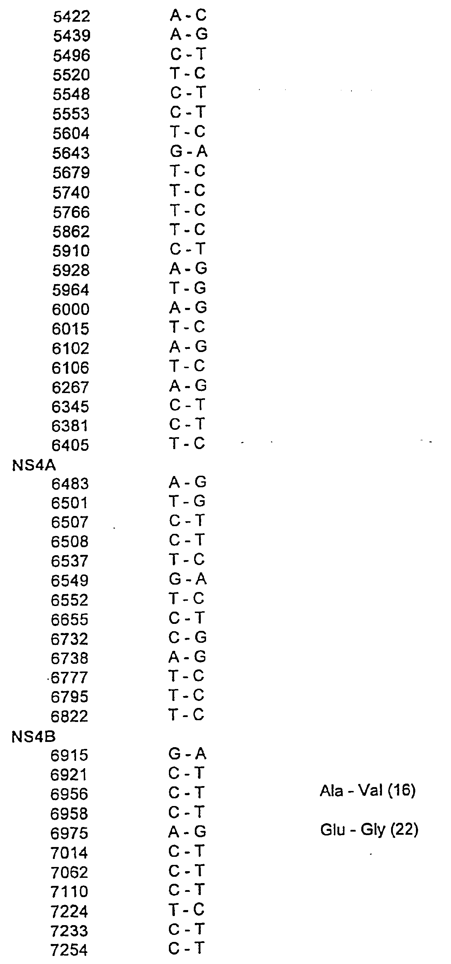

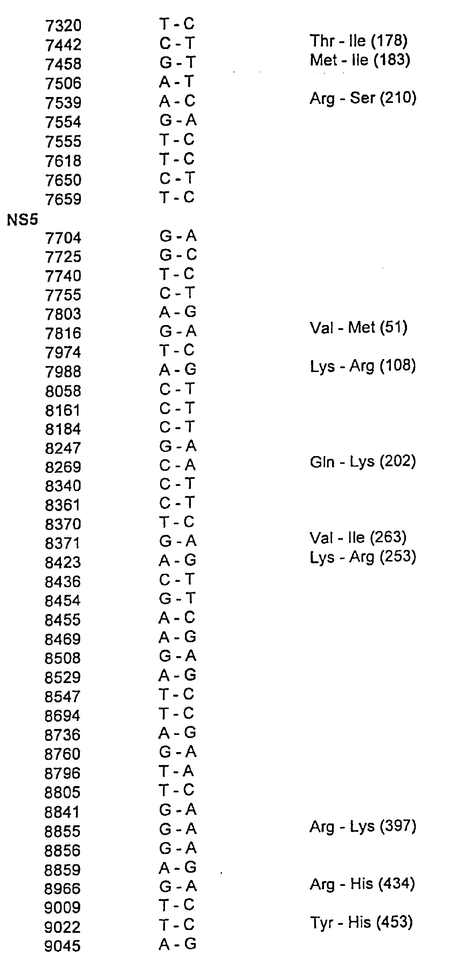

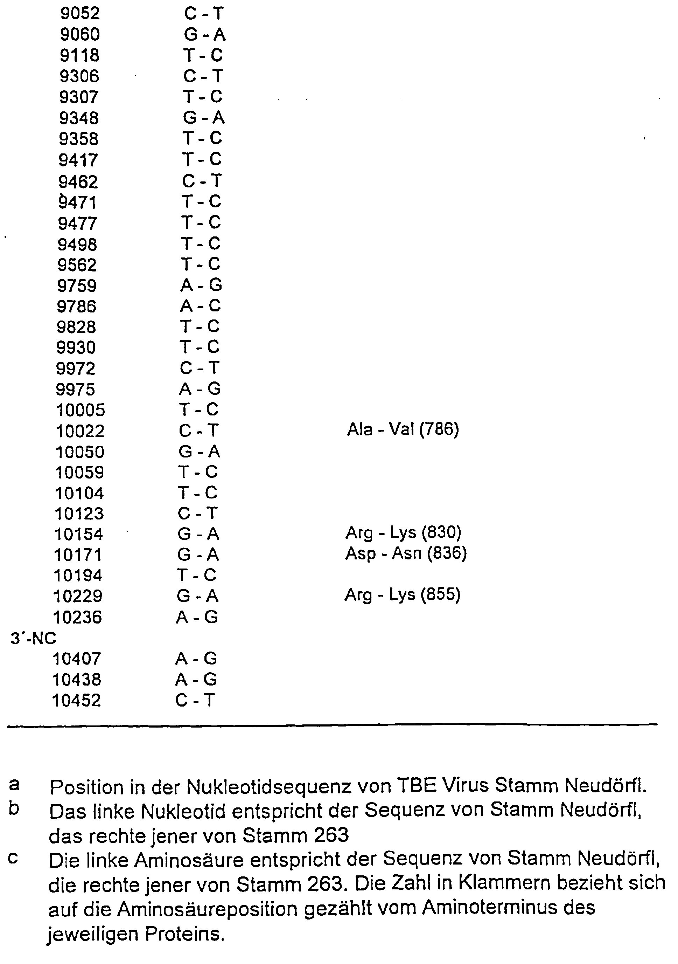

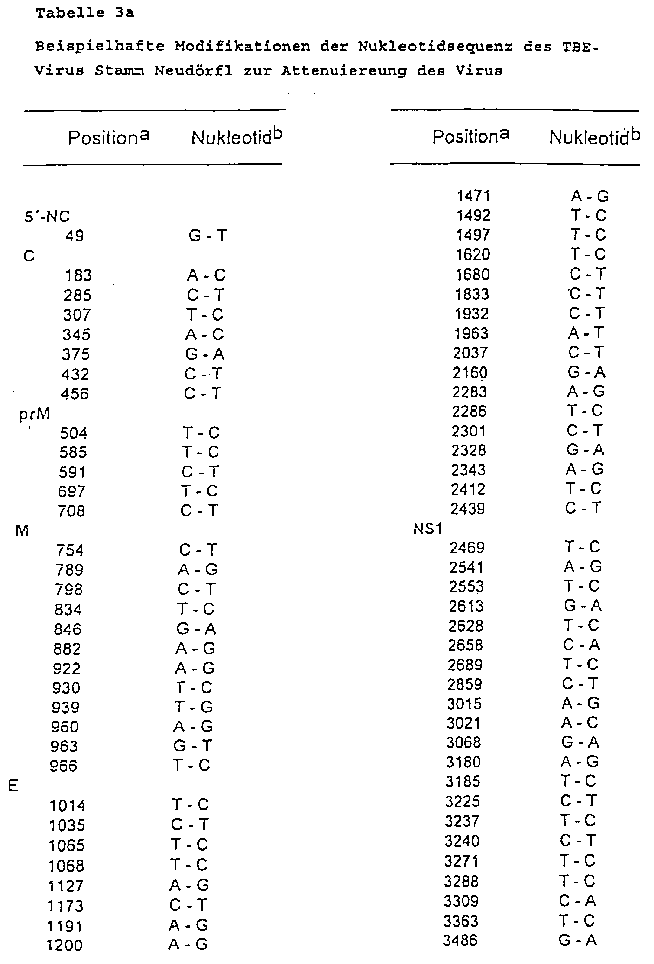

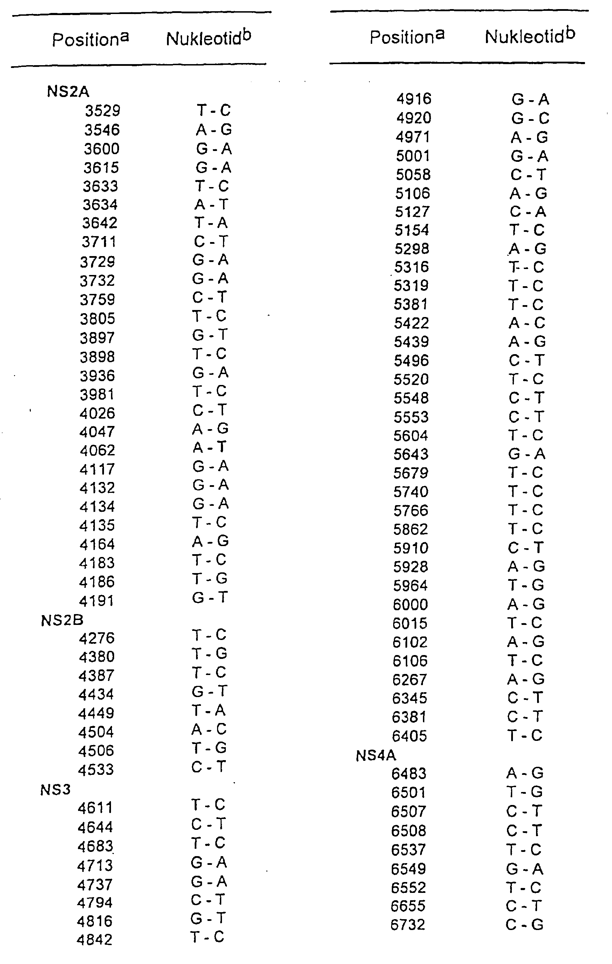

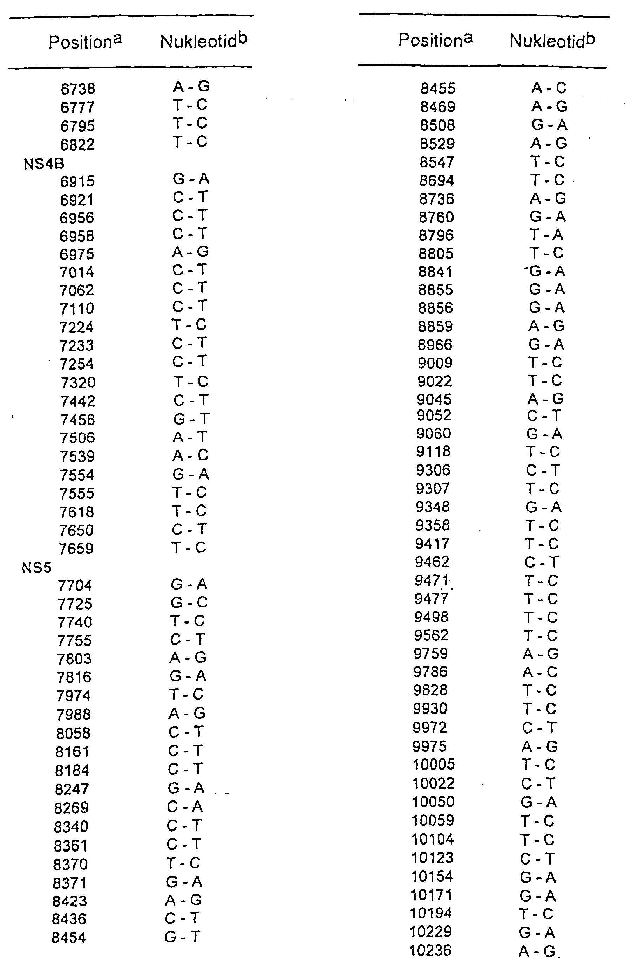

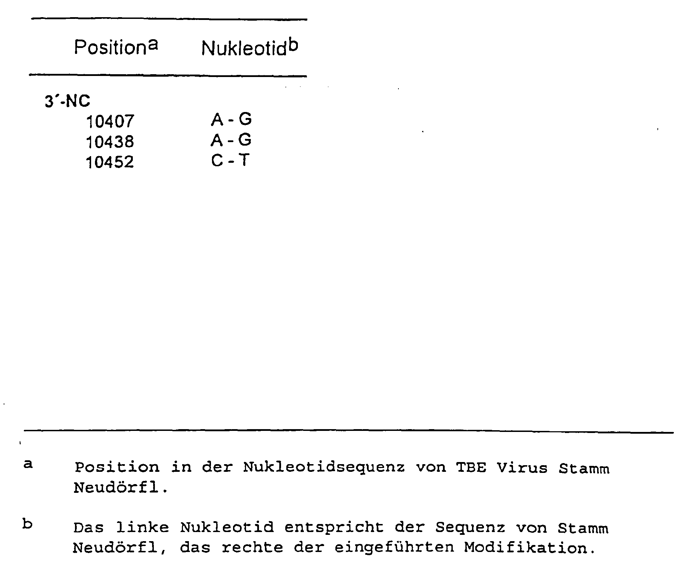

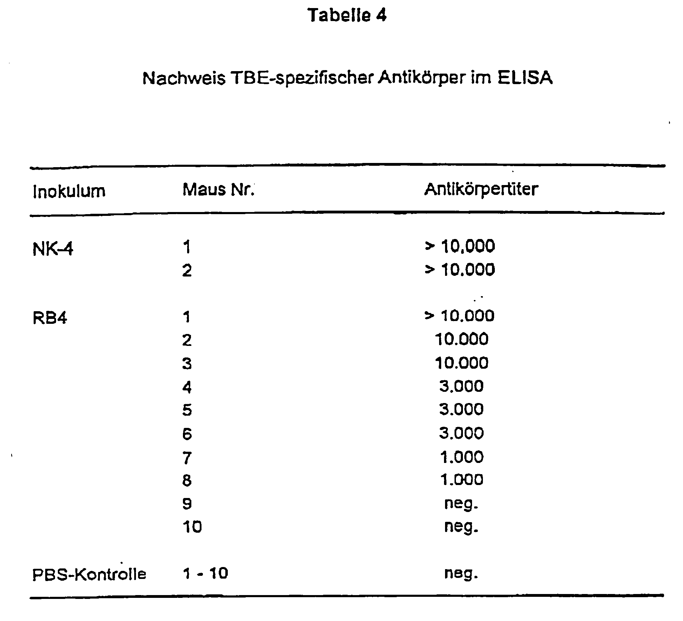

Die wie in Beispiel 14 hergestellte RNA wurde in 2 ml RNasefreiem PBS-Buffer resuspendiert, bevor 10 g schwere Swiss Albino Mäuse damit inokuliert wurden. Pro Gruppe wurden je 10 Mäusen jeweils 200 ml dieser Lösungen, etwa 5 mg infektiöse RNA enthaltend, subkutan injiziert. Ausser den beiden Gruppen, die mit dem Wildtypstamm Neudörfl und der attenuierten Mutante RB4 entsprechenden RNAs inokuliert wurden, wurde eine weitere Gruppe von 10 Mäusen zur negativen Kontrolle reiner PBS-Buffer injiziert. Die Anzahl überlebender Mäuse wurde über einen Zeitraum von 4 Wochen, wie in Abbildung 9 dargestellt beobachtet. Bei der PBS-Kontrollgruppe und der mit RB4-Genomen infizierten Gruppe von Mäusen erkrankten oder starben keine Mäuse während der gesamten Dauer des Experimentes. Bei den meisten der mit NK-4 RNA infizierten Gruppe zeigten sich ab dem 10. Tag die für TBE-Virus typischen neurologischen Symptome, bis zum 20. Tag waren 8 von 10 Mäusen gestorben. Dieses Ergebnis entspricht einem typischen Infektionsversuch mit einer mittleren Dosis (z.B.: 100 pfu) von TBE-Virus Stamm Neudörfl, wobei ebenfalls üblicherweise nicht alle Mäuse getötet werden, überlebende Tiere aber meist als Folge einer stattgefundenen Infektion hohe Titer schützender Antikörper aufweisen.

### Beispiel 16:

### Bestimmung der Antikörper-Titer in überlebenden Mäusen.

Den überlebenden Mäusen aller drei Gruppen wurde Blut aus dem Augenwinkel entnommen, und die Seren wurden auf das Vorhandensein TBE-spezifischer Antikörper mit Hilfe eines ELISA-Tests untersucht. Die Ergebnisse, die in Tabelle 4 zusammengefasst sind, zeigen, dass die beiden überlebenden Mäuse aus der mit NK-4 infizierten Gruppe sehr hohe Antikörpertiter (>1:10.000) aufwiesen, und dass ein Grossteil der RB4 Gruppe TBE-spezifische Antikörpertiter zwischen 1:3.000 und >1:10.000 aufwies, während zwei Tiere aus dieser Gruppe sowie erwartungsgemäss alle Mäuse der PBS-Kontrollgruppe keine TBE-spezifischen Antikörper besassen.

Dieses Ergebnis bedeutet, dass die beiden überlebenden Mäuse der NK-4 Gruppe eine Infektion mit dem TBE-Virus Stamm Neudörfl durchgemacht und überlebt haben. Von der RB4 Gruppe wurden 8 von 10 Mäusen infiziert, überlebten aber erwartungsgemäss alle die Infektion mit dieser attenuierten Mutante.

### Beispiel 17:

### Schutz der Antikörper-positiven Mäuse gegen einen Challenge mit TBE-Virus Stamm Hypr.

Die überlebenden Antikörper-positiven und -negativen Mäuse wurden nach Standardprotokollen mit dem virulenten TBE-Virus Stamm Hypr infiziert. Erwartungsgemäss starben alle Mäuse der PBS-Kontrollgruppe und die zwei Antikörper-negativen Mäuse der RB4 Gruppe innerhalb von 10 Tagen. Alle Antikörper positiven Mäuse, hingegen, waren gegen diesen Challenge mit virulentem TBE-Virus geschützt und zeigten keinerlei Anzeichen einer Erkrankung.

### Literaturverzeichnia

ACSADI, G., DICKSON, G., LOVI, D.R., JANI, A., WALSH, F.S., GURUSINGHE, A., WOLFF, J.A., DAVIES, K.E., (1991). Human dystrophin expression in mdx after intramuscular injection of DNA construct. Nature 352, 815-818.
CHAMBERS, T.J., HAHN, C.S., GALLER, R., RICE, C.M. (1990). Flavirus genome organization, expression and replication. Ann. Rev. Microbiol. 44, 649-688.
COHEN, J., (1993). Naked DNA points way to vaccines. Science 259, 1691-1692.
Cox, G.J.M., ZAMB, T.J., BABIUK, L.A., (1993). Bovine Herpesvirus 1: Immune responses in mice and cattle injected with plasmid DNA. J. Virol. 67, 5664-5667
DAVIS, H.L., MICHEL, M.L., WHALEN, R.G., (1993). DNA-based immunization induces continuous secretion of hepatitis B surface antigen and high levels of circulating antibody. Hum. Mol. Gen. 2, 1847-1651.
EMINI, E.A., SCHLEIF, W.A., NUNBERG, J.H., CONLEY, A.J., EDA, Y., TOKIYOSHI, S., PUTTTEY, S.D., MATSUSHITA, S., COBB, K.E., JETT, C.M., EICHBERG, J.W., AND MURTHY, K.K. (1992). Prevention of HIV-1 infection in chimpanzees by gp 120 V3 domain-specific monoclonal antibody. Nature 355, 728-729.
FYNAN, E.F., WEBSTER, R.G. FULLER, D.H., HAYNES, J.R., SANTORO, J.C., ROBINSON, H.L., (1993). DNA vaccines: protective immunizations by parenteral, mucosal, and gene-gun inoculations. Proc. Natl. Acad. Sci. USA 90, 11478-11482.
GAO, G.F., JIANG, W.R., HUSSAIN, M.H., VENUGOPAL, K., GRITSUN, T.S., REID, H.W., AND GOULD, E.A., (1993). Sequencing and antigenic studies of a Norwegian virus isolated from encephalomyelitic sheep confirm the existence of louping ill outside Great Britain and Ireland. J. Gen. Virol. 74, 109-114.
GUBLER, U., AND HOFFMAN, B.J. (1983). A simple and very efficient method for generating cDNA libraries. Gene 25, 263-269.
GULRAKHOO, F., HEINZ, F.X., and KUNZ, C. (1989). Epitope model of tick-borne Encephalitis virus envelope glycoprotein E: Analysis of structural properties, role of carbohydrate side chain, and conformational changes occuring at acidic pH. Virology 169, 90-99.
HAFFAR, O., GARRIGUES J., TRAVIS, B., MORAN, P., ZAARLING, J., HU S-L. (1990). Human immunodaficiency virus-like, nonreplicating, gag-env particles assemble in a recombinant vaccinia virus expression system. J. Virol. 64, 2653-2659.
HANSEN, E., FERNANDEZ, K., GOLDSPINK, G., BUTIERWORTH, P., UMEDA, P.K., CHANG, K.C., (1991). Strong expression of foreign genes following direct injection into fish muscle. Febs Lett, 290, 73-76.
HASLOV, K., PETERSON, J.W., BAKER, P.J. (1991). Adjuvanticity of pertussis toxin is mediated by differential effects on the activity of T suppresors, T amolifier and T helper cell. Immunobiol. 183, 40-54
HANKTNS, R.E., WINTER, G., HAMBLIN, T.J., STEVENSON, F.K.,RUSSELL, S.J., (1993). A genetic approach to idiotypic vaccination. J. Immunotherapy 14, 273-278
HAZAME, M., MAYUMI-AONO, A., ASAKAWA, N., KURODA, S., HINUMA, S., AND FUJISAWA, Y. (1993). Adjuvant-independent enhanced immune responses to recombinant herpes Simplex virus type 1 glycoprotein D by fusion with biologically active interleukin-2. Vaccine 11, 629-635.
HEINZ, F.X., AND KUNZ C. (1981). Homogeneity of the structural glycoprotein from European isolates of tick-borne encephalitis virus: Comparison with other flaviviruses. J. Gen. Virol. 57, 263-274.
HEINZ, F.X., BERGER, R., TUMA, W., and KUNZ, C. (1983). A topological and functional model of epitopes on the structural glycoprotein of tick-borne encephalitis virus defined by monoclonal antibodies. Virology 126, 525-537.
HEINZ, F.X., TUMA W., GUIRAKHOO, F., AND KUNZ, C. (1986). A model study of the use of monoclonal antibodies in capture enzyme immunoassays for antigen quantification exploiting the epitope map of tick-borne encephalitis virus. J. Biol. Standard. 14, 133-141.
HOLZMANN, H., HEINZ, F.X., MANDL, C.W., GUIRAKHOO, F., AND KUNZ, C. (1990). A single amino acid substitution in the envelope protein E of tick-borne encephalitis virus leads to attenuation in the mouse model. J. Virol. 64, 5156-5159.
HOLZMANN, H., VOROBYOVA, M . S . , LADYZHENSKAYA, I.P., FERENCZI, E., KUNDI, M., KUNZ, C., AND HEINZ, F.X. (1992). Molecular epidemiology of tick-borne encephalitis virus: crossprotection between European and Far Eastern subtypes. Vaccines 10, 345-349
HUSSAIN, A., HIMENO, K.,MAYUMI, H., KAWAMURA, I., TSURU, S., NOMOTO, K. (1989). Immunomodulatory effects of cholera toxin in mice. Nat. Immun. Cell. Growth Regul. 8, 231-244.
JAVAHERIAN, K., LANGLOIS, A.J., MCDANAL, C., Ross, K.L., ECKLER, L.I., JELLIS, C.L., PROFY, A.T., RUSCHE, J.R., BOLOGNESI, D.P., PUTNEY, S.D., AND MATTHEWS, T.J. (1989). Principal neutralizing domain of the human immunodeficiency virus type 1 envelope protein. Proc. Natl.Acad. Sci. USA 86, 6768-6772.
KARAKOSTAS, V., NAGASHIMA, K., GONDA, M.A., Moss, B. (1989). Human immunodeficiency virus-like particles produced by a vaccinia virus expression vector. PNAs 86, 8964-8967.
KRAUSSLICH, H-G., OCHSENBAUER, C. TRAENCKNER, A-M., MERGENER, K., FACKE, M., GELDERBLOM, H.R., BOSCH, V. (1993). Analysis of protein expression and virus-like particle formation in mammalian cell lines stably expressing HIV-1 gag and env gene products with or without active HIV protease. Virology 192, 605-617.
LAI, C., IHAO, B., HORI, H., BRAY, M. (1991). Infectious RNA transcribed from cloned full-lenght cDNA of Dengue type 4 virus. Proc. Natl. Acad. Sci. USA. 88, 5139-5143.
LEDGER, T.N., SIL, B.K., WILLS, M.R., LEWIS, G., KINNEY, R.M., JENNINGS, J.R., STEPHENSON, J.R., AND BARRETT, A.D.T. (1992). Variation in the biological function of envelope protein epitopes of yellow fever vaccine viruses detected with monoclonal antibodies. Biologicals 20, 117-128
MANDL, C.W., HEINZ, F.X., AND KUNZ, C. (1988). Sequence of the structural proteins of tick-borne encephalitis virus (Western subtype) and comparative analysis with other flaviviruses. Virology 166, 197-205.
MANDL, C.W., HEINZ, F.X., STÖCKL, E., AND KUNZ, C. (1989). Genome sequence of tick-borne encephalitis virus (Western subtype) and comparative analysis of nonstructural proteins with other flaviviruses. Virology 173, 291-301.
MANDL, C.W., HOLZMANN, H., KUNZ, C., AND HEINZ, F.X. (1993). Complete genomic sequence of Powassan virus: Evaluation of genetic elements in tick-borne versus mosquito-borne flaviviruses. Virology 194, 173-184.
MANDL, C.W., KUNZ, C., AND HEINZ, F.X. (1991). Presence of poly(A) in a flavivirus: Significant differences between the 3' noncoding regions of the genomic RNAs of tick-borne encephalitis virus strains. J. Virol. 65, 4070-4077.
MANIATIS, T., FRITSCH, E.F., AND SAMBROOK, J. (eds.) (1982). "Molecular Cloning, a Laboratory Manual", Cold Spring Harbor Laboratory, Cold Spring Harbor, New York.
McMASTER, G.K., AND CARMICHAEL, G.G. (1977). Analysis of single- and double-stranded nucleic acids on polyacrylamide and agarose gels by using glyoxal and acridine orange. Proc. Natl. Acad. Sei. USA 11, 4835-4838.
MONATE, T.P. (1990), Flaviviruses In: "Virology 2nd edn." 1, (B.N. Fields and D.M. Knipe ed.) pp. 763-814, Raven Press, New York.
MONTGOMERY, D.L., SHIVER, J.W., LEANDER, K.R., PERRY, H.C., FRIEOMAN, A., MARTINEZ, D., ULMER, J.B.,DONNELLY, J.J., LIU, M.A., (1993). Heterologous and homologous protection against influenza A by DNA vaccination: optimization of DNA vectors. DNA Cell Biol. 12, 777-783.
PLETNEV, A.G., BRAY, M., AND LAI, C.-J. (1993).Chimeric tick-borne encephalitis and dengue type 4 viruses: Effects of mutations on neurovirulence in mice. J. Virol. 67, 4956-4963
POST, P.R., SANTOS, C.N.D., CARVALHO, R., CRUZ, A.C.R., RICE, C.M., AND GALLER, R. (1992). Heterogeneity in envelope protein sequences and N-linked glycosylation among yellow fever virus vaccine strains. Virology 188, 160-167.
RANCANIELLO, V.R., BALTTIMORE, D. (1981) Science, 214, 916-919 RAz, E., CARSON, D.A., PARKER, S.E., PARR, T.B., ABAI, A.M., AICBINGER, G., GROMKOWSKI, S.H., SINGH, M., LEW, D., YANKAUCKAS, M.A., BAIRD, S.M., RHODES, G.H., (1994). Intradermal gene immunization: the possible role of DNA uptake in the induction of cellular immunity to viruses. Proc. Natl. Acad. Sci. USA 91, 9519-9523.
REVAILLARD, J.P., COZON, G., CZERKINSKY, C. (1992). Oral administration of immunomodulators and the mucosal immune system. Dev. Biol. Stand. 77, 31-37.
RICE, C.M., GRAKOUI, A., GALLER, R., AND CHAMBERS, T.J. (1989). Transcription of infectious yellow fever RNA from full-length cDNA templates produced by in vitro ligation. The New Biol. 1, 285-296.
ROBINSON, H.L., HUNT; L. A., WEBSTER, R.G., (1993). Protection against a lehtal influenza virus challenge by immunization with a haemagglutinin-expressing plasmid DNA. Vaccine 11, 957-960.
RUECKERT, R. R. (1990). Picornaviridae and their replication. In: "Virology 2nd edn." 1, (B.N. Fields and D.M. Knipe ed.) pp. 507-548, Raven Press, New York.
SHAH, K.V. (1990). Polyomaviruses. In: "Virology 2nd edn." 2, (B.N. Fields and D.M. Knipe ed.) pp. 1609-1624, Raven Press, New York.
SUMIYOSHI, H., HOKE, C., TRENT, D.W. (1992). Infectious Japanese Encephalitis Virus RNA can be synthesized from in vitro ligated cDNA template. J. Virol. 66, 5425-5431.
TAGLIABUE, A., AND BORASCHI, D. (1993). Cytokines as vaccine adjuvants: Interleukin 1 and its synthetic peptide 163-171. Vaccine 11, 594-595.
ULMER, J.B., DONNELLY, J.J., PARKER, S.E., RHODES, G.H., FELGNER, P.L., DWARKI, V.J., GROMKOWSKI, S.H., DECK, R.R., DEWITT, C.M., FRIEDMAN, A., HAWE, L.A., LEANDER, K.R., MARTINEZ, D., PERRY, H.C., SHIVER, J.W., MONTGOMERY, D.L., LIU, M.A., (1993). Heterologous protection against influenza by injection of DNA encoding a viral protein. Science 259, 1745-1749.
ULMER, J.F, DONNELLEY J.J., PARKER, S.E., RHODES, G.H, FELGNER P.L., DWARKI, V.J., GROMKOWSKI, S.H, DECK, R.R., DEWITT, C.M., FRIEDMAN, A., HAWE, L.A., LEANDER, K.R., MARTINEZ, D., PERRY, H.C., SHTVER, J.W., MONTGOMERY, D.L., LIU, M.A. (1993). Heterologous protection against influenza by injection of DNA encoding a viral protein. SCIENCE 259, 1745
VENUGOPAL, K., BUCKLEY, A., REIP, H.W., AND GOULD E.A. (1992). Nucleotide sequence of the envelope glycoprotein of Negishi virus shows very close homology to louping ill virus. Virology 190, 515-521.
WANG, B., UGEN, K.E., SRIKANTAN, V., AGADJANYAN, M.G., DANG, K., REFAELI, Y., SATO, A.I., BOYER, J., WILLIAMS, W.V., WEINER, D.B., (1993). Gene inoculation generates immune responses against human immunodeficiency virus type 1. Proc. Natl. Acad. Sci. USA 90, 4156-4160-
WATANABE, A., RAZ, E., KOHSAKA, H., TIGHE, H., BAIRD, S.M., KIPPS, T.J., CARSON, D.A., (1993). Induction of antibodies to a k V region by gene immunization. J. Immunol. 151, 2871-2876.
WHITBY, J.E., WHITBY, S.N., JENNINGS, A.D., STEPBEM50M, J.R., AND BARRETT, A.D.T. (1993). Nucleotide sequence of the envelope protein of a Turkish isolate of tick-borne encephalitis (TBE) virus is distinct from other viruses of the TBE virus complex. J. Gen. Virol. 74, 921-924.
WOLFF, J.A., MALONE, R.W., WILLIAMS, P.,CHONG, W., ACSADI, G., JANI, A., FELGNER, P.L., (1990). Direct gene transfer into muscle in vivo. Science 247, 1465-1468.
EP 0 198 474, Endotronics Inc.
WO 90/11092, Vical Inc.
WO 93/17706, Agracetus Inc.
WO 93/19183, University of Massachusetts Med. Cent.
WO 94/16737, Weiner, D.B. et al.

## Patentansprüche

1. In vivo replikationsfähige, infektiöse Nukleinsäure kodierend für ein komplettes Tick-Borne Enzephalitis (TBE)-Virus oder für ein modifiziertes TBE-Viruspartikel zur Verwendung als pharmazeutisches Produkt.

2. In vivo replikationsfähige, infektiöse Nukleinsäure nach Anspruch 1, **dadurch gekennzeichnet, dass** die replikationsfähige Nukleinsäure eine DNA ist.

3. In vivo replikationsfähige, infektiöse Nukleinsäure nach Anspruch 1, **dadurch gekennzeichnet, dass** die replikationsfähige Nukleinsäure eine RNA ist.

4. In vivo replikationsfähige, infektiöse Nukleinsäure nach einem der Ansprüche 1 bis 3, **dadurch gekennzeichnet, dass** das komplette Virus ein attenuiertes TBE-Virus ist.

5. In vivo replikationsfähige, infektiöse Nukleinsäure nach einem der Ansprüche 1 bis 4, **dadurch gekennzeichnet, dass** die replikationsfähige Nukleinsäure zusätzliche Nukleinsäuresequenzen enthält.

6. In vivo replikationsfähige, infektiöse Nukleinsäure nach einem der Ansprüche 1 bis 5, **dadurch gekennzeichnet, dass** die replikationsfähige Nukleinsäure in einem Nukleinsäurevektor, der die Replikation in prokaryotischen Zellen erlaubt, enthalten ist.

7. In vivo replikationsfähige, infektiöse Nukleinsäure nach einem der Ansprüche 1 bis 6, **dadurch gekennzeichnet, dass** die Nukleinsäure modifiziert ist.

8. In vivo replikationsfähige, infektiöse Nukleinsäure nach einem der Ansprüche 1 bis 7, **dadurch gekennzeichnet, dass** die Nukleinsäure in einer physiologisch wirksamen Menge enthalten ist.

9. In vivo replikationsfähige, infektiöse Nukleinsäure nach Anspruch 8, **dadurch gekennzeichnet, dass** es in einer therapeutisch, insbesondere intramuskulär, oral oder subkutan verabreichbaren Form vorliegt.

10. Verwendung einer in vivo replikationsfähigen, infektiösen Nukleinsäure kodierend für ein komplettes TBE-Virus oder für ein modifiziertes TBE-Viruspartikel, zur Herstellung eines Arzneimittels zur Therapie oder Prophylaxe.

11. Verwendung einer in vivo replikationsfähigen, infektiösen Nukleinsäure nach Anspruch 10 zur Herstellung eines Impfstoffes zur Immunisierung von Vertebraten.

## Claims

1. In vivo replicable, infectious nucleic acid coding for a complete tick-borne encephalitis (TBE) virus or for a modified TBE virus particle for use as a pharmaceutical product.

2. In vivo replicable, infectious nucleic acid according to claim 1, **characterised in that** the replicable nucleic acid is a DNA.

3. In vivo replicable, infectious nucleic acid according to claim 1, **characterised in that** the replicable nucleic acid is a RNA.

4. In vivo replicable, infectious nucleic acid according to any of claims 1 to 3, **characterised in that** the complete virus is an attenuated TBE virus.

5. In vivo replicable, infectious nucleic acid according to any of claims 1 to 4, **characterised in that** the replicable nucleic acid contains additional nucleic acid sequences.

6. In vivo replicable, infectious nucleic acid according to any of claims 1 to 5, **characterised in that** the replicable nucleic acid is contained in a nucleic acid vector allowing replication in prokaryotic cells.

7. In vivo replicable, infectious nucleic acid according to any of claims 1 to 6, **characterised in that** the nucleic acid is modified.

8. In vivo replicable, infectious nucleic acid according to any of claims 1 to 7, **characterised in that** the nucleic acid is present in a physiologically effective quantity.

9. In vivo replicable, infectious nucleic acid according to claim 8, **characterised in that** it is present in a therapeutically, in particular intramuscularly, orally or subcutaneously administrable form.

10. Use of an in vivo replicable, infectious nucleic acid coding for a complete TBE virus or for a modified TBE virus particle for manufacturing a drug for therapy or prophylaxis.

11. Use of an in vivo replicable, infectious nucleic acid according to claim 10 for manufacturing a vaccine for immunising vertebrates.

## Revendications

1. Acide nucléique infectieux, apte à une réplication in vivo, codant pour un virus complet de l'encéphalite transmise par les tiques (TBE) ou pour une particule de virus TBE modifiée, pour utilisation en tant que produit pharmaceutique.

2. Acide nucléique infectieux apte à une réplication in vivo selon la revendication 1, **caractérisé en ce que** l'acide nucléique apte à la réplication est un ADN.

3. Acide nucléique infectieux apte à une réplication in vivo selon la revendication 1, **caractérisé en ce que** l'acide nucléique apte à la réplication est un ARN.

4. Acide nucléique infectieux apte à une réplication in vivo selon l'une des revendications 1 à 3, **caractérisé en ce que** le virus complet est un virus TBE atténué.

5. Acide nucléique infectieux apte à une réplication in vivo selon l'une des revendications 1 à 4, **caractérisé en ce que** l'acide nucléique apte à la réplication contient des séquences d'acide nucléique supplémentaires.

6. Acide nucléique infectieux apte à une réplication in vivo selon l'une des revendications 1 à 5, **caractérisé en ce que** l'acide nucléique apte à la réplication est contenu dans un vecteur d'acide nucléique permettant la réplication en des cellules procaryotiques.

7. Acide nucléique infectieux apte à une réplication in vivo selon l'une des revendications 1 à 6, **caractérisé en ce que** l'acide nucléique est modifié.

8. Acide nucléique infectieux apte à une réplication in vivo selon l'une des revendications 1 à 7, **caractérisé en ce que** l'acide nucléique est contenu en une quantité physiologiquement efficace.

9. Acide nucléique infectieux apte à une réplication in vivo selon la revendication 8, **caractérisé en ce qu'**il se présente sous une forme pouvant être administrée thérapeutiquement, en particulier par voie intramusculaire, orale ou sous-cutanée.

10. Utilisation d'un acide nucléique infectieux apte à une réplication in vivo, codant pour un virus TBE complet ou pour un virus TBE modifié, pour la préparation d'un médicament pour la thérapie ou la prophylaxie.

11. Utilisation d'un acide nucléique infectieux apte à la réplication in vivo selon la revendication 10, pour la préparation d'un vaccin pour l'immunisation de vertébrés.
